# EUROPEAN PATENT APPLICATION

(11) **EP 4 692 337 A1**
(43) Date of publication of application: **11.02.2026**
(21) Application number: 24777751.9
(22) Date of filing: 14.03.2024
(51) Int. Cl.: C12N 9/22, C12N 9/78, C07K 19/00, C12N 15/55, C12N 15/113, C12N 15/70, C12N 15/85, C12N 5/10, C12Q 1/6816, A61K 38/46, A61K 38/50, A61P 35/00, A61P 31/00, A61P 25/00, A61P 37/02, A61P 27/16

(54) **CAS PROTEIN AND MUTANT THEREOF, AND CORRESPONDING GENE EDITING SYSTEM AND USE THEREOF**

(30) Priority: 24.03.2023 CN 202310302690
(71) Applicant: Yoltech Therapeutics Co., Ltd, Shanghai 201109 (CN)
(72) Inventor: ZHANG, Hongling, Shanghai 201109 (CN)
(74) Representative: CH Kilger Anwaltspartnerschaft mbB
(86) International application number: PCT/CN2024/081724
(87) International publication number: WO 2024/198961

(57) **Abstract**

The present invention provides a Cas protein and its variants, and the corresponding gene-editing system and uses. Specifically, the Cas protein of the present invention has excellent gene-editing activity, can effectively edit or cleave a target gene, and can effectively treat disorders or diseases of a subject in need. The present invention also relates to an editing system and uses of a fusion protein containing the Cas protein or its variant.

## Description

### TECHNICAL FIELD

The disclosure relates to the field of gene editing, specifically, to a Cas protein and mutants thereof, and a corresponding gene editing system and use thereof.

### BACKGROUND

The Clustered Regularly Interspaced Short Palindromic Repeats (CRISPR) system is developed by bacteria and archaea to defend against the DNA of invading bacteriophages. The most common one is the CRISPR/Cas9 system. The Cas9 protein, with the assistance of a trans-encoded small RNA (tracrRNA), can process pre-crRNA into mature crRNA bound to the tracrRNA. It was then discovered that an artificially constructed single-stranded chimeric guide RNA (gRNA) that mimics the crRNA-tracrRNA complex can effectively mediate the recognition and cleavage of a target site by the Cas9 protein. The three bases adjacent to the 3' end of the target site must be in the form of 5'-NGG-3', thereby forming the proto-spacer adjacent motif (PAM) structure required by the Cas/crRNA complex to recognize the target site.

However, current different CRISPR/Cas systems have different advantages and disadvantages. For example, there are differences in the guide RNA, the PAM, and the size of the Cas protein.

Therefore, there is still a need to develop new Cas proteins and mutants thereof, as well as CRISPR-Cas systems to meet diverse application requirements.

### SUMMARY

The main objective of the disclosure is to provide a novel Cas protein and mutants thereof, and a gene editing system and use thereof, to meet the above-mentioned application requirements. Based on this, the disclosure also provides a new CRISPR-Cas composition, as well as a gene editing method and a nucleic acid detection method based on this system.

In a first aspect of the disclosure, a Cas protein is provided, wherein the Cas protein is selected from the group consisting of:
(a) a polypeptide having the amino acid sequence of SEQ ID NO: 1;
(b) a polypeptide having a homology (or identity) of ≥ 80%, 81%, 82%, 83%, 84%, 85%, 86%, 87%, 88%, 89%, 90%, 91%, 92%, 93%, 94%, 95%, 96%, 97%, 98%, 99% or 99.5% with the amino acid sequence of SEQ ID NO:1, and having the biological function of SEQ ID NO: 1; and
(c) a derivative polypeptide generated by substitution, deletion, or addition of one or more (preferably 1-20, more preferably 2-18, even more preferably 3-16) amino acid residues in the amino acid sequence of SEQ ID NO: 1, and retaining the biological function of SEQ ID NO: 1.

In some embodiments, the Cas protein includes wild-type and mutant Cas proteins (i.e., orthologs, homologs, variants, or functional fragments of the Cas protein, wherein the orthologs, homologs, variants, or functional fragments essentially retain the biological function of the sequence from which they are derived).

In certain embodiments, the Cas protein is a variant of the protein having the amino acid sequence of SEQ ID NO: 1.

In some embodiments, the Cas protein does not naturally exist.

In certain embodiments, the Cas protein is a Cas protein variant. The variant is an unnatural protein, and comprises mutations at one or more key amino acid sites related to cleavage activity in SEQ ID NO: 1 corresponding to the wild-type Cas protein, wherein the one or more key amino acid sites are selected from the group consisting of: E118, G119, D120, V121, Q122, Y123, K241, S242, S243, S246, L272, S275, C277, T285, S286, C287, D288, E289, A290, I292, K299, R300, W301, L394, D395, R396, D397, R398, K399, E400, D401, E402, D403, S404, C405, R406, F407, E408, K502, K557, L657, C672, P758, L789, K827, and V860.

In some embodiments, compared to the cleavage activity of the wild-type Cas protein, the cleavage activity of the Cas protein variant on a target sequence of a target molecule complementary to the guide RNA is increased, or is equivalent to that of the wild-type Cas protein, or the binding of the CRISPR composition containing the Cas protein variant to the binding site is enhanced, or the editing preference is altered.

In certain embodiments, compared to the wild-type Cas protein, the spacer-specific endonuclease cleavage activity of the Cas protein variant on a target sequence of a target DNA complementary to the spacer is significantly increased (for example, increased by at least 5%, preferably at least 10%, more preferably at least 20%, such as at least 30%, at least 40%, at least 50%, at least 60%, at least 70%, at least 80%, at least 90%, or at least 100%, for instance, at least by 100%, at least by 200%, at least by 300%, at least by 400%, at least by 500%, at least by 600%, at least by 800%, at least by 900%, at least by 1,000%).

In certain embodiments, compared to the polypeptide having the amino acid sequence of SEQ ID NO: 1, the Cas protein comprises:
(1) a mutation at E118 to any amino acid, preferably to an amino acid selected from those shown in Table A, Table B, and Table C, more preferably selected from E118D, E118K, E118I, E118Q, E118P, E118S, E118T, and E118Y;
(2) a mutation at G119 to any amino acid, preferably to an amino acid selected from those shown in Table A, Table B, and Table C, more preferably selected from G119P, G119A, G119V, G119L, G119I, G119D, G119M, G119R, and G119S, even more preferably selected from G119D, G119M, G119L, G119P, G119R, G119S, and G119A, even more preferably selected from G119D, G119M, G119L, G119P, G119R, and G119S;
(3) a mutation at D120 to any amino acid, preferably to an amino acid selected from those shown in Table A, Table B, and Table C, more preferably selected from D120E, D120K, D120L, D120N, D120G, D120S, and D120T;
(4) a mutation at V121 to any amino acid, preferably to an amino acid selected from those shown in Table A, Table B, and Table C, preferably selected from V121I, V121L, V121M, V121F, V121A, V121G, V121E, V121P, V121R, V121S, and V121T, more preferably selected from V121L, V121A, V121E, V121G, V121P, V121R, V121S, and V121T, even more preferably selected from V121A, V121E, V121G, V121P, V121R, V121S, and V121T;
(5) a mutation at Q122 to any amino acid, preferably to an amino acid selected from those shown in Table A, Table B, and Table C, more preferably, the mutation is selected from Q122N, Q122S, Q122T, Q122K, Q122F, Q122K, Q122H, Q122G, Q122R, Q122S, Q122T, and Q122Y, even more preferably, the mutation is selected from Q122K, Q122F, Q122K, Q122H, Q122G, Q122R, Q122S, Q122T, Q122Y, and Q122N, most preferably, the mutation is selected from Q122K, Q122F, Q122K, Q122H, Q122G, Q122R, Q122S, Q122T, and Q122Y;
(6) a mutation at Y123 to any amino acid, preferably to an amino acid selected from those shown in Table A, Table B, and Table C, more preferably, the mutation is selected from Y123F, Y123L, Y123I, Y123S, Y123T, and Y123W, even more preferably selected from Y123F, Y123L, Y123I, Y123S, Y123T, F, most preferably selected from Y123F, Y123L, Y123I, Y123S, and Y123T;
(7) a mutation at K241 to any amino acid, preferably to an amino acid selected from those shown in Table A, Table B, and Table C, more preferably, the mutation is selected from K241A, K241R, K241Q, K241N, K241H, K241A, and K241R, even more preferably selected from K241A;
(8) a mutation at S242 to any amino acid, preferably to an amino acid selected from those shown in Table A, Table B, and Table C, more preferably, the mutation is selected from S242T, S242N, S242Q, S242A, S242F, and S242Y, even more preferably selected from S242F, S242Y, and S242T, most preferably selected from S242F and S242Y;
(9) a mutation at S243 to any amino acid, preferably to an amino acid selected from those shown in Table A, Table B, and Table C, more preferably, the mutation is selected from S243A, S243C, S243N, S243Q, and S243T, even more preferably selected from S243A, S243C, and S243T, most preferably selected from S243A and S243C;
(10) a mutation at S246 to any amino acid, preferably to an amino acid selected from those shown in Table A, Table B, and Table C, more preferably, the mutation is selected from S246S, S246T, S246N, S246Q, S246A, and S246R, even more preferably selected from S246R and S246T;
(11) a mutation at L272 to any amino acid, preferably to an amino acid selected from those shown in Table A, Table B, and Table C, more preferably, the mutation is selected from L272I, L272V, L272M, L272A, L272F, L272H, and L272G, even more preferably selected from L272H and L272I, further preferably selected from L272H;
(12) a mutation at S275 to any amino acid, preferably to an amino acid selected from those shown in Table A, Table B, and Table C, more preferably, the mutation is selected from S275T, S275A, S275R, S275N, and S275Q, even more preferably selected from S275T and S275R, further preferably selected from S275R;
(13) a mutation at C277 to any amino acid, preferably to an amino acid selected from those shown in Table A, Table B, and Table C, more preferably, the mutation is selected from C277S, C277M, C277L, and C277P, preferably selected from C277S and C277L, even more preferably selected from C277L;
(14) a mutation at T285 to any amino acid, preferably to an amino acid selected from those shown in Table A, Table B, and Table C, more preferably, the mutation is selected from T285A, T285S, T285R, T285N, and T285Q, even more preferably selected from T285S and T285R, more preferably selected from T285R;
(15) a mutation at S286 to any amino acid, preferably to an amino acid selected from those shown in Table A, Table B, and Table C, more preferably, the mutation is selected from S286A, S286T, S286F, S286N, and S286Q, even more preferably selected from S286F and S286T, further preferably selected from S286F;
(16) a mutation at C287 to any amino acid, preferably to an amino acid selected from those shown in Table A, Table B, and Table C, more preferably, the mutation is selected from C287S, C287M, C287I, and C287P, even more preferably selected from C287S and C287I, further preferably selected from C287I;
(17) a mutation at D288 to any amino acid, preferably to an amino acid selected from those shown in Table A, Table B, and Table C, more preferably, the mutation is selected from D288W and D288E, even more preferably selected from D288W;
(18) a mutation at E289 to any amino acid, preferably to an amino acid selected from those shown in Table A, Table B, and Table C, more preferably, the mutation is selected from E289D, E289A, E289L, E289R, and E289S, preferably selected from E289A, E289L, E289R, and E289S;
(19) a mutation at A290 to any amino acid, preferably to an amino acid selected from those shown in Table A, Table B, and Table C, more preferably, the mutation is selected from A290L, A290I, A290N, A290H, A290V, A290G, A290S, and A290T, even more preferably selected from A290N, A290H, and A290V;
(20) a mutation at I292 to any amino acid, preferably to an amino acid selected from those shown in Table A, Table B and Table C, more preferably, the mutation is selected from I292L, I292V, I292M, I292A, I292F, and I292G, and even more preferably from I292L and I292V;
(21) a mutation at K299 to any amino acid, preferably to an amino acid selected from those shown in Table A, Table B and Table C, more preferably, the mutation is selected from K299R, K299Q, K299N, and K299H, and even more preferably from K299R;
(22) a mutation at R300 to any amino acid, preferably to an amino acid selected from those shown in Table A, Table B and Table C, more preferably, the mutation is selected from R300K, R300Q, R300N, R300D, R300H, and even more preferably from R300K and R300D, and further preferably from R300D;
(23) a mutation at W301 to any amino acid, preferably to an amino acid selected from those shown in Table A, Table B and Table C, more preferably, the mutation is selected from W301Y and W301F, and even more preferably from W301Y and W301F, and further preferably from W301F;
(24) a mutation at L394 to any amino acid, preferably to an amino acid selected from those shown in Table A, Table B and Table C, more preferably, the mutation is selected from L394I, L394V, L394M, L394A, L394F, L394A, L394H, L394R, L394P, and L394G, and even more preferably from L394I, L394A, L394H, L394R, and L394P, and more preferably from L394A, L394H, L394R, and L394P;
(25) a mutation at D395 to any amino acid, preferably to an amino acid selected from those shown in Table A, Table B and Table C, more preferably, the mutation is selected from D395E, D395C, D395V, D395P, and D395S, and even more preferably from D395C, D395V, D395P, and D395S;
(26) a mutation at R396 to any amino acid, preferably to an amino acid selected from those shown in Table A, Table B and Table C, more preferably, the mutation is selected from R396K, R396Q, R396N, R396G, R396T, R396Y, and R396H, and even more preferably from R396K, R396G, R396T, and R396Y, and further preferably from R396G, R396T, and R396Y;
(27) a mutation at D397 to any amino acid, preferably to an amino acid selected from those shown in Table A, Table B and Table C, more preferably, the mutation is selected from D397E, D397F, D397H, D397N, D397R, D397S, and D397T, and even more preferably from D397F, D397H, D397N, D397R, D3975, and D397T;
(28) a mutation at R398 to any amino acid, preferably to an amino acid selected from those shown in Table A, Table B and Table C, more preferably, the mutation is selected from R398K, R398Q, R398N, R398A, R398D, R398G, R398N, R398I, R398S, and R398H, and even more preferably from R398A, R398D, R398G, R398N, R398I, R398S, and R398K, and further preferably from R398A, R398D, R398G, R398N, R398I, and R398S;
(29) a mutation at K399 to any amino acid, preferably to an amino acid selected from those shown in Table A, Table B and Table C, more preferably, the mutation is selected from K399R, K399Q, K399N, K399D, K399P, K399L, K399I, K399V, K399E, K399R, K3995, K399T, K399P, and K399H, and even more preferably from K399R, K399D, K399P, K399L, K399I, K399V, K399E, K399R, K399S, K399T, and K399P, and further preferably from K399D, K399P, K399L, K399I, K399V, K399E, K399R, K399S, K399T, and K399P;
(30) a mutation at E400 to any amino acid, preferably to an amino acid selected from those shown in Table A, Table B and Table C, more preferably, the mutation is selected from E400D, E400A, E400C, E400F, E400P, E400R, E400S, E400T, and E400V, and further preferably from E400A, E400C, E400F, E400P, E400R, E400S, E400T, and E400V;
(31) a mutation at D401 to any amino acid, preferably to an amino acid selected from those shown in Table A, Table B, and Table C, more preferably selected from D401E, D401C, D401F, D401H, D401G, D401M, D401K, D401P, D401R, D401S, D401T, and D401V, further preferably selected from D401C, D401F, D401H, D401G, D401M, D401K, D401P, D401R, D401S, D401T, and D401V;
(32) a mutation at E402 to any amino acid, preferably to an amino acid selected from those shown in Table A, Table B, and Table C, more preferably selected from E402D, E402A, E402F, E402G, E402L, E402I, E402R, E402S, E402T, and E402Y, further preferably selected from E402A, E402F, E402G, E402L, E402I, E402R, E402S, E402T, and E402Y;
(33) a mutation at D403 to any amino acid, preferably to an amino acid selected from those shown in Table A, Table B, and Table C, more preferably selected from D403E, D403G, D403P, D403R, D403S, and D403T, even more preferably selected from D403G, D403P, D403R, D403S, and D403T;
(34) a mutation at S404 to any amino acid, preferably to an amino acid selected from those shown in Table A, Table B, and Table C, more preferably selected from S404A, S404T, S404K, S404Q, S404R, S404P, S404Y, and S404N, even more preferably selected from S404T, S404K, S404Q, S404R, S404P, and S404Y, further preferably selected from S404K, S404Q, S404R, S404P, and S404Y;
(35) a mutation at C405 to any amino acid, preferably to an amino acid selected from those shown in Table A, Table B, and Table C, more preferably selected from C405S, C405M, C405A, C405K, C405L, C405R, and C405P, even more preferably selected from C405S, C405A, C405K, C405L, C405R, and C405P, further preferably selected from C405A, C405K, C405L, C405R, and C405P;
(36) a mutation at R406 to any amino acid, preferably to an amino acid selected from those shown in Table A, Table B, and Table C, more preferably selected from R406K, R406Q, R406N, R406R, R406L, R406H, R406G, R406T, and R406P, even more preferably selected from R406K, R406L, R406H, R406G, R406T, and R406P, further preferably selected from R406L, R406H, R406G, R406T, and R406P;
(37) a mutation at F407 to any amino acid, preferably to an amino acid selected from those shown in Table A, Table B, and Table C, more preferably selected from F407L, F407V, F407I, F407A, F407Y, F407W, F407M, F407S, F407L, F407P, and F407R, even more preferably selected from F407L, F407M, F407S, F407L, F407P, and F407R, further preferably selected from F407M, F407S, F407L, F407P, and F407R;
(38) a mutation at E408 to any amino acid, preferably to an amino acid selected from those shown in Table A, Table B, and Table C, more preferably selected from E408D, E408L, E408I, E408R, and E408V, further preferably selected from E408L, E408I, E408R, and E408V;
(39) a mutation at K502 to any amino acid, preferably to an amino acid selected from those shown in Table A, Table B, and Table C, more preferably selected from K502R, K502Q, K502N, and K502H, further preferably selected from K502R;
(40) a mutation at K557 to any amino acid, more preferably selected from K502R, K502Q, and K502N, even more preferably selected from K502N;
(41) a mutation at L657 to any amino acid, preferably to an amino acid selected from those shown in Table A, Table B, and Table C, more preferably selected from L657I, L657V, L657M, L657A, L657F, and L657G, even more preferably selected from L657I and L657F, further preferably selected from L657F;
(42) a mutation at C672 to any amino acid, preferably to an amino acid selected from those shown in Table A, Table B, and Table C, more preferably selected from C672S, C672M, C672R, and C672P, even more preferably selected from C672S and C672R, further preferably selected from C672R;
(43) a mutation at P758 to any amino acid, preferably to an amino acid selected from those shown in Table A, Table B, and Table C, more preferably selected from P758L, P758A, P758C, and P758M, even more preferably selected from P758L;
(44) a mutation at L789 to any amino acid, preferably to an amino acid selected from those shown in Table A, Table B, and Table C, more preferably selected from L789I, L789V, L789M, L789A, L789F, L789S, and L789G, even more preferably selected from L789I and L789S, further preferably selected from L789S;
(45) a mutation at K827 to any amino acid, preferably to an amino acid selected from those shown in Table A, Table B, and Table C, more preferably selected from K827R, K827Q, K827N, K827E, and K827H, even more preferably selected from K827R and K827E, further preferably selected from K827E; or
(46) a mutation at V860 to any amino acid, preferably to an amino acid selected from those shown in Table A, Table B, and Table C, more preferably selected from V860I, V860L, V860M, V860F, V860A, and V860G, even more preferably selected from V860L.

In some embodiments, the mutation is selected from the group consisting of: E118D, E118K, E118I, E118Q, E118P, E118S, E118T, E118Y, G119D, G119M, G119L, G119P, G119R, G119S, D120E, D120K, D120L, D120N, D120G, D120S, D120T, V121A, V121E, V121G, V121P, V121R, V121S, V121T, Q122K, Q122F, Q122K, Q122H, Q122G, Q122R, Q122S, Q122T, Q122Y, Y123F, Y123L, Y123I, Y123S, Y123T, K241A, S242F, S242Y, S243A, S243C, S246R, L272H, S275R, C277L, T285R, S286F, C287I, D288W, E289A, E289L, E289R, E2895, A290N, A290H, A290V, I292L, I292V, K299R, R300D, W301F, L394A, L394H, L394R, L394P, D395C, D395V, D395P, D395S, R396G, R396T, R396Y, D397F, D397H, D397N, D397R, D3975, D397T, R398A, R398D, R398G, R398N, R398I, R398S, K399D, K399P, K399L, K399I, K399V, K399E, K399R, K399S, K399T, K399P, E400A, E400C, E400F, E400P, E400R, E400S, E400T, E400V, D401C, D401F, D401H, D401G, D401M, D401K, D401P, D401R, D401S, D401T, D401V, E402A, E402F, E402G, E402L, E402I, E402R, E402S, E402T, E402Y, D403G, D403P, D403R, D403S, D403T, S404K, S404Q, S404R, S404P, S404Y, C405A, C405K, C405L, C405R, C405P, R406L, R406H, R406G, R406T, R406P, F407M, F407S, F407L, F407P, F407R, E408L, E408I, E408R, E408V, K502R, K557N, L657F, C672R, P758L, L789S, K827E, V860L, or combinations thereof.

In some embodiments, the mutation comprises L272H, S275R, and C277L.

In some embodiments, the Cas protein further comprises mutations at two or more key amino acid residues related to cleavage activity in SEQ ID NO: 1, said sites being selected from E289, A290, and I292.

In some embodiments, the Cas protein further comprises mutations at key amino acid residues related to cleavage activity in SEQ ID NO: 1, selected from K399, E400, D401, E402, and D403.

In some embodiments, the Cas protein further comprises mutations at three or more key amino acid residues related to cleavage activity in SEQ ID NO: 1, selected from D403, S404, C405, R406, F407, and E408.

In some embodiments, the Cas protein further comprises mutations at four or more key amino acid residues related to cleavage activity in SEQ ID NO: 1, selected from L394, D395, R396, D397, and R398.

In some embodiments, the Cas protein further comprises mutations at five or more key amino acid residues related to cleavage activity in SEQ ID NO: 1, selected from D397, R398, K399, E400, D401, and E402.

In some embodiments, the Cas protein further comprises mutations at key amino acid residues related to cleavage activity in SEQ ID NO: 1, selected from S242, S243, and S246.

In some embodiments, the Cas protein further comprises mutations at four or more key amino acid residues related to cleavage activity in SEQ ID NO: 1, selected from E118, G119, D120, V121, Q122, and Y123.

In some embodiments, the Cas protein further comprises mutations at key amino acid residues related to cleavage activity in SEQ ID NO: 1, selected from D397, R398, E400R, D401, E402, and L789.

In some embodiments, the Cas protein further comprises mutations at key amino acid residues related to cleavage activity in SEQ ID NO: 1, selected from T285, S286, C287, D288, D403, C405, R406, F407, E408, K502, and K557.

In some embodiments, the Cas protein further comprises mutations at key amino acid residues related to cleavage activity in SEQ ID NO: 1, selected from E289, I292, K399, E400, D401, E402, and D403.

In some embodiments, the Cas protein further comprises mutations at key amino acid residues related to cleavage activity in SEQ ID NO: 1, selected from E289, A290, I292, P758, and V860.

In some embodiments, the Cas protein further comprises mutations at key amino acid residues related to cleavage activity in SEQ ID NO: 1, selected from K299, R300, W301, C672, and P758.

In some embodiments, the Cas protein further comprises mutations at key amino acid residues related to cleavage activity in SEQ ID NO: 1, selected from D397, R398, E400, D401, E402, L657, and L789.

In some embodiments, the Cas protein further comprises mutations at key amino acid residues related to cleavage activity in SEQ ID NO: 1, selected from E118, G119, D120, V121, Q122, C672, and P758.

In some embodiments, the Cas protein further comprises mutations at key amino acid residues related to cleavage activity in SEQ ID NO: 1, selected from K399, E400, D401, E402, D403, P758, and K827.

In some embodiments, the Cas protein further comprises mutations at key amino acid residues related to cleavage activity in SEQ ID NO: 1, selected from E118, G119, D120, V121, Q122, L394, D395, R396, D397, R398, C672, and P758.

In some embodiments, the Cas protein further comprises mutations at twelve or more key amino acid residues related to cleavage activity in SEQ ID NO: 1, selected from E118, G119, D120, V121, Q122, D403, S404, C405, R406, F407, E408, C672, and P758.

In some embodiments, the Cas protein further comprises mutations at key amino acid residues related to cleavage activity in SEQ ID NO: 1, selected from K241, S242, S243, S246, D397, R398, E400, D401, E402, and L789.

In some embodiments, the mutation is selected from the group consisting of:
(1) L272H+S275R+C277L;
(2) L272H+S275R+C277L+E289A+I292L;
(3) L272H+S275R+C277L+E289L+A290N+I292L;
(4) L272H+S275R+C277L+E289L+A290H+I292V;
(5) L272H+S275R+C277L+K399P+E400F+D401S+E402Y+D403P;
(6) L272H+S275R+C277L+K399I+E400C+D401T+E402A+D403P;
(7) L272H+S275R+C277L+K399V+E400P+D401V+E402L+D403S;
(8) L272H+S275R+C277L+K399E+E400A+D401C+E402R+D403P;
(9) L272H+S275R+C277L+K399R+E400V+D401G+E402T+D403R;
(10) L272H+S275R+C277L+K399T+E400P+D401R+E402F+D403S;
(11) L272H+S275R+C277L+K399P+E400A+D401R+E402F+D403T;
(12) L272H+S275R+C277L+K399S+E400P+D401H+E402T+D403T;
(13) L272H+S275R+C277L+S404Q+C405A+E408I;
(14) L272H+S275R+C277L+S404R+C405R+R406T+E408L;
(15) L272H+S275R+C277L+S404Y+C405K+R406G+F407P+E408V;
(16) L272H+S275R+C277L+D403R+S404K+C405R+R406P+F407L+E408R;
(17) L272H+S275R+C277L+D403S+C405P+R406P+F407R+E408V;
(18) L272H+S275R+C277L+L394H+D395C+R396Y+D397F+R398S;
(19) L272H+S275R+C277L+L394A+D395V+D397S+R398A;
(20) L272H+S275R+C277L+D397S+R398I+K399D+E400S+D401K+E402I;
(21) L272H+S275R+C277L+D397N+R398G+E400R+D401T+E402S;
(22) L272H+S275R+C277L+D397H+R398A+K399S+E400A+D401M+E402S;
(23) L272H+S275R+C277L+D397R+R398D+K399R+E400S+D401P+E402S;
(24) L272H+S275R+C277L+D397N+R398G+E400R+D401T+E402S;
(25) S242Y+S243A+S246R+L272H+S275R+C277L;
(26)G119P+D120L+V121S+Q122K+Y123F+L272H+S275R+C277L;
(27) E118D+G119S+D120K+V121S+Q122G+Y123F+L272H+S275R+C277L;
(28) E118Y+G119S+D120G+V121P+Q122G+Y123L+L272H+S275R+C277L;
(29) E118S+G119D+D120S+V121A+Q122F+Y123L+L272H+S275R+C277L;
(30) E118K+G119S+D120T+V121G+Q122G+Y123I+L272H+S275R+C277L;
(31) E118Q+G119S+D120S+V121T+Q122G+Y123F+L272H+S275R+C277L;
(32) E118T+G119D+D120T+V121S+Q122K+Y123I+L272H+S275R+C277L;
(33) E118I+G119P+D120E+V121P+Q122H+Y123F+L272H+S275R+C277L;
(34) E118P+G119R+D120T+V121G+Q122R+L272H+S275R+C277L;
(35) E118P+G119P+D120N+V121R+Q122R+Y123I+L272H+S275R+C277L;
(36) E118S+G119M+D120S+Q122S+Y123T+L272H+S275R+C277L;
(37) G119S+V121S+Q122K+Y123S+L272H+S275R+C277L;
(38) E118S+G119D+V121E+Q122Y+Y123T+L272H+S275R+C277L;
(39) G119L+D120S+V121S+Q122T+L272H+S275R+C277L;
(40) L272H+S275R+C277L+D397N+R398G+E400R+D401T+E402S+L789S;
(41)L272H+S275R+C277L+T285R+S286F+C287I+D288W+D403S+C405P+R406P+F407R+E408V+K502R +K557N;
(42) L272H+S275R+C277L+E289R+I292L+K399S+E400P+D401H+E402T+D403T;
(43) L272H+S275R+C277L+E289S+A290V+I292L+P758L+V860L;
(44) L272H+S275R+C277L+K299R+R300D+W301F+C672R+P758L;
(45) L272H+S275R+C277L+D397N+R398G+E400R+D401T+E402S+L657F+L789S;
(46) E118P+G119R+D120T+V121G+Q122R+L272H+S275R+C277L+C672R+P758L;
(47) L272H+S275R+C277L+K399L+E400T+D401F+E402G+D403G+P758L+K827E;
(48)E118P+G119R+D120T+V121G+Q122R+L272H+S275R+C277L+L394R+D395S+R396T+D397T+R398 N+C672R+P758L;
(49)E118P+G119R+D120T+V121G+Q122R+L272H+S275R+C277L+L394P+D395P+R396G+D397F+C672 R+P758L;
(50)E118P+G119R+D120T+V121G+Q122R+L272H+S275R+C277L+D403S+C405R+R406L+F407S+E408R +C672R+P758L;
(51)E118P+G119R+D120T+V121G+Q122R+L272H+S275R+C277L+D403S+S404P+C405L+R406H+F407 M+E408V+C672R+P758L;
(52)K241A+5242F+5243C+5246R+L272H+527SR+C277L+D397N+R398G+E400R+D401T+E4025+L7895 (numbered as in SEQ ID NO: 1).

In some embodiments, the amino acid sequence of the Cas protein variant is the same as or substantially the same as that of the wild-type Cas protein, except for the mutation (at positions 118, 119, 120, 121, 122, 123, 241, 242, 243, 246, 272, 275, 277, 285, 286, 287, 288, 289, 290, 292, 299, 300, 301, 394, 395, 396, 397, 398, 399, 400, 401, 402, 403, 404, 405, 406, 407, 408, 502, 557, 657, 672, 758, 789, 860 and/or 827).

In some embodiments, "substantially the same" means that there is a difference of at most 50 (preferably 1-20, more preferably 1-10, even more preferably 1-5) amino acids. Such difference includes amino acid substitution, deletion, or addition, and the gene-editing activity of the Cas protein variant is enhanced.

In some embodiments, the homology between the Cas protein variant and the wild-type Cas protein is at least 80%, preferably at least 85% or 90%, more preferably at least 95%, most preferably at least 98% or 99%.

In some embodiments, the Cas protein variant is generated by mutating the wild-type Cas protein.

The second aspect of the disclosure provides a protein variant which is an unnatural protein, and comprises mutations at one or more key amino acid residues related to cleavage activity in SEQ ID NO: 1 corresponding to the wild-type protein, wherein the sites are selected from the group consisting of:
the aspartic acid (D) residue at position 659; and/or
the aspartic acid (D) residue at position 711; and/or
the glutamic acid (E) residue at position 895; and/or
the aspartic acid (D) residue at position 1069.

In some embodiments, the protein variant is a variant of an effector protein in the CRISPR/Cas system.

In another preferred embodiment, compared to the cleavage activity of the wild-type protein, the cleavage activity of the protein variant on a target sequence of a target molecule complementary to the guide RNA is decreased (e.g., decreased by 50%, 60%, 70%, 80%, 90%, 95% or more) or substantially lost.

In some embodiments, the aspartic acid (D) at position 659 is mutated to any amino acid, preferably to an amino acid selected from those shown in Table A, Table B, or Table C, preferably to one or more amino acids selected from Ala (A), Val (V), Leu (L), and Ile (I), more preferably to Ala (A) or Val (V), and most preferably to Ala (A).

In some embodiments, the aspartic acid (D) at position 711 is mutated to any amino acid, preferably to an amino acid selected from those shown in Table A, Table B, or Table C, preferably to one or more amino acids selected from Ala (A), Val (V), Leu (L), and Ile (I), more preferably to Ala (A) or Val (V), and most preferably to Ala (A).

In some embodiments, the glutamic acid (E) at position 895 is mutated to any amino acid, preferably to an amino acid selected from those shown in Table A, Table B, or Table C, preferably to one or more amino acids selected from Ala (A), Val (V), Leu (L), and Ile (I), more preferably to Ala (A) or Val (V), and most preferably to Ala (A).

In some embodiments, the aspartic acid (D) at position 1069 is mutated to any amino acid, preferably to an amino acid selected from those shown in Table A, Table B, or Table C, preferably to one or more amino acids selected from Ala (A), Val (V), Leu (L), and Ile (I), more preferably to Ala (A), Val (V), and most preferably to Ala (A).

In some embodiments, the aspartic acid (D) at position 659 is mutated to alanine (A).

In some embodiments, the aspartic acid (D) at position 711 is mutated to alanine (A).

In some embodiments, the glutamic acid (E) at position 895 is mutated to alanine (A).

In some embodiments, the aspartic acid (D) at position 1069 is mutated to alanine (A).

In some embodiments, the mutation is selected from the group of D659A, D711A, E895A, D1069A, and combinations thereof.

In some embodiments, the amino acid sequence of the protein variant is any one of SEQ ID NO: 44-47.

In some embodiments, the protein variant is a polypeptide having the amino acid sequence represented by any one of SEQ ID NO: 44-47, an active fragment thereof, or a conservative variant polypeptide thereof.

In some embodiments, the amino acid sequence of the protein variant is the same as or substantially the same as the sequence of the wild-type protein or its variant according to the first aspect of the disclosure except for the mutations at for example positions 659, 711, 895, and/or 1069.

In some embodiments, "substantially the same" means that there is a difference of at most 50 (preferably 1-20, more preferably 2-18, even more preferably 3-16) amino acids. The difference includes amino acid substitution, deletion, or addition, and the cleavage activity of the protein variant is decreased.

In some embodiments, the homology between the variant and the wild-type protein is at least 80%, preferably at least 85% or 90%, more preferably at least 95%, most preferably at least 98% or 99%.

In some embodiments, the protein variant is selected from the group consisting of:
(a) a polypeptide having the amino acid sequence of any one of SEQ ID NO: 44-47;
(b) a polypeptide derived from (a) by substitution, deletion, or addition of one or more (such as 2, 3, 4, or 5) amino acid residues in any one of SEQ ID NO: 44-47, and having decreased cleavage activity.

In some embodiments, the homology of the derived polypeptide to the sequence of any one of SEQ ID NO: 44-47 is at least 60%, preferably at least 70%, more preferably at least 80%, most preferably at least 90%, such as 95%, 97%, 99%.

In some embodiments, the protein variant is generated by mutating the wild-type protein.

The third aspect of the disclosure provides a fusion protein, comprising the Cas protein according to the first aspect of the disclosure or the protein variant according to the second aspect of the disclosure; and one or more functional domains.

In some embodiments, the functional domain is selected from a localization signal, a reporter protein, a Cas protein-targeting moiety, a DNA-binding domain, an epitope tag, a transcriptional activation domain, a transcriptional repression domain, a nuclease, a deaminase domain, a methylase, a demethylase, a transcription release factor, HDAC, a polypeptide with cleavage activity, a ligase, an integrase, a transposase, a recombinase, a polymerase, and a base-excision repair inhibitor (such as uracil-DNA glycosylase inhibitor (UGI)).

In some embodiments, the functional domain includes one or more of the following enzymatic activities on the target sequence: methylase activity, demethylase activity, acetyltransferase activity, deacetylase activity, kinase activity, phosphatase activity, ubiquitin-ligase activity, deubiquitination activity, adenylation activity, deadenylation activity, SUMOylation activity, deSUMOylation activity, ribosylation activity, deribosylation activity, myristoylation activity, demyristoylation activity, glycosylation activity (e.g., from O-GlcNAc transferase), and deglycosylation activity.

In some embodiments, the functional domain is selected from an adenosine deaminase catalytic domain or a cytidine deaminase catalytic domain.

In some embodiments, the adenosine deaminase catalytic domain or cytidine deaminase catalytic domain includes one or more of ADAR1, ADAR2, APOBEC, AID, or TAD.

In some embodiments, the adenosine deaminase catalytic domain comprises an amino acid sequence having at least 80%, 82%, 85%, 87%, 90%, 92%, 95%, 96%, 97%, 98%, or 99% identity to the amino acid sequence of SEQ ID NO: 28 (the 005V1 deaminase from CN114634923A, with the amino acid sequence of SEQ ID NO: 2 in that application), and retains the deamination activity of the amino acid sequence of SEQ ID NO: 28.

In some embodiments, the amino acid sequence of the adenosine deaminase catalytic domain has amino acid additions, insertions, deletions, or substitutions relative to the amino acid sequence of SEQ ID NO: 28.

In some embodiments, the adenosine deaminase catalytic domain includes mutations Q148G+Q149M+P150R of the amino acid sequence of SEQ ID NO: 28, named deaminase 005V1-10-3 (SEQ ID NO: 29).

In some embodiments, the adenosine deaminase catalytic domain comprises an amino acid sequence having at least 80%, 82%, 85%, 87%, 90%, 92%, 95%, 96%, 97%, 98%, or 99% identity to the amino acid sequence of SEQ ID NO: 54 (the 004V1 deaminase from CN114634923A, with the amino acid sequence of SEQ ID NO: 1 in that application), and retains the deamination activity of the amino acid sequence of SEQ ID NO: 54.

In some embodiments, the amino acid sequence of the adenosine deaminase catalytic domain has amino acid additions, insertions, deletions, or substitutions relative to the amino acid sequence of SEQ ID NO: 54.

In some embodiments, the functional domain is the full-length TadA8e (SEQ ID NO: 55) or a functional fragment thereof.

In some embodiments, the localization signal includes a nuclear localization signal (NLS) and/or a nuclear export signal (NES).

In some embodiments, the sequence of the nuclear localization signal is PKKKRKV (SEQ ID NO: 41), or any one of SEQ ID NO: 35-40 and 42.

In some embodiments, the sequence of the nuclear localization signal is located at, close to, or near the end (e.g. the N-terminus or C-terminus) of the Cas protein according to the first aspect.

In some embodiments, the nuclear export signal includes protein tyrosine kinase 2 (e.g., human protein tyrosine kinase 2).

In some embodiments, the reporter protein includes glutathione-S-transferase (GST), horseradish peroxidase (HRP), chloramphenicol acetyltransferase (CAT), β-galactosidase, β-glucuronidase, and self-fluorescent protein.

In some embodiments, the self-fluorescent protein includes green fluorescent protein (e.g., GFP, GFP-2, tagGFP, turboGFP, eGFP, CopGFP, AceGFP, etc.), HcRed, DsRed, cyan fluorescent protein (e.g., eCFP, Cerulean, CyPet, AmCyanl, etc.), yellow fluorescent protein (e.g., YFP, eYFP, Citrine, Venus, YPet, PhiYFP, etc.), blue fluorescent protein (e.g., eBFP, eBFP2, Azurite, mKalamal, GFPuv, Sapphire, T-sapphire).

In some embodiments, the DNA-binding domain includes methyl-binding protein, LexADBD, and Gal4DBD.

In some embodiments, the epitope tag includes a histidine tag, a V5 tag, a FLAG tag, an influenza virus hemagglutinin tag, a Myc tag, a VSV-G tag, a thioredoxin tag, and a streptavidin tag.

In some embodiments, the transcriptional activation domain includes VP64 and/or VPR.

In some embodiments, the transcriptional repression domain includes KRAB and/or SID.

In some embodiments, the nuclease includes Fokl.

In some embodiments, the polypeptide with cleavage activity includes a polypeptide with single-stranded RNA cleavage activity, a polypeptide with double-stranded RNA cleavage activity, a polypeptide with single-stranded DNA cleavage activity, or a polypeptide with double-stranded DNA cleavage activity.

In some embodiments, the ligase includes a DNA ligase and/or an RNA ligase.

In some embodiments, the functional domain is linked to the N-terminus and/or C-terminus of the Cas protein.

In some embodiments, the functional domain is inserted between the N-terminus and C-terminus of the Cas protein.

In some embodiments, the one or more functional domains are optionally linked to the N-terminus and/or C-terminus of the Cas protein via a linker.

In some embodiments, the functional domain is inserted between the N-terminus and C-terminus of the Cas protein via a linker.

In some embodiments, the fusion protein has the following structure from the N-terminus to the C-terminus:
Z1-Z2 (I); or
Z2-Z1 (II); or
Z3-Z1-Z4 (III);
wherein Z1 is a cytosine deaminase or an adenosine deaminase;
Z2 is the Cas protein according to the first aspect of the disclosure or the Cas protein variant according to the second aspect of the disclosure;
Z3 is the N-terminal fragment of the Cas protein according to the first aspect of the disclosure or the Cas protein variant according to the second aspect of the disclosure;
Z4 is the C-terminal fragment of the Cas protein according to the first aspect of the disclosure or the Cas protein variant according to the second aspect of the disclosure;
and each "-" independently represents a bond or a linker.

In some embodiments, the fusion protein has the amino acid sequence of SEQ ID NO: 43.

The fourth aspect of the disclosure provides an isolated polynucleotide, which encodes the Cas protein according to the first aspect of the disclosure, or the protein variant according to the second aspect of the disclosure, or the fusion protein according to the third aspect of the disclosure.

In some embodiments, the polynucleotide is selected from the group consisting of:
(a) a polynucleotide having a sequence of any one of SEQ ID NO: 2 or 34;
(b) a polynucleotide having a nucleotide sequence with a homology of ≥ 70% (preferably ≥ 80%, more preferably ≥ 90%, even more preferably ≥ 95%, most preferably ≥ 99%) to the sequence of any one of SEQ ID NO: 2 or 34, and encoding the polypeptide of any one of SEQ ID NO: 1 or 43; and
(c) a polynucleotide complementary to the polynucleotide of any one of (a)-(b).

In some embodiments, the isolated polynucleotide includes a humanized sequence.

In some embodiments, the polynucleotide additionally contains auxiliary elements selected from the group consisting of a signal peptide, a secretion peptide, a tag sequence (such as 6His), or a combination thereof, flanking the ORF of the variant.

In some embodiments, the polynucleotide is selected from the group consisting of a genomic sequence, a cDNA sequence, an RNA sequence, or a combination thereof.

In some embodiments, the polynucleotide further comprises a promoter operably linked to the ORF sequence of the variant.

In some embodiments, the promoter is selected from the group consisting of a constitutive promoter, a tissue-specific promoter, an inducible promoter, or a strong promoter.

In some embodiments, the polynucleotide is a polynucleotide with codons optimized according to the codon preference of a host cell.

In some embodiments, the host cell includes a prokaryotic cell or a eukaryotic cell.

In some embodiments, the host cell is a eukaryotic cell, such as a yeast cell, a plant cell, or a mammalian cell (including human and non-human mammals).

In some embodiments, the host cell is a prokaryotic cell, such as Escherichia coli.

In some embodiments, the yeast cell is from one or more selected from Pichia yeast, Kluyveromyces yeast, or a combination thereof; preferably, the yeast cell includes Kluyveromyces yeast, more preferably Kluyveromyces marxianus and/or Kluyveromyces lactis.

In some embodiments, the host cell is selected from the group consisting of *Escherichia coli,* wheat germ cells, insect cells, SF9, Hela, HEK293, CHO, yeast cells, or a combination thereof.

The fifth aspect of the disclosure provides an isolated nucleic acid molecule, which comprises or consists of a sequence selected from the group consisting of:
(i) the sequence of SEQ ID NO: 5;
(ii) a sequence having one or more base substitutions, deletions, or additions (e.g., 1, 2, 3, 4, 5, 6, 7, 8, 9, or 10 base substitutions, deletions, or additions) relative to the sequence of SEQ ID NO: 5;
(iii) a sequence having at least 20%, at least 30%, at least 40%, at least 50%, at least 60%, at least 70%, at least 80%, at least 90%, or at least 95% sequence identity to the sequence of SEQ ID NO: 5;
(iv) a sequence that hybridizes to the sequence of any one of (i)-(iii) under stringent conditions; or
(v) a sequence complementary to the sequence of any one of (i)-(iii);

and the sequence of any one of (ii)-(v) substantially retains the biological function of the sequence from which it is derived;
for example, the isolated nucleic acid molecule is RNA;
for example, the isolated nucleic acid molecule contains a direct repeat sequence in the CRISPR/Cas system.

In some embodiments, the nucleic acid molecule comprises one or more stem-loops or an optimized secondary structure;
for example, the sequence according to any one of (ii)-(v) retains the secondary structure of the sequence from which it is derived.

In some embodiments, the nucleic acid molecule is a nucleic acid molecule with codons optimized according to the codon preference of a host cell.

In some embodiments, the nucleic acid molecule comprises or consists of a sequence selected from the group consisting of:
(a) the nucleotide sequence of SEQ ID NO: 5;
(b) a sequence that hybridizes to the sequence of (a) under stringent conditions; or
(c) a sequence complementary to the nucleotide sequence of SEQ ID NO: 5.

The sixth aspect of the disclosure provides a guide RNA (gRNA) that includes a direct repeat (DR) sequence capable of binding to the Cas protein according to the first aspect of the disclosure and a spacer sequence capable of targeting a target sequence.

The seventh aspect of the disclosure provides a complex, comprising:
(i) a protein component selected from the group consisting of the Cas protein according to the first aspect of the disclosure, or the protein variant according to the second aspect of the disclosure, or the fusion protein according to the third aspect of the disclosure, or a combination thereof; and
(ii) a nucleic acid component selected from the group consisting of the guide RNA according to the sixth aspect of the disclosure, a nucleic acid encoding the guide RNA according to the sixth aspect of the disclosure, a precursor RNA of the guide RNA according to the sixth aspect of the disclosure, a nucleic acid encoding the precursor RNA of the guide RNA according to the sixth aspect of the disclosure, or a combination thereof;
wherein the protein component and the nucleic acid component bind to each other to form a complex.

In some embodiments, the direct repeat (DR) sequence in the guide RNA (gRNA) is linked to the 3'-end or 5'-end of the nucleic acid molecule.

In some embodiments, the spacer sequence in the guide RNA (gRNA) contains a sequence complementary to the target sequence.

The eighth aspect of the disclosure provides a vector, which comprises the polynucleotide according to the fourth aspect of the disclosure or the nucleic acid molecule according to the fifth aspect of the disclosure.

In some embodiments, the vector comprises:
(1) a first regulatory element, which is operably linked to a nucleotide sequence encoding the Cas protein according to the first aspect of the disclosure, or a nucleotide sequence encoding the protein variant according to the second aspect of the disclosure, or a nucleotide sequence encoding the fusion protein according to the third aspect of the disclosure; and
(2) a second regulatory element, which is operably linked to a nucleotide sequence encoding a guide RNA, wherein the guide RNA comprises:
   (a) a spacer sequence capable of hybridizing to a target sequence, and
   (b) a direct repeat (DR) sequence linked to the spacer sequence and capable of guiding the Cas protein variant according to the first aspect of the disclosure or the protein variant according to the second aspect of the disclosure to bind to the guide RNA to form the complex according to the seventh aspect of the disclosure targeting the target sequence.

In some embodiments, the first regulatory element and the second regulatory element are located on the same vector or on different vectors.

In some embodiments, the first regulatory element and/or the second regulatory element is a promoter, such as an inducible promoter, a constitutive promoter, a ubiquitous promoter, and a cell-type or tissue-specific promoter.

In some embodiments, the vector comprises one or more promoters, and the promoters are operably linked to the nucleic acid sequence, an enhancer, a transcription termination signal, a polyadenylation sequence, an origin of replication, a selectable marker, a nucleic acid restriction site, and/or a homologous recombination site.

In some embodiments, the vector includes a plasmid or a viral vector.

In some embodiments, the viral vector is selected from the group consisting of an adeno-associated virus (AAV), an adenovirus, a lentivirus, a retrovirus, a herpesvirus, SV40, a poxvirus, or a combination thereof.

In some embodiments, the vector includes a cloning vector, a transformation vector, an expression vector, a shuttle vector, an integration vector, and a multifunctional vector.

The ninth aspect of the disclosure provides a CRISPR-Cas composition, comprising:
(i) a first component selected from the group consisting of the Cas protein according to the first aspect of the disclosure, the protein variant according to the second aspect of the disclosure, the fusion protein according to the third aspect of the disclosure, a nucleotide sequence encoding the Cas protein according to the first aspect of the disclosure or the protein variant according to the second aspect of the disclosure or the fusion protein according to the third aspect of the disclosure, and any combination thereof; and
(ii) a second component, which is a nucleotide sequence comprising one or more guide RNAs according to the sixth aspect of the disclosure, or encoding the one or more guide RNAs according to the sixth aspect of the disclosure;
wherein the guide RNA is capable of forming a complex with the protein or fusion protein described in (i).

In some embodiments, the guide RNA comprises a direct repeat sequence and a spacer sequence from the 5' to 3' end, and the spacer sequence is capable of hybridizing to the target sequence.

In some embodiments, the composition further includes a pharmaceutically acceptable carrier.

In some embodiments, the composition includes a pharmaceutical composition.

In some embodiments, the dosage form of the composition is selected from the group consisting of a freeze-dried preparation, a liquid preparation, or a combination thereof.

In some embodiments, the dosage form of the composition is a liquid.

In some embodiments, the dosage form of the composition is an injection.

In some embodiments, the composition is a cell preparation.

The tenth aspect of the disclosure provides a CRISPR-Cas system, comprising one or more vectors comprising:
(i) a first nucleic acid, which is a nucleotide sequence encoding the Cas protein according to the first aspect of the disclosure, or the protein variant according to the second aspect of the disclosure, or the fusion protein according to the third aspect of the disclosure; optionally, the first nucleic acid being operably linked to a first regulatory element; and
(ii) a second nucleic acid, which is a nucleotide sequence encoding the guide RNA according to the sixth aspect of the disclosure; optionally, the second nucleic acid being operably linked to a second regulatory element;
wherein:
the first nucleic acid and the second nucleic acid are present on the same vector or on different vectors; and
the guide RNA is capable of forming a complex with the protein or fusion protein described in (i).

In some embodiments, the vector includes a plasmid or a viral vector.

In some embodiments, the guide RNA includes a spacer sequence capable of hybridizing to the target sequence; and a direct repeat (DR) sequence linked to the spacer sequence and capable of guiding the protein to bind to the guide RNA, thereby forming a CRISPR-Cas composition or complex targeting the target sequence.

In some embodiments, the guide RNA includes unmodified and modified guide RNAs.

In some embodiments, the modified guide RNA includes chemical modifications of bases.

In some embodiments, the chemical modifications include methylation modification, methoxy modification, fluorination modification, or thio modification.

In some embodiments, the first regulatory element and/or the second regulatory element is a promoter, such as an inducible promoter.

In some embodiments, at least one component in the composition is unnaturally occurring or modified.

In some embodiments, the spacer sequence is linked to the 3'-end of the direct repeat (DR) sequence.

In some embodiments, the spacer sequence contains a sequence complementary to the target sequence.

In some embodiments, when the target sequence is DNA, the target sequence is located at the 3'-end of the proto-spacer adjacent motif (PAM), and the PAM has a sequence with 5'-PAM being TTN, where N is A, T, C, or G.

In some embodiments, the target sequence is a DNA from a prokaryotic cell or a eukaryotic cell, or a DNA sequence reverse-transcribed from an RNA; alternatively, the target sequence is an unnaturally occurring DNA or a DNA sequence reverse-transcribed from an RNA.

In some embodiments, the target sequence includes a cDNA sequence.

In some embodiments, the target sequence includes a single-stranded DNA or a double-stranded DNA sequence.

In some embodiments, the target sequence is within a cell.

In some embodiments, the target sequence is within the nucleus or cytoplasm (e.g. in an organelle) of a cell.

In some embodiments, the cell is a eukaryotic cell.

In some embodiments, the cell is a prokaryotic cell.

In some embodiments, the target sequence is outside the cell.

In some embodiments, the Cas protein according to the first aspect of the disclosure is linked to one or more NLS sequences, or the fusion protein comprises one or more NLS sequences.

In some embodiments, the NLS sequence is linked to the N-terminus or C-terminus of the Cas protein according to the first aspect of the disclosure.

In some embodiments, the NLS sequence is fused to the N-terminus or C-terminus of the Cas protein according to the first aspect of the disclosure.

The eleventh aspect of the disclosure provides a kit, which includes one or more components selected from the group consisting of: the Cas protein according to the first aspect of the disclosure, the protein variant according to the second aspect of the disclosure, the fusion protein according to the third aspect of the disclosure, the polynucleotide according to the fourth aspect of the disclosure, the complex according to the seventh aspect of the disclosure, the vector according to the eighth aspect of the disclosure, the CRISPR-Cas composition according to the ninth aspect of the disclosure, or the system according to the tenth aspect of the disclosure.

In some embodiments, the kit further includes labels or instructions.

In some embodiments, the kit is used for one or more of gene editing or genome editing, disease treatment, targeting of a target gene, and cleaving of a target or non-target gene.

The twelfth aspect of the disclosure provides a delivery composition, which comprises a delivery vector, and one or more components selected from the group consisting of: the Cas protein according to the first aspect of the disclosure, the protein variant according to the second aspect of the disclosure, the fusion protein according to the third aspect of the disclosure, the polynucleotide according to the fourth aspect of the disclosure, the complex according to the seventh aspect of the disclosure, the vector according to the eighth aspect of the disclosure, the CRISPR-Cas composition according to the ninth aspect of the disclosure, or the system according to the tenth aspect of the disclosure.

In some embodiments, the delivery vector is a particle.

In some embodiments, the delivery vector is selected from lipid particles, sugar particles, metal particles, protein particles, liposomes, exosomes, microbubbles, gene guns, or viral vectors (e.g., replication-defective retroviruses, lentiviruses, adenoviruses, or adeno-associated viruses).

The thirteenth aspect of the disclosure provides a host cell, which comprises the Cas protein according to the first aspect of the disclosure, the protein variant according to the second aspect of the disclosure, the fusion protein according to the third aspect of the disclosure, the polynucleotide according to the fourth aspect of the disclosure, the complex according to the seventh aspect of the disclosure, the vector according to the eighth aspect of the disclosure, the composition according to the ninth aspect of the disclosure, the system according to the tenth aspect of the disclosure, or the delivery composition according to the twelfth aspect of the disclosure.

In some embodiments, the host cell is a eukaryotic cell, such as a yeast cell, a plant cell, or a mammalian cell (including human and non-human mammals).

In some embodiments, the host cell is a prokaryotic cell, such as *Escherichia coli.*

In some embodiments, the yeast cell is from one or more selected from the group consisting of Pichia yeast, Kluyveromyces yeast, or a combination thereof; preferably, the yeast cell includes Kluyveromyces yeast, more preferably Kluyveromyces marxianus and/or Kluyveromyces lactis.

In some embodiments, the host cell is selected from the group consisting of *Escherichia coli,* wheat germ cells, insect cells, SF9, Hela, HEK293, CHO, yeast cells, or a combination thereof.

The fourteenth aspect of the disclosure provides an enzyme preparation, which includes the Cas protein according to the first aspect of the disclosure, the protein variant according to the second aspect of the disclosure, the fusion protein according to the third aspect of the disclosure, the complex according to the seventh aspect of the disclosure, the CRISPR-Cas composition according to the ninth aspect of the disclosure, the system according to the tenth aspect of the disclosure, or the delivery composition according to the twelfth aspect of the disclosure.

In some embodiments, the enzyme preparation includes an injection and/or a freeze-dried preparation.

The fifteenth aspect of the disclosure provides a medicine kit, which includes:
a first container, and
within the first container, the complex according to the seventh aspect of the disclosure, or the composition according to the ninth aspect of the disclosure, or the system according to the tenth aspect of the disclosure; or a drug containing the complex according to the seventh aspect of the disclosure, or the composition according to the ninth aspect of the disclosure, or the system according to the tenth aspect of the disclosure.

In some embodiments, the drug in the first container is a single-active-ingredient preparation comprising the complex according to the seventh aspect of the disclosure, or the composition according to the ninth aspect of the disclosure, or the system according to the tenth aspect of the disclosure.

In some embodiments, the dosage form of the drug is selected from the group consisting of a freeze-dried preparation, a liquid preparation, or a combination thereof.

In some embodiments, the dosage form of the drug is an oral dosage form or an injection.

In some embodiments, the medicine kit further contains instructions.

The sixteenth aspect of the disclosure provides a medicine kit, which includes:
(a1) a first container, and
   within the first container, the Cas protein according to the first aspect of the disclosure, or the protein variant according to the second aspect of the disclosure, or the fusion protein according to the third aspect of the disclosure, or its encoding gene or its expression vector; or a drug containing the Cas protein according to the first aspect of the disclosure, the protein variant according to the second aspect of the disclosure, or the fusion protein according to the third aspect of the disclosure, or its encoding gene or its expression vector; and
optionally (b1) a second container, and within the second container, the guide RNA according to the sixth aspect of the disclosure or its expression vector, or a drug containing the guide RNA according to the sixth aspect of the disclosure or its expression vector.

In some embodiments, the first container and the second container are different containers.

In some embodiments, the drug in the first container is a single-active-ingredient preparation comprising the Cas protein according to the first aspect of the disclosure, the protein variant according to the second aspect of the disclosure, or the fusion protein according to the third aspect of the disclosure, or its encoding gene or its expression vector.

In some embodiments, the drug in the second container is a single-active-ingredient preparation comprising the guide RNA according to the sixth aspect of the disclosure or its expression vector.

In some embodiments, the dosage form of the drug is selected from the group consisting of a freeze-dried preparation, a liquid preparation, or a combination thereof.

In some embodiments, the dosage form of the drug is an oral dosage form or an injection.

In some embodiments, the medicine kit further contains instructions.

The seventeenth aspect of the disclosure provides a method for targeting and editing a target gene or cleaving a target gene, comprising: contacting the Cas protein according to the first aspect of the disclosure, the protein variant according to the second aspect of the disclosure, or the fusion protein according to the third aspect of the disclosure, or the complex according to the seventh aspect of the disclosure, or the composition according to the ninth aspect of the disclosure, or the system according to the tenth aspect of the disclosure, or the delivery composition according to the twelfth aspect of the disclosure, or the enzyme preparation according to the fourteenth aspect of the disclosure, or the medicine kit according to the fifteenth or sixteenth aspect of the disclosure with the target gene, or delivering it into a cell containing the target gene,
wherein the target sequence is in the target gene.

In some embodiments, the target gene is within a cell.

In some embodiments, the cell is a prokaryotic cell.

In some embodiments, the cell is a eukaryotic cell, such as a mammalian cell (e.g., a human cell) or a plant cell.

In some embodiments, the target gene is in an *in vitro* nucleic acid molecule (e.g. a plasmid).

In some embodiments, the editing or cleaving the target gene comprises breaking the target sequence, such as breaking the double strand of a DNA or the single strand of an RNA, or inserting an exogenous nucleic acid into the break.

In some embodiments, the target gene includes a DNA.

In some embodiments, the DNA includes a single-stranded DNA or a double-stranded DNA.

The eighteenth aspect of the disclosure provides a method for inducing a change in the state of a cell, comprising contacting the Cas protein according to the first aspect of the disclosure, the protein variant according to the second aspect of the disclosure, or the fusion protein according to the third aspect of the disclosure, or the complex according to the seventh aspect of the disclosure, or the composition according to the ninth aspect of the disclosure, or the system according to the tenth aspect of the disclosure, or the delivery composition according to the twelfth aspect of the disclosure, or the enzyme preparation according to the fourteenth aspect of the disclosure, or the medicine kit according to the fifteenth or sixteenth aspect of the disclosure with the target gene in the cell.

The nineteenth aspect of the disclosure provides a method for altering the expression of a gene product, comprising: contacting the Cas protein according to the first aspect of the disclosure, the protein variant according to the second aspect of the disclosure, or the fusion protein according to the third aspect of the disclosure, or the complex according to the seventh aspect of the disclosure, or the composition according to the ninth aspect of the disclosure, or the system according to the tenth aspect of the disclosure, or the delivery composition according to the twelfth aspect of the disclosure, or the enzyme preparation according to the fourteenth aspect of the disclosure, or the medicine kit according to the fifteenth or sixteenth aspect of the disclosure with a nucleic acid molecule encoding the gene product, or delivering it into a cell containing the nucleic acid molecule, wherein the target sequence is in the nucleic acid molecule.

In some embodiments, the nucleic acid molecule is in an *in vitro* nucleic acid molecule (e.g., a plasmid).

In some embodiments, the expression of the gene product is altered (e.g., enhanced or decreased).

In some embodiments, the gene product is a protein.

In some embodiments, the protein, fusion protein, polynucleotide, isolated nucleic acid molecule, complex, vector, or composition is contained in a delivery vector.

In some embodiments, the delivery vector is selected from lipid particles, sugar particles, metal particles, protein particles, liposomes, exosomes, and viral vectors (such as replication-defective retroviruses, lentiviruses, adenoviruses, or adeno-associated viruses).

In some embodiments, one or more target sequences in the target gene or the nucleic acid molecule encoding the target gene product are altered to modify a cell, a cell line, or an organism.

The twentieth aspect of the disclosure provides a cell or its progeny obtained by the method according to any one of the seventeenth to nineteenth aspects of the disclosure, wherein the cell contains a modification that is not present in its wild-type form, or contains a modification that is not present in a corresponding cell unmodified by the method; or the modified cell has resolved or improved its abnormal traits.

The twenty-first aspect of the disclosure provides a cell product of the cell or its progeny according to the twentieth aspect of the disclosure.

The twenty-second aspect of the disclosure provides an *in vitro, ex vivo,* or *in vivo* cell or cell line or their progeny, wherein the cell or cell line or their progeny contains: the Cas protein according to the first aspect of the disclosure, the protein variant according to the second aspect of the disclosure, the fusion protein according to the third aspect of the disclosure, the polynucleotide according to the fourth aspect of the disclosure, the complex according to the seventh aspect of the disclosure, the vector according to the eighth aspect of the disclosure, the CRISPR-Cas composition according to the ninth aspect of the disclosure, the system according to the tenth aspect of the disclosure, or the delivery composition according to the twelfth aspect of the disclosure.

In some embodiments, the cell is a prokaryotic cell.

In some embodiments, the cell is a eukaryotic cell, such as a mammalian cell (e.g., a human cell) or a plant cell.

In some embodiments, the cell is a stem cell or a stem cell line.

The twenty-third aspect of the disclosure provides the use of the Cas protein according to the first aspect of the disclosure, the protein variant according to the second aspect of the disclosure, the fusion protein according to the third aspect of the disclosure, the polynucleotide according to the fourth aspect of the disclosure, the complex according to the seventh aspect of the disclosure, the vector according to the eighth aspect of the disclosure, the CRISPR-Cas composition according to the ninth aspect of the disclosure, the system according to the tenth aspect of the disclosure, the kit according to the eleventh aspect of the disclosure, the delivery composition according to the twelfth aspect of the disclosure, the enzyme preparation according to the fourteenth aspect of the disclosure, or the medicine kit according to the fifteenth or sixteenth aspect of the disclosure, for the manufacture of a medicament or formulation for nucleic acid editing (e.g. gene editing or genome editing).

In some embodiments, the gene editing or genome editing includes modifying a gene, knocking out a gene, altering the expression of a gene product, repairing a mutation, and/or inserting a polynucleotide.

The twenty-fourth aspect of the disclosure provides the use of the Cas protein according to the first aspect of the disclosure, the protein variant according to the second aspect of the disclosure, the fusion protein according to the third aspect of the disclosure, the polynucleotide according to the fourth aspect of the disclosure, the complex according to the seventh aspect of the disclosure, the vector according to the eighth aspect of the disclosure, the CRISPR-Cas composition according to the ninth aspect of the disclosure, the system according to the tenth aspect of the disclosure, the kit according to the eleventh aspect of the disclosure, the delivery composition according to the twelfth aspect of the disclosure, the enzyme preparation according to the fourteenth aspect of the disclosure, or the medicine kit according to the fifteenth or sixteenth aspect of the disclosure, for the manufacture of a medicament or formulation for one or more of:
*(i) ex vivo* gene editing or genome editing;
(ii) *ex vivo* detection of a single-stranded DNA;
(iii) modifying a living organism or non-human organism by editing a target sequence in a target locus;
(iv) treating a disorder caused by a defect in a target sequence in a target locus; and
(v) treating a disorder or disease in a subject in need.

In some embodiments, the disorder or disease includes cancer, infectious diseases, neurological diseases, ophthalmic diseases, and hearing diseases.

In some embodiments, the disease or disorder includes cystic fibrosis, Duchenne muscular dystrophy (DMD), Becker muscular dystrophy, α-1-antitrypsin deficiency, Pompe disease (glycogen storage disease type II), myotonic dystrophy, Huntington's disease, fragile X syndrome, Friedreich's ataxia, amyotrophic lateral sclerosis, hereditary chronic kidney disease, sickle-cell disease, β-thalassemia, frontotemporal dementia, Leber congenital amaurosis, hyperlipidemia, hypercholesterolemia, transthyretin amyloidosis, retinal diseases, macular degeneration, Wilms tumor, Ewing's sarcoma, neuroendocrine tumors, glioblastoma, neuroblastoma, melanoma, skin cancer, breast cancer, colon cancer, rectal cancer, prostate cancer, liver cancer, kidney cancer, pancreatic cancer, lung cancer, biliary tract cancer, cervical cancer, endometrial cancer, esophageal cancer, gastric cancer, head and neck cancer, medullary thyroid cancer, ovarian cancer, glioma, lymphoma, leukemia, myeloma, acute lymphoblastic leukemia, acute myeloid leukemia, chronic lymphocytic leukemia, chronic myeloid leukemia, Hodgkin's lymphoma, non-Hodgkin's lymphoma, and urinary bladder cancer.

In some embodiments, the disorder or disease is caused by a pathogenic point mutation.

The twenty-fifth aspect of the disclosure provides a method for detecting the presence of a target nucleic acid molecule in a sample, comprising: contacting the sample with the Cas protein according to the first aspect of the disclosure, the protein variant according to the second aspect of the disclosure, the fusion protein according to the third aspect of the disclosure, or the complex according to the seventh aspect of the disclosure, the CRISPR-Cas composition according to the ninth aspect of the disclosure, the system according to the tenth aspect of the disclosure, the kit according to the eleventh aspect of the disclosure, the delivery composition according to the twelfth aspect of the disclosure, or the enzyme preparation according to the fourteenth aspect of the disclosure, and a non-target sequence; and detecting a detectable signal generated by the cleavage of the non-target sequence to detect the target nucleic acid molecule; wherein the non-target sequence does not hybridize to the guide RNA.

In some embodiments, if the non-target sequence is cleaved by the protein in the complex, the CRISPR-Cas composition, the system, or the delivery composition, it indicates the presence of the target nucleic acid molecule in the sample; and if the non-target sequence is not cleaved by the protein in the complex, the CRISPR-Cas composition, the system, or the delivery composition, it indicates the absence of the target nucleic acid molecule in the sample.

In some embodiments, the target nucleic acid molecule is a target DNA.

In some embodiments, the target DNA includes a DNA reverse-transcribed from an RNA.

In some embodiments, the target DNA includes a cDNA.

In some embodiments, the target DNA is selected from the group consisting of a single-stranded DNA, a double-stranded DNA, or a combination thereof.

It should be understood that within the scope of the disclosure, the above-mentioned technical features of the disclosure and the technical features specifically described below (such as in the examples) can be combined with each other to formulate new or preferred technical solutions. Due to space limits, they will not be described in detail one by one here.

### DESCRIPTION OF THE DRAWINGS

Figure 1 shows the maps of the recombinant expression plasmids CasY6 (Figure 1A) and LbCpf1 (Figure 1B).
Figure 2 shows the predicted secondary structure of the direct repeat (DR) sequence corresponding to CasY6.
Figure 3 shows the map of the Target plasmid.
Figure 4 shows a comparison of editing efficiencies between CasY6 and LbCpf1 in *Escherichia coli.*
Figure 5 shows the map of the PHK09T plasmid.
Figure 6 shows a comparison of editing efficiencies between CasY6 and LbCpf1 in HEK293T cells.
Figure 7 shows four mutants of CasY6, which eliminate the catalytic activity (cleavage activity) compared with the wild-type CasY6 protein.
Figure 8 shows the detection of base-editing activity of a base editor containing dCasY6 at target sites. Figure 8A shows the single-base editing (A>G) efficiency of the base editor composed of dCasY6 at sites A2, A4, A13, and A15-A17; Figure 8B shows the single-base editing (A>G) efficiency of the base editor composed of dCasY6 at sites A7, A13, and A20.
Figure 9 shows the map of the dual-luciferase reporter plasmid.
Figure 10 shows a schematic diagram of the process of testing the cleavage activity of CasY6 mutants using the dual-luciferase reporter system.

### Detailed Description of the Embodiments

After extensive and in-depth research, the inventors have unexpectedly discovered a novel Cas protein for the first time. The Cas protein of the disclosure exhibits excellent gene-editing activity, enabling effective editing or cleavage of target genes, and can effectively treat disorders or diseases of subjects in need. Compared with the Cas enzymes disclosed in the prior art, the editing efficiency of the Cas protein of the disclosure has advantages, providing more options for base-editing tools. The base editors constructed with the Cas enzymes disclosed in the disclosure can effectively perform base-editing and have potential application prospects. Moreover, the inventors have also discovered a novel Cas protein variant for the first time. The Cas protein variant of the disclosure has equivalent or better gene-editing activity compared to the wild-type Cas protein, enabling effective editing or cleavage of target genes, and can effectively treat disorders or diseases of subjects in need. Based on this, the inventors completed the invention.

### Terms

The following examples are only used to describe the disclosure, rather than limiting it. Unless otherwise specified, the experiments and methods described in the examples are basically carried out in accordance with the conventional methods well-known in the art and described in various references.

In addition, for those examples where specific conditions are not indicated, they are carried out according to the conventional conditions or the conditions recommended by the manufacturer. Reagents or instruments for which the manufacturers are not specified are all conventional products commercially available. Those skilled in the art are aware that the examples illustrate the disclosure by way of example and are not intended to limit the scope of protection of the disclosure. All publications and other references mentioned herein are incorporated herein by reference in their entireties.

To provide a better understanding of the disclosure, certain terms are first defined. As used in this application, unless otherwise explicitly defined herein, each of the following terms shall have the meaning given below. Other definitions are set forth throughout the application.

The term "about" may refer to a value or composition within an acceptable error range for a specific value or composition determined by those of ordinary skill in the art, which partially depends on how the value or composition is measured or determined. For example, as used herein, the expression "about 100" includes all values between 99 and 101 (e.g. 99.1, 99.2, 99.3, 99.4, etc.).

As used herein, the terms "comprising" or "including (containing)" may be open-ended, semi-close-ended, or close-ended. In other words, these terms also include "essentially consisting of" or "consisting of".

Sequence identity (or homology) is determined by comparing two aligned sequences along a predetermined comparison window (which can be 50%, 60%, 70%, 80%, 90%, 95%, or 100% of the length of a reference nucleotide sequence or protein) and determining the number of positions where identical residues occur. Usually, it is expressed as a percentage. The measurement of sequence identity between nucleotide sequences is a method well-known to those skilled in the art.

### Wild-type Cas Protein

As used herein, a "wild-type Cas protein" refers to a naturally occurring Cas protein that has not been artificially modified. Its nucleotide sequence can be obtained through genetic engineering techniques, such as genome sequencing, polymerase chain reaction (PCR), etc., and its amino acid sequence can be deduced from the nucleotide sequence. In a preferred embodiment of the disclosure, the wild-type Cas protein is CasY6, represented by SEQ ID NO: 1.

### Cas Protein

In the disclosure, the Cas protein, Cas enzyme, and Cas effector protein can be used interchangeably. The term "Cas protein" is used in its broadest sense and includes wild-type Cas proteins, their derivatives or variants, analogs, and functional fragments thereof, such as oligonucleotide-binding fragments.

The term "wild-type" has the meaning commonly understood by those skilled in the art. It refers to the typical form of an organism, a strain, a gene, or a protein, or when it exists in nature its characteristics that distinguish it from mutant or variant forms. It can be isolated from natural sources and has not been intentionally artificially modified.

The terms "variant", "derivative", and "analog" refer to polypeptides that substantially retain the functions or activities of the Cas protein of the disclosure.

Generally, the derivatization of a protein does not adversely affect the desired activities of the protein (e.g., the activity of binding to guide RNA, endonuclease activity, the activity of binding to and cleaving specific sites of a target sequence under the guidance of a guide RNA), that is, the derivative of a protein has the same activity as the protein. The modified forms of "derivatives" may include deletion, insertion, modification, and/or substitution of one or more amino acids of the protein. The terms "unnaturally occurring" or "engineered" are used interchangeably and indicate human intervention.

In one aspect, the disclosure provides a Cas protein, which comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence of SEQ ID NO: 1, and substantially retains the biological function of the sequence from which it is derived.

In one embodiment, the amino acid sequence of the Cas protein has one or more amino acid substitutions, deletions, or additions compared to the amino acid sequence of SEQ ID NO: 1, and substantially retains the biological function of the sequence from which it is derived.

In one embodiment, the Cas protein comprises the amino acid sequence of SEQ ID NO: 1; or has a sequence with one or more amino acid substitutions, deletions, or additions (e.g., 1, 2, 3, 4, 5, 6, 7, 8, 9, or 10 amino acid substitutions, deletions, or additions) compared to the sequence of SEQ ID NO: 1; or has an amino acid sequence with at least 90%, 91%, 92%, 93%, 94%, 95%, 96%, 97%, 98%, 99%, or 100% amino acid sequence identity to the amino acid sequence of SEQ ID NO: 1.

In one embodiment, the protein has the amino acid sequence of SEQ ID NO: 1.

Those skilled in the art are aware that the structure of a protein can be altered without adversely affecting its activity and functionality. For example, one or more conservative amino acid substitutions can be introduced into the amino acid sequence of a protein without adversely affecting the activity and/or three-dimensional structure of the protein molecule.

Those skilled in the art are familiar with examples and embodiments of conservative amino acid substitutions. Specifically, an amino acid residue can be replaced with another amino acid residue belonging to the same category as the site to be replaced, that is, a non-polar amino acid residue can replace another non-polar amino acid residue, a polar uncharged amino acid residue can replace another polar uncharged amino acid residue, a basic amino acid residue can replace another basic amino acid residue, or an acidic amino acid residue can replace another acidic amino acid residue. Such substituted amino acid residues may or may not be encoded by the genetic code. As long as the substitution does not inactivate the biological activity of the protein, a conservative substitution in which one amino acid is replaced by another amino acid belonging to the same category falls within the scope of the disclosure. Therefore, the protein of the disclosure can comprise one or more conservative substitutions in the amino acid sequence, and these conservative substitutions are preferably generated according to Table A. In addition, the disclosure also encompasses proteins that contain one or more non-conservative substitutions, as long as the non-conservative substitutions do not significantly affect the desired functions and biological activities of the proteins of the disclosure.

Conservative amino acid substitutions can be made at one or more predicted non-essential amino acid residues. A "non-essential" amino acid residue refers to one that can be altered (deleted, substituted, or replaced) without altering the biological activity, while an "essential" amino acid residue is required for biological activity. A "conservative amino acid substitution" is a substitution in which an amino acid residue is replaced with an amino acid residue having a similar side chain. Amino acid substitutions can be made in the non-conserved regions of the Cas enzyme. Generally, such substitutions are not made to conserved amino acid residues, or to amino acid residues located within conserved motifs, wherein such residues are required for the protein's activity. However, those skilled in the art should understand that functional variants may have a few conservative or non-conservative changes in conserved regions.

**Table A**

| Original Residue | Representative Substitutes | Preferred Substitute |
|---|---|---|
| Ala(A) | Val(V); Leu(L); Ile(I) | Val(V) |
| Arg(R) | Lys(K); Gln(Q); Asn(N) | Lys(K) |
| Asn(N) | Gln(Q); His(H); Lys(K); Arg(R) | Gln(Q) |
| Asp(D) | Glu(E) | Glu(E) |
| Cys(C) | Ser(S) | Ser(S) |
| Gln(Q) | Asn(N) | Asn(N) |
| Glu(E) | Asp(D) | Asp(D) |
| Gly(G) | Pro(P); Ala(A) | Ala(A) |
| His(H) | Asn(N); Gln(Q); Lys(K); Arg(R) | Arg(R) |
| Ile(I) | Leu(L); Val(V); Met(M); Ala(A); Phe(F) | Leu(L) |
| Leu(L) | Ile(I); Val(V); Met(M); Ala(A); Phe(F) | Ile(I) |
| Lys(K) | Arg(R); Gln(Q); Asn(N) | Arg(R) |
| Met(M) | Leu(L); Phe(F); Ile(I) | Leu(L) |
| Phe(F) | Leu(L); Val; Ile(I); Ala(A); Tyr(Y) | Leu(L) |
| Pro(P) | Ala(A) | Ala(A) |
| Ser(S) | Thr(T) | Thr(T) |
| Thr(T) | Ser(S) | Ser(S) |
| Trp(W) | Tyr(Y); Phe(F) | Tyr(Y) |
| Tyr(Y) | Trp(W); Phe(F); Thr(T); Ser(S) | Phe(F) |
| Val(V) | Ile(I); Leu(L); Met(M); Phe(F); Ala(A) | Leu(L) |

Those skilled in the art are already aware that altering (substituting, deleting, truncating, or inserting) one or more amino acid residues from the N- and/or C-terminus of a protein may still preserve its functional activity. Therefore, proteins with one or more amino acid residues altered from the N-and/or C-terminus of the Cas protein of the disclosure, while retaining the desired functional activity, also fall within the scope of the disclosure. These alterations may include those introduced by modern molecular biology methods such as PCR. Such methods include PCR amplification that modifies or extends the protein-coding sequence by incorporating amino-acid-coding sequences into the oligonucleotides used in the PCR amplification.

It should be recognized that proteins can be modified in various ways, including amino-acid substitutions, deletions, truncations, and insertions, and the methods for such manipulations are generally known to those skilled in the art.

For example, variants of the amino-acid sequence of a Cas protein can be prepared by mutating the DNA. It can also be accomplished through other forms of mutagenesis and/or by directed evolution. For instance, one or more amino-acid substitutions, or deletions of one to several amino acids and/or insertions of one to several amino acids can be carried out using known mutagenesis, recombination, and/or shuffling methods in combination with relevant screening methods.

Those skilled in the art can understand that these minor amino-acid changes in the Cas protein of the disclosure can occur (e.g., naturally-occurring mutations) or be generated (e.g., using r-DNA technology) without loss of protein function or activity. If these mutations occur in the catalytic domain, active site, or other functional domains of the protein, the properties of the polypeptide may change, but the polypeptide can retain its activity. If the mutations are present not close to the catalytic domain, active site, or other functional domains, a smaller impact can be expected.

Those skilled in the art can identify the essential amino acids of the Cas protein according to methods known in the art, such as site-directed mutagenesis, protein evolution, or bioinformatics analysis. The catalytic domain, active site, or other functional domains of the protein can also be determined through physical analysis of the structure, such as by techniques like nuclear magnetic resonance, crystallography, electron diffraction, or photo-affinity labeling, in combination with the mutagenesis of putative key amino acids.

### Orthologue (ortholog)

As used herein, the term "orthologue (ortholog)" has the meaning commonly understood by those skilled in the art. For further guidance, an "orthologue" of a protein as described herein refers to a protein belonging to a different species that performs the same or similar functions as the protein of which it is an orthologue.

The nucleic acid cleavage of the disclosure includes: breakage of DNA or RNA in the target nucleic acid caused by the Cas protein (cis-cleavage), and breakage of the collateral nucleic acid substrate (single-stranded nucleic acid substrate) of DNA or RNA caused by the collateral cleavage activity of the Cas protein (i.e., non-specific or non-targeted cleavage, trans-cleavage or collateral cleavage activity). In some embodiments, the cleavage is breakage of a double-strand DNA. In some embodiments, the cleavage is breakage of a single-strand DNA or a single-strand RNA.

Trans-cleavage refers to the sustained non-specific cleavage of a non-target oligonucleotide by an activated Cas12 family protein that in certain environments remains active after binding to the target sequence. This collateral cleavage activity can be used to detect the presence of specific target oligonucleotides by using the Cas system. For example, the Cas12i system is engineered to non-specifically cleave ssDNA or transcripts. The collateral cleavage activity is used for a highly sensitive and specific nucleic acid detection platform called SHERLOCK, which can be used in many clinical diagnoses (Gootenberg, J.S. et al., Nucleic acid detection with CRISPR-Cas13a/C2c2. Science 356, 438-442 (2017)).

### Cas Protein Variants and Their Encoding Nucleic Acids

As used herein, the terms "Cas protein variant", "variant of the disclosure", "gene-editing mutant protein of the disclosure", and "mutant protein" can be used interchangeably, and all refer to an unnaturally occurring mutant Cas protein comprising mutations at one or more key amino acid sites related to cleavage activity in SEQ ID NO: 1 corresponding to the wild-type Cas protein, said sites being selected from the group consisting of:
E118, G119, D120, V121, Q122, Y123, K241, S242, S243, S246, L272, S275, C277, T285, S286, C287, D288, E289, A290, I292, K299, R300, W301, L394, D395, R396, D397, R398, K399, E400, D401, E402, D403, S404, C405, R406, F407, E408, K502, K557, L657, C672, P758, L789, K827, and V860.

The term "key amino acid" refers to, specific amino acids described herein at corresponding sites based on the wild-type Cas protein in a sequence having a homology of at least 80%, such as 84%, 85%, 90%, 92%, 95%, 98%, or 99% with the wild-type Cas protein. For example, based on the wild-type gene-editing protein, the key amino acids are:
E118, G119, D120, V121, Q122, Y123, K241, S242, S243, S246, L272, S275, C277, T285, S286, C287, D288, E289, A290, I292, K299, R300, W301, L394, D395, R396, D397, R398, K399, E400, D401, E402, D403, S404, C405, R406, F407, E408, K502, K557, L657, C672, P758, L789, K827, and V860.

The mutant protein obtained by mutating the above-mentioned key amino acids has a higher cleavage activity than the wild-type Cas protein (SEQ ID NO: 1); alternatively, the cleavage activity of the mutant protein is equivalent to that of the wild-type Cas protein, or the binding of the CRISPR composition containing the Cas protein variant to the binding site is enhanced, or the editing preference is changed.

Preferably, in the disclosure, the key amino acids of the disclosure are mutated as follows:
E118D, E118K, E118I, E118Q, E118P, E118S, E118T, E118Y, G119D, G119M, G119L, G119P, G119R, G119S, D120E, D120K, D120L, D120N, D120G, D120S, D120T, V121A, V121E, V121G, V121P, V121R, V121S, V121T, Q122K, Q122F, Q122K, Q122H, Q122G, Q122R, Q122S, Q122T, Q122Y, Y123F, Y123L, Y123I, Y123S, Y123T, K241A, S242F, S242Y, S243A, S243C, S246R, L272H, S275R, C277L, T285R, S286F, C287I, D288W, E289A, E289L, E289R, E2895, A290N, A290H, A290V, I292L, I292V, K299R, R300D, W301F, L394A, L394H, L394R, L394P, D395C, D395V, D395P, D395S, R396G, R396T, R396Y, D397F, D397H, D397N, D397R, D3975, D397T, R398A, R398D, R398G, R398N, R398I, R398S, K399D, K399P, K399L, K399I, K399V, K399E, K399R, K3995, K399T, K399P, E400A, E400C, E400F, E400P, E400R, E400S, E400T, E400V, D401C, D401F, D401H, D401G, D401M, D401K, D401P, D401R, D401S, D401T, D401V, E402A, E402F, E402G, E402L, E402I, E402R, E402S, E402T, E402Y, D403G, D403P, D403R, D403S, D403T, S404K, S404Q, S404R, S404P, S404Y, C405A, C405K, C405L, C405R, C405P, R406L, R406H, R406G, R406T, R406P, F407M, F407S, F407L, F407P, F407R, E408L, E408I, E408R, E408V, K502R, K557N, L657F, C672R, P758L, L789S, K827E, V860L.

It should be understood that the numbering of amino acids in the mutant proteins of the disclosure is based on the wild-type Cas protein. When the sequence homology between a specific mutant protein and the wild-type Cas protein reaches 80% or more, the numbering of amino acids of the mutant protein may be shifted relative to that of the wild-type Cas protein, such as being shifted by 1-100 positions towards the N-terminus or C-terminus of the amino acid sequence. Using conventional sequence alignment techniques in the art, those skilled in the art can generally understand that such a shift is within a reasonable range, and mutant proteins with a homology of 80% (such as 90%, 95%, 98%) and having the same or similar gene-editing activity (such as cleavage activity) equivalent to or higher than that of the wild-type Cas protein (SEQ ID NO: 1) should not be excluded from the scope of the mutant proteins of the disclosure simply because of the shift of numbering of amino acids.

The mutant proteins of the disclosure are synthetic or recombinant proteins, that is, they may be products of chemical synthesis or be produced from prokaryotic or eukaryotic hosts (such as bacteria, yeast, plants) by recombinant techniques. Depending on the host used in the recombinant production protocol, the mutant proteins of the disclosure may be glycosylated or non -glycosylated. The mutant proteins of the disclosure may or may not include the starting methionine residue.

The disclosure also includes fragments, derivatives, and analogs of the mutant proteins. As used herein, the terms "fragment", "derivative", and "analog" refer to proteins that essentially retain the same biological function or activity as the mutant proteins.

The fragments, derivatives, or analogs of the mutant proteins of the disclosure may be: (i) mutant proteins in which one or more conservative or non-conservative amino acid residues (preferably conservative amino acid residues) are substituted, and such substituted amino acid residues may or may not be encoded by the genetic code; (ii) mutant proteins having substituents in one or more amino acid residues; (iii) mutant proteins formed by conjugating a mature mutant protein to another compound (such as a compound that prolongs the half-life of the mutant protein, e.g., polyethylene glycol); or (iv) mutant proteins formed by fusing an additional amino acid sequence to the mutant protein sequence (such as a leader sequence, a secretion sequence, a sequence for purifying the mutant protein, a proprotein sequence, or a fusion protein formed with an IgG fragment). Based on the teachings herein, these fragments, derivatives, and analogs are within the knowledge of those skilled in the art.

In certain embodiments, other categories of amino acids that are considered to be conservative substitutes of each other are as follows.

**Table A**

| Original Residue | Representative Substitutions | Preferred Substitutions |
|---|---|---|
| Ala (A) | Val(V); Leu(L); Ile (I) | Val(V) |
| Arg (R) | Lys (K); Gln (Q); Asn(N) | Lys (K) |
| Asn (N) | Gln (Q); His (H); Lys (K); Arg (R) | Gln (Q) |
| Asp (D) | Glu (E) | Glu (E) |
| Cys (C) | Ser (S) | Ser (S) |
| Gln (Q) | Asn (N) | Asn (N) |
| Glu (E) | Asp (D) | Asp (D) |
| Gly (G) | Pro (P); Ala (A) | Ala (A) |
| His (H) | Asn (N); Gln (Q); Lys (K); Arg (R) | Arg (R) |
| Ile (I) | Leu (L); Val (V); Met (M); Ala (A); Phe (F) | Leu (L) |
| Leu (L) | Ile (I); Val (V); Met (M); Ala (A); Phe (F) | Ile (I) |
| Lys (K) | Arg (R); Gln (Q); Asn (N) | Arg (R) |
| Met (M) | Leu(L); Phe (F); Ile (I) | Leu (L) |
| Phe (F) | Leu(L); Val (V); Ile (I); Ala (A); Tyr (Y) | Leu (L) |
| Pro (P) | Ala (A) | Ala (A) |
| Ser (S) | Thr (T) | Thr (T) |
| Thr (T) | Ser (S) | Ser (S) |
| Trp (W) | Tyr (Y); Phe (F) | Tyr (Y) |
| Tyr (Y) | Trp (W); Phe (F); Thr (T); Ser (S) | Phe (F) |
| Val (V) | Ile (I); Leu (L); Met (M); Phe (F); Ala (A) | Leu (L) |

In certain embodiments, other categories of amino acids that are considered to be conservative substitutes of each other are as follows (see e.g. Creighton, "Proteins" (1984)):

**Table B**

| | |
|---|---|
| Group 1 | Ala(A), Gly(G) |
| Group 2 | Asp(D), Glu(E) |
| Group 3 | Asn(N), Gln(Q) |
| Group 4 | Arg (R), Lys(K) |
| Group 5 | Ile (I), Leu (L), Met (M),Val (V) |
| Group 6 | Phe(F), Tyr(Y), Trp(W) |
| Group 7 | Ser(S), Thr(T) |
| Group 8 | Cys(C), Met(M) |

In certain embodiments, other categories of amino acids that are considered to be conservative substitutes of each other are as follows.

**Table C**

| | |
|---|---|
| Group 1 | Ala(A), Ser(S), Thr(T) |
| Group 2 | Asp(D), Glu(E) |
| Group 3 | Asn(N), Gln(Q) |
| Group 4 | Arg(R), Lys(K) |
| Group 5 | Ile(I), Leu(L), Met(M) |
| Group 6 | Phe(F), Tyr(Y), Trp(W) |

The active mutant proteins of the disclosure have equivalent or higher gene-editing activity (such as cleavage activity) compared to the wild-type Cas protein (SEQ ID NO: 1).

In addition, the mutant proteins of the disclosure may be further modified. Such modification (usually without changing the primary structure) includes *in vivo* or *in vitro* chemical derivatization of the mutant proteins, such as acetylation or carboxylation. The modification also includes glycosylation, such as those mutant proteins generated by glycosylation during the synthesis and processing of the mutant protein or in further processing steps. This modification can be accomplished by exposing the mutant protein to a glycosylating enzyme (such as mammalian glycosyltransferases or deglycosylating enzymes). The modification also includes phosphorylation of amino acid residues (such as phosphotyrosine, phosphoserine, phosphothreonine) in a sequence. Also included are mutant proteins that have been modified to improve their anti-proteolytic properties or optimize their solubility properties.

The term "polynucleotide encoding a mutant protein" may refer to a polynucleotide that includes the sequence encoding the mutant protein of the disclosure, or a polynucleotide that also includes additional coding and/or non-coding sequences.

The disclosure also relates to variants of the above-mentioned polynucleotides, which encode polypeptides having the same amino acid sequence as those of the disclosure, or fragments, analogs, and derivatives of the mutant protein. These nucleotide variants include substitution variants, deletion variants, and insertion variants. As is known in the art, an equivalent variant is an alternative form of a polynucleotide, which may involve substitution, deletion, or insertion of one or more nucleotides, but does not substantially change the function of the mutant protein it encodes.

The disclosure also relates to polynucleotides that hybridize to the above-mentioned sequences and have at least 50%, preferably at least 70%, more preferably at least 80% identity between the two sequences. The disclosure particularly relates to polynucleotides that can hybridize to the polynucleotides of the disclosure under stringent conditions. In the context of the present disclosure, "stringent conditions" refer to: (1) hybridization and washing under low ionic strength and a high temperature, such as 0.2×SSC, 0.1% SDS, 60°C; or (2) hybridization in the presence of denaturing agents, such as 50% (v/v) formamide, 0.1% calf serum/0.1% Ficoll, 42°C, etc.; or (3) hybridization occurring only when the identity between the two sequences is at least 90% or more, preferably 95% or more.

The mutant proteins and polynucleotides of the disclosure are preferably provided in an isolated form, and more preferably purified to homogeneity.

The full-length sequence of the polynucleotide of the disclosure can generally be obtained by PCR amplification, recombination, or artificial synthesis methods. For the PCR amplification, primers can be designed according to the nucleotide sequences disclosed in the disclosure, especially the open-reading-frame sequences, and commercially available cDNA libraries or cDNA libraries prepared by conventional methods known to those skilled in the art can be used as templates for amplification to obtain the relevant sequences. When the sequence is long, it often requires two or more rounds of PCR amplification, and then the amplified fragments are spliced together in the correct order.

Once the relevant sequence is obtained, the recombination method can be used to obtain the relevant sequence in large quantities. This is usually achieved by cloning it into a vector, then transferring it into cells, and then isolating the relevant sequence from the proliferated host cells by conventional methods.

In addition, the relevant sequence can also be synthesized by artificial synthesis methods, especially when the fragment length is short. Usually, a very long fragment can be obtained by first synthesizing multiple small fragments and then ligating them.

At present, the DNA sequence encoding the protein of the disclosure (or its fragment, or its derivative) can be obtained completely by chemical synthesis. Then this DNA sequence can be introduced into various existing DNA molecules (such as vectors) and cells known in the art. In addition, mutations can also be introduced into the protein sequence of the disclosure by chemical synthesis.

The method for amplifying DNA/RNA by PCR technology is preferably used to obtain the polynucleotides of the disclosure. Especially when it is difficult to obtain the full-length cDNA from the library, the RACE method (rapid amplification of cDNA ends) can be preferably used. Primers for PCR can be appropriately selected according to the sequence information of the disclosure disclosed herein and can be synthesized by conventional methods. The amplified DNA/RNA fragments can be separated and purified by conventional methods such as gel electrophoresis.

### Fusion Protein

In one aspect, the disclosure provides a fusion protein, which comprises the Cas protein according to any one of the foregoing aspects and one or more functional domains.

In one embodiment, the functional domains include one or more of a localization signal, a reporter protein, a Cas protein targeting moiety, a DNA-binding domain, an epitope tag, a transcriptional activation domain, a transcriptional repression domain, a nuclease, a deaminase domain, a methylase, a demethylase, a transcription release factor, HDAC, a polypeptide with cleavage activity, and a ligase.

In one example, "methylase" includes, for example, Hhal DNA m5c-methyltransferase (M.Hhal), DNA methyltransferase 1 (DNMT1), DNA methyltransferase 3a (DNMT3a), DNA methyltransferase 3b (DNMT3b), METI, DRM3, ZMET2, CMT1, CMT2, etc.

"Demethylase" refers to an enzyme that removes a methyl group (CH₃-) from a nucleic acid, a protein (e.g., histone), and other molecules. Demethylases are important in epigenetic modification mechanisms. Demethylases alter the transcriptional regulation of the genome by controlling the level of methylation of DNA and histone, and further regulate the chromatin state at specific loci in an organism, such as TET1 (ten-eleven translocation 1), ten-eleven translocation (TET) dioxygenase 1 (TET1CD), DME, DML1, DML2, ROS1, etc.

In some embodiments, the transcription release factor includes, for example, eukaryotic release factor 1 (ERF1), and eukaryotic release factor 3 (ERF3).

In one embodiment, the functional domain is selected from an adenosine deaminase catalytic domain or a cytidine deaminase catalytic domain.

In one embodiment, the localization signal includes a nuclear localization signal and/or a nuclear export signal.

Preferably, the nuclear export signal includes human protein tyrosine kinase 2.

Preferably, the reporter protein includes one or more of glutathione-S-transferase, horseradish peroxidase, chloramphenicol acetyltransferase, β-galactosidase, β-glucuronidase, or a self-fluorescent protein.

Preferably, the self-fluorescent protein includes one or more of green fluorescent protein, HcRed, DsRed, cyan fluorescent protein, yellow fluorescent protein, or blue fluorescent protein.

Preferably, the DNA-binding domain includes one or more of a methyl-binding protein, LexADBD, or Gal4DBD.

Preferably, the epitope tag includes one or more of a histidine tag, a V5 tag, a FLAG tag, an influenza virus hemagglutinin tag, a Myc tag, a VSV-G tag, or a thioredoxin tag.

Preferably, the transcriptional activation domain includes VP64 and/or VPR.

Preferably, the transcriptional repression domain includes KRAB and/or SID.

Preferably, the nuclease includes Fokl.

Preferably, the deaminase domain includes one or more of ADAR1, ADAR2, APOBEC, AID, or TAD.

In one embodiment, the deaminase domain comprises an amino acid sequence having at least 60%, 65%, 70%, 75%, 80%, 85%, 90%, 91%, 92%, 93%, 94%, 95%, 96%, 97%, 98%, 99% or higher sequence identity to the amino acid sequence of any one of SEQ ID NO: 28-29 and 54-55.

In one embodiment, the functional domain is the full-length or a functional fragment of TadA8e (SEQ ID NO: 55).

Preferably, the polypeptide with cleavage activity includes a polypeptide with single-stranded RNA cleavage activity, a polypeptide with double-stranded RNA cleavage activity, a polypeptide with single-stranded DNA cleavage activity, or a polypeptide with double-stranded DNA cleavage activity.

Preferably, the ligase includes a DNA ligase and/or an RNA ligase.

### Polynucleotide

In one aspect, the disclosure provides a polynucleotide, which is a polynucleotide sequence encoding the Cas protein or a polynucleotide sequence encoding the fusion protein described above.

In one embodiment, the polynucleotide is a DNA molecule with codons optimized according to the codon preference of a host cell.

The optimization described in the present disclosure may require mutation of the nucleotide sequence encoding a protein (such as the Cas protein of the present disclosure) to mimic the codon preference of the intended host organism or cell when it encodes the same protein. Thus, the codons can be altered, but the encoded protein remains unchanged. For example, if the intended target cell is a human cell, a nucleotide sequence encoding the protein optimized with human codons can be used. As another non-limiting example, if the intended host cell is an animal cell (such as a mouse cell or an insect cell), a nucleotide sequence encoding the protein optimized with the codons of that animal can be generated. As yet another non-limiting example, if the intended host cell is a plant cell, a nucleotide sequence encoding the protein optimized with plant codons can be generated.

Choices of codons are readily available. For example, the "Codon Usage Database" is available at www.kazusa.or.jp/codon. In some cases, the nucleic acid of the present disclosure contains a nucleotide sequence encoding CasY6 or its variants or its fusion protein, and the codons of the nucleotide sequence are optimized for expression in eukaryotic cells. In some cases, the nucleic acid of the present disclosure contains a nucleotide sequence encoding CasY6 or its variants or its fusion protein, and the codons of the nucleotide sequence are optimized for expression in animal cells. In some cases, the nucleic acid of the present disclosure contains a nucleotide sequence encoding CasY6 or its variants or its fusion protein, and the codons of the nucleotide sequence are optimized for expression in fungal cells. In some cases, the nucleic acid of the present disclosure contains a nucleotide sequence encoding CasY6 or its variants or its fusion protein, and the codons of the nucleotide sequence are optimized for expression in plant cells.

In one embodiment, the host cell includes a prokaryotic cell or a eukaryotic cell.

### CRISPR System

The terms "clustered regularly interspaced short palindromic repeats (CRISPR)-CRISPR-associated (Cas) (CRISPR-Cas) system" or "CRISPR system" are used interchangeably and have the meaning commonly understood by those skilled in the art. It generally includes transcriptional products or other elements related to the expression of a CRISPR-associated ("Cas") gene, or transcriptional products or other elements capable of guiding the activity of the Cas gene.

### CRISPR-Cas Composition

In one aspect, the disclosure also provides a CRISPR-Cas composition, which comprises:
(1) a protein component: the aforementioned Cas protein, or the aforementioned fusion protein; or a nucleic acid molecule encoding the Cas protein or the fusion protein;
(2) an RNA component: guide RNA, or one or more nucleic acids encoding the guide RNA, or a precursor RNA of the guide RNA, or a nucleic acid encoding the precursor RNA of the guide RNA;
wherein the protein component and the nucleic acid component bind to each other to form a complex.

In one embodiment, the composition is an activated CRISPR complex, which further comprises: a target sequence of a target nucleic acid bound to the guide RNA.

In one embodiment, the CRISPR-Cas composition includes one or more vectors, and the one or more vectors comprise:
(1) a first regulatory element, which is operably linked to a nucleotide sequence encoding the Cas protein or a nucleotide sequence encoding the fusion protein; and
(2) a second regulatory element, which is operably linked to a nucleotide sequence encoding the guide RNA, wherein the guide RNA comprises:
   (a) a spacer sequence capable of hybridizing to a target sequence of a target nucleic acid, and
   (b) a direct repeat (DR) sequence linked to the spacer sequence and capable of guiding the Cas protein to bind to the guide RNA to form a CRISPR-Cas complex targeting the target sequence;
wherein the first regulatory element and the second regulatory element are located on the same vector or on different vectors of the CRISPR-Cas vector system.

In one embodiment, the first regulatory element or the second regulatory element includes a promoter, and the promoter includes one or more of an inducible promoter, a constitutive promoter, or a tissue-specific promoter.

In one embodiment, the promoter includes one or more of T7, SP6, T3, CMV, EF1a, SV40, PGK1, human β-actin, CAG, U6, H1, T7, T7lac, araBAD, trp, lac, or Ptac.

In one embodiment, the first regulatory element and the second regulatory element are located on the same vector or on different vectors.

In one embodiment, the vector includes a retroviral vector, a lentiviral vector, an adenoviral vector, an adeno-associated viral vector, a herpes simplex vector, or a phagemid vector.

In one embodiment, the vector includes a plasmid vector.

In one embodiment, the target nucleic acid includes a DNA derived from a eukaryote or a DNA derived from a prokaryote.

In one embodiment, the eukaryote includes an animal or a plant.

In one embodiment, the target nucleic acid includes a non-human mammalian DNA, a human DNA, an insect DNA, an avian DNA, a reptilian DNA, an amphibian DNA, a rodent DNA, a fish DNA, a worm DNA, a nematode DNA, or a yeast DNA.

In one embodiment, the non-human mammalian DNA includes a non-human primate DNA.

### CRISPR/Cas Complex

The term "CRISPR/Cas complex" refers to a complex formed by the binding of a guide RNA, a gRNA (guide RNA), or mature crRNA (or guiding RNA) to a Cas protein. It contains a direct repeat sequence that hybridizes to the guide sequence of the target sequence and binds to the Cas protein. This complex is capable of recognizing and cleaving the target nucleotide that can hybridize to the guide RNA or mature crRNA.

### Guide RNA (gRNA)

The terms "guide RNA (gRNA)", "mature crRNA", "crRNA", "guide sequence", and "guiding RNA" are used interchangeably and have the meaning commonly understood by those skilled in the art. Generally, a guide RNA can include a direct repeat (DR) sequence and a spacer sequence, or consist of or essentially consist of a direct repeat (DR) sequence and a spacer sequence.

In some cases, the spacer sequence is any polynucleotide sequence that has sufficient complementarity to the target sequence to hybridize to the target sequence and guide the specific binding of the CRISPR-Cas complex to the target sequence. In one embodiment, when optimally aligned, the degree of complementarity between the spacer sequence and its corresponding target sequence is at least 50%, at least 60%, at least 70%, at least 80%, at least 90%, at least 95%, or at least 99%. The guiding sequence contains a sequence (such as a direct repeat (DR) sequence) that has sufficient complementarity to the target nucleic acid sequence to hybridize to the target nucleic acid sequence and guide the sequence-specific binding of the complex to the target nucleic acid sequence.

It is known in the art that when the complementarity is sufficient to exert the function, complete complementarity is not required. Therefore, if necessary, the cleavage efficiency can be regulated by introducing mismatches (e.g. one or more mismatches between the spacer sequence and the target nucleic acid, such as a mismatch of 1 or 2 nucleotides (including the position of the mismatch along the spacer sequence/target sequence)). For example, if a cleavage ratio of less than 100% of the target (e.g., in a cell population) is desired, 1 or 2 mismatches between the spacer sequence and the target sequence may be introduced into the spacer sequence.

In one aspect, the disclosure provides a guide RNA, which includes a direct repeat (DR) sequence capable of binding to the Cas protein and a spacer sequence capable of targeting a target sequence.

In one embodiment, the direct repeat (DR) sequence contains the sequence of SEQ ID NO: 5.

In one embodiment, the 3'-end of the direct repeat sequence contains a stem-loop structure, and further includes a stem formed by hybridization of a first stem nucleotide strand and a second stem nucleotide strand to each other, and a loop nucleotide strand forms the loop of the stem-loop structure.

In one embodiment, the direct repeat sequence includes a nucleotide sequence having at least 80% identity to the nucleotide sequence of SEQ ID NO: 5.

In one embodiment, the direct repeat sequence includes a nucleotide sequence having at least 85% or more, more preferably 90% or more, and further preferably 95% or more identity to the nucleotide sequence of SEQ ID NO: 5.

In one embodiment, the direct repeat sequence includes the nucleotide sequence of SEQ ID NO: 5.

In one embodiment, more than 80% of the spacer sequence is complementary to the target nucleic acid.

In one embodiment, more than 90%, more preferably more than 95%, further preferably more than 99%, and still further preferably 100% of the spacer sequence is complementary to the target nucleic acid.

In one embodiment, the length of the spacer sequence is greater than 15 nucleotides, preferably 15-100 nt, more preferably 15-50 nt (e.g., 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 nt), more preferably 15-25 nt, more preferably 15-23 nt, more preferably 18-22 nt.

In one embodiment, the length of the spacer sequence is 15 nt or 18-23 nt or 25 nt, preferably 15 nt or 18-23 nt. For example, the length of the spacer sequence can be selected from 15, 18, 19, 20, 21, 22, 23, or 25 nt.

### Target Nucleic Acid

In the present disclosure, "target nucleic acid" is used interchangeably with "target sequence", "target nucleic acid sequence", or "target nucleic acid molecule", and refers to a specific nucleic acid that contains a nucleic acid sequence that is fully or partially complementary to the spacer sequence in the guide RNA. A "target sequence" refers to a polynucleotide targeted by the spacer sequence in the guide RNA, for example, a sequence complementary to the spacer sequence, where hybridization between the target sequence and the spacer sequence facilitates the formation of a CRISPR-Cas complex (including the Cas protein and the guide RNA). Complete complementarity is not required, as long as the complementarity is sufficient to cause hybridization and facilitate the formation of a CRISPR-Cas complex. In some embodiments, the target nucleic acid contains non-coding regions (e.g., a promoter or terminator). In some embodiments, the target nucleic acid is single-stranded or double-stranded.

The target sequence can contain any polynucleotide, such as DNA. In some cases, the target sequence is located inside or outside a cell. In some cases, the target sequence is located within the nucleus, the cytoplasm, or an organelle (such as mitochondrion or chloroplast) of a cell.

The target nucleic acid can be a sequence encoding a gene product (e.g. a protein) or a non-coding sequence (e.g. a regulatory polynucleotide or junk DNA). In some cases, the target sequence should be associated with a proto-spacer adjacent motif (PAM).

### Donor Template

In the present disclosure, "donor template nucleic acid" and "donor template" are used interchangeably, and refer to a nucleic acid molecule that one or more cellular proteins can use to alter the structure of a target nucleic acid after the target nucleic acid is modified by the Cas protein described herein.

In some embodiments, the donor template nucleic acid is a double-stranded nucleic acid or a single-stranded nucleic acid. In some embodiments, the donor template nucleic acid is linear or circular (e.g. a plasmid). In some instances, the donor template nucleic acid is an exogenous nucleic acid molecule. In some instances, the donor template nucleic acid is an endogenous nucleic acid molecule (e.g. a chromosome). In some embodiments, gene recombination can be achieved by using the donor template, and this recombination is homologous recombination.

### Cleavage

Cleavage refers to DNA breakage in a target nucleic acid by the Cas protein described herein. In some embodiments, the cleavage is breakage of a double-strand DNA. In some embodiments, the cleavage is breakage of a single-strand DNA.

In the present disclosure, the meanings of cleaving a target nucleic acid and modifying a target nucleic acid may overlap. Modifying a target nucleic acid includes not only modification of single nucleotides but also insertion or deletion of nucleic acid fragments.

### Reporter Nucleic Acid

A reporter nucleic acid refers to a molecule that can be cleaved or otherwise inactivated by the activated CRISPR system proteins described herein. The reporter nucleic acid contains a nucleic acid element that can be cleaved by the CRISPR protein (for example, a single-stranded non-target nucleic acid molecule with different reporter groups or labeling molecules at both ends). Cleavage of the nucleic acid element generates a detectable signal. Before cleavage, or when the reporter nucleic acid is in an "active" state, the reporter nucleic acid prevents the generation or detection of a positive detectable signal. It will be understood that in some example embodiments, a minimal background signal may be generated in the presence of an active reporter nucleic acid. The positive detectable signal can be any signal that can be detected by an optical, fluorescent, chemiluminescent, or electrochemical method, or other detection methods known in the art. For example, in some embodiments, a first signal (i.e., a negative detectable signal) can be detected when the reporter nucleic acid is present, and then it is converted to a second signal (e.g., a positive detectable signal) after the detection of a target molecule and its cleavage or inactivation by the activated CRISPR protein. The reporter nucleic acid may be a single-stranded DNA molecule, a single-stranded RNA molecule, or a single-stranded DNA-RNA hybrid.

The detection method according to the present disclosure can be used for the quantitative detection of a target nucleic acid to be detected. The quantitative detection index can be quantified according to the strength of the signal of the reporter group, such as according to the luminescence intensity of a fluorescent group, or according to the width of a color-developing band, etc.

### Functional Domain

The term "functional domain" is used herein in its broadest sense and includes proteins such as enzymes or factors themselves or their fragments/domains having a specific function. A Cas protein (e.g., a dCas protein) is linked to/associated with one or more functional domains, and the functional domains are selected from one or more of a localization signal, a reporter protein, a Cas protein targeting moiety, a DNA-binding domain, an epitope tag, a transcriptional activation domain, a transcriptional repression domain, a nuclease, a deaminase domain, a methylase, a demethylase, a transcription release factor, HDAC, a polypeptide with cleavage activity, and a ligase. When more than one functional domain is included, the functional domains may be the same or different.

### Deaminase Domain

In the present disclosure, the deaminase domain includes the catalytic domain of a deaminase (such as adenosine deaminase or cytidine deaminase). As used herein, an "adenosine deaminase" or "adenosine deaminase protein" refers to a protein, polypeptide, or one or more functional domains of a protein or polypeptide that are capable of catalyzing the hydrolytic deamination reaction that converts adenine (or the adenine moiety of a molecule) into hypoxanthine (or the hypoxanthine moiety of a molecule).

In some embodiments, the adenine-containing molecule is adenosine (A), and the hypoxanthine-containing molecule is inosine (I). The adenine-containing molecule may be deoxyribonucleic acid (DNA) or ribonucleic acid (RNA).

Adenosine deaminases include, but are not limited to, members of the enzyme family called adenosine deaminases acting on RNA (ADAR), members of the enzyme family called adenosine deaminases acting on tRNA (ADAT), and members of other families containing adenosine deaminase domains (ADAD). According to the present disclosure, adenosine deaminases are capable of targeting adenine in RNA/DNA and RNA duplexes. In certain embodiments, adenosine deaminases have been modified to increase their ability to edit DNA in RNA/DNA heteroduplexes of RNA duplexes.

In some examples, the deaminase is a cytidine deaminase. The term "cytidine deaminase" or "cytidine deaminase protein" refers to a protein, polypeptide, or one or more functional domains of a protein or polypeptide that are capable of catalyzing the hydrolytic deamination reaction that converts cytosine (or the cytosine moiety of a molecule) into uracil (or the uracil moiety of a molecule). In some embodiments, the cytosine-containing molecule is cytidine (C), and the uracilcontaining molecule is uridine (U). The cytosine-containing molecule may be deoxyribonucleic acid (DNA) or ribonucleic acid (RNA).

Cytidine deaminases include, but are not limited to, members of the enzyme family called the apolipoprotein B mRNA-editing complex (APOBEC) family of deaminases, activation-induced deaminase (AID), or cytidine deaminase 1 (CDA1). In certain embodiments, deaminases of the APOBEC family are included.

In some embodiments, the cytidine deaminase comprises the wild-type amino acid sequence of cytosine deaminase. In some embodiments, the cytidine deaminase contains one or more mutations in the cytosine deaminase sequence such that the editing efficiency and/or substrate editing preference of the cytosine deaminase is altered according to specific needs.

In one embodiment, the deaminase domain comprises an amino acid sequence having at least 60%, 65%, 70%, 75%, 80%, 85%, 90%, 91%, 92%, 93%, 94%, 95%, 96%, 97%, 98%, 99% or higher sequence identity to the amino acid sequence of any one of SEQ ID NO: 28, 29, and 54-55.

In one embodiment, the functional domain is the full-length or a functional fragment of TadA8e (SEQ ID NO: 55).

### Identity

"Identity" is used to refer to the matching of sequences between two polypeptides or two nucleic acids. "Identity" represents the percentage of the number of identical residues among the total number of residues between the polypeptide or nucleic acid sequences, and the calculation of the total number of residues is determined based on the type of mutation. Mutation types include insertions (extensions) at one or both ends of the sequence, deletions (truncations) from one or both ends of the sequence, substitution/replacement of one or more amino acids/nucleotides, insertions within the sequence, and deletions within the sequence.

Taking polypeptide sequences as an example, the total number of residues is calculated as the number of residues of the larger one of the two molecules being compared, if the mutation type is one or more of: substitution/replacement of one or more amino acids/nucleotides, insertion within the sequence, and deletion within the sequence. If the mutation type also includes insertions (extensions) at one or both ends of the sequence or deletions (truncations) from one or both ends of the sequence, then the number of amino acids inserted or deleted at one or both ends (for example, the number of insertions or deletions at both ends is less than 20) is not counted in the total number of residues. When calculating the percentage identity, the sequences being compared are aligned in a way that produces the maximum match between the sequences, and gaps (if present) in the alignment are resolved through a specific algorithm. The calculation of nucleotide identity is the same.

### Vector

A vector is a nucleic acid molecule capable of transporting another nucleic acid molecule linked to it.

Vectors include, but are not limited to, single-stranded, double-stranded, or partially double-stranded nucleic acid molecules; nucleic acid molecules with one or more free ends or without free ends (such as circular); nucleic acid molecules including DNA, RNA, or both; and various other polynucleotides known in the art. A vector may be introduced into a host cell through transformation, transduction, or transfection, enabling the genetic elements it carries to be expressed in the host cell. A vector can be introduced into a host cell to generate transcripts, proteins, or peptides, including proteins and their variants, fusion proteins, isolated nucleic acid molecules, etc. as described herein (for example, CRISPR transcripts, such as nucleic acid transcripts, proteins, or enzymes). A vector can contain various elements controlling expression, including but not limited to promoter sequences, transcription initiation sequences, enhancer sequences, selection elements, and reporter genes. A vector can also contain an origin of replication.

Vectors include plasmids and viral vectors. A plasmid refers to a circular double-stranded DNA loop into which additional DNA fragments can be inserted by, for example, standard molecular cloning techniques. For viral vectors, virus-derived DNA or RNA sequences are present in a vector used for virus packaging. Viruses include, for example, retroviruses, replication-defective retroviruses, adenoviruses, replication-defective adenoviruses, and adeno-associated viruses. A viral vector also contains a polynucleotide carried by a virus for transfection into a host cell. Some vectors (such as bacterial vectors with a bacterial origin of replication and episomal mammalian vectors) can replicate autonomously in the host cells into which they are introduced.

Other vectors (such as non-episomal mammalian vectors) are integrated into the genome of a host cell after being introduced into the host cell and thus replicate together with the host genome. Moreover, certain vectors can direct the expression of genes to which they are operably linked. Such vectors are called "expression vectors".

In some embodiments, vectors (such as viral vectors or non-viral vectors, such as lentiviral vectors or plasmids) can be delivered to target tissue by, for example, intramuscular injection, intravenous administration, transdermal administration, intranasal administration, oral administration, or mucosal administration. The above-mentioned delivery can be carried out at a single dose or multiple doses. Those skilled in the art should understand that the actual dose to be delivered herein may vary to a large extent depending on various factors, including but not limited to vector selection, target cells, organisms, tissues, the general condition of the subject to be treated, the degree of transformation/modification sought, the administration route, the mode of administration, and the type of transformation/modification sought.

### Regulatory Elements

Herein "regulatory elements" include promoters, enhancers, internal ribosome entry sites (IRES), and other expression control elements (such as transcription termination signals, like polyadenylation signals, poly-U sequences), and detailed descriptions can be seen in Goeddel, GENE EXPRESSION TECHNOLOGY: METHODS IN ENZYMOLOGY 185, Academic Press, San Diego, Calif (1990). In some cases, regulatory elements include those sequences that direct constitutive expression of a nucleotide sequence in many types of host cells, as well as those sequences that direct the expression of the nucleotide sequence only in certain host cells (e.g., tissue-specific regulatory sequences). Tissue-specific promoters can primarily direct expression in the desired tissue of interest, such as muscle, neurons, bone, skin, blood, specific organs (e.g., liver, pancreas), or specific cell types (e.g., lymphocytes). In other cases, regulatory elements can also direct expression in a timedependent manner (such as in a cell-cycle-dependent or development-stage-dependent manner), which may or may not be tissue-specific or cell-type-specific.

The term "promoter" refers to a non-coding nucleotide sequence that is located upstream of a gene and can initiate the expression of a downstream gene. A constitutive promoter is a nucleotide sequence that, when operably linked to a polynucleotide encoding or defining a gene product, causes production of the gene product in a cell under most or all physiological conditions of the cell. An inducible promoter is a promoter that selectively expresses a coding sequence or functional RNA in response to the presence of an endogenous or exogenous stimulus, such as in response to a chemical compound (chemical inducer), or in response to environmental, hormonal, chemical, and/or developmental signals. Inducible or regulatable promoters include, for example, those induced or regulated by light, heat, stress, waterlogging or drought, salt stress, osmotic stress, plant hormones, wounding, or chemicals (such as ethanol, abscisic acid (ABA), jasmonates, salicylic acid, or safeners).

### Host Cells

Herein "host cells" refer to eukaryotic cells (e.g., animal cells, plant cells, fungal cells, etc.), prokaryotic cells (e.g., some microbial cells, *Escherichia coli, Bacillus subtilis,* etc.), or cells from a multicellular organism cultured as a single-cell entity (e.g., cell lines). These cells serve as recipients of nucleic acids (e.g. expression vectors), and include the progeny of the original cells that have been genetically modified by nucleic acids.

It should be understood that the progeny of a single cell may not necessarily have exactly the same morphology, genome, or the like as the original parent cell due to natural, accidental, or intentional mutations. A "recombinant host cell" (also known as a "genetically modified host cell") is a host cell into which a heterologous nucleic acid, such as an expression vector, has been introduced.

Those skilled in the art will understand that the design of an expression vector can depend on factors such as the choice of host cell to be transformed and the desired level of expression.

In another aspect, the disclosure also provides a host cell or its progeny, which contains the aforementioned Cas protein, or the aforementioned fusion protein, or the aforementioned polynucleotide, or the aforementioned vector system, or the aforementioned CRISPR-Cas system, or the aforementioned composition.

In one embodiment, the host cell includes the cell of a non-human mammalian, a human, an insect, an avian, a reptilian, an amphibian, a rodent, a fish, a worm, a nematode, or yeast.

In one aspect, the disclosure also provides a multicellular organism, which contains the aforementioned cells or their progeny.

In one embodiment, the multicellular organism is an animal model or a plant model for related diseases.

### NLS

NLS refers to "nuclear localization sequence" or "nuclear localization signal", which is an amino acid sequence that facilitates the entry of a protein into the cell nucleus. Nuclear localization sequences are known in the art (for example, as described in the PCT application PCT/EP2000/011690 filed by Plank et al. on November 23, 2000 and published as WO/2001/038547 on May 31, 2001), and this patent application is incorporated herein by reference to its disclosure of exemplary nuclear localization sequences. In other embodiments, the NLS is an optimized NLS, for example, as described in Koblan et al., Nature Biotech. 2018 doi: 10.1038/nbt.4172. In some embodiments, the NLS contains the following amino acid sequences: KRTADGSEFESPKKKRKV (SEQ ID NO: 35), AVKRPAATKKAGQAKKKKLD (SEQ ID NO: 36), KRPAATKKAGQAKKKK (SEQ ID NO: 37), KKTELQTTNAENKTKKL (SEQ ID NO: 38), KRGINDRNFWRGENGRKTR (SEQ ID NO: 39), RKSGKIAAIVVKRPRK (SEQ ID NO: 40), PKKKRKV (SEQ ID NO: 41), or MDSLLMNRRKFLYQFKNVRWAKGRRETYLC (SEQ ID NO: 42).

### Operably Linked

"Operably linked" means that a target nucleotide sequence is linked to a regulatory element in a manner that allows expression of the nucleotide sequence (for example, in an *in vitro* transcription/translation system, or in a host cell when the vector is introduced into the host cell). Advantageous vectors include lentiviruses and adeno-associated viruses, and the types of these vectors can also be selected to target specific types of cells.

### Complementarity

"Complementarity" refers to the ability of one nucleic acid sequence to form one or more hydrogen bonds with another nucleic acid sequence by means of traditional Watson-Crick base pairing or other non-traditional types of base pairing. The percentage of complementarity represents the percentage of residues in one nucleic acid molecule that can form hydrogen bonds (e.g., Watson-Crick base-pairing) with another nucleic acid sequence (for example, if 5, 6, 7, 8, 9, and 10 out of 10 are complementary, the percentage of complementarity is 50%, 60%, 70%, 80%, 90%, and 100%). "Fully complementary" means that all consecutive residues of one nucleic acid sequence form hydrogen bonds with the same number of consecutive residues in another nucleic acid sequence. "Substantially complementary" refers to a degree of complementarity of at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50 or more nucleotides, or refers to two nucleic acids that hybridize under stringent conditions.

The term "stringent condition" related to hybridization refers to a condition under which a nucleic acid complementary to a target sequence hybridizes predominantly to the target sequence, and substantially does not hybridize to non-target sequences. Stringent conditions are usually sequence-dependent and depend on many factors. Generally, the longer the sequence, the higher the temperature at which the sequence specifically hybridizes to its target sequence.

"Hybridization" refers to a reaction in which one or more polynucleotides react to form a complex that is stabilized by hydrogen-bonding between the bases of these nucleotide residues. The complex may include two strands forming a duplex, three or more strands forming a multi-chain complex, a self-hybridizing single strand, or any combination of these. The hybridization reaction can serve as a step in a larger process (such as the start of PCR, or the cleavage of a polynucleotide by an enzyme). A sequence capable of hybridizing to a given sequence is called the "complement" of the given sequence.

The hybridization between a target sequence and a gRNA means that at least 60%, 65%, 70%, 75%, 80%, 85%, 90%, 91%, 92%, 93%, 94%, 95%, 96%, 97%, 98%, 99%, or 100% of the nucleic acid sequences of the target sequence and the gRNA can hybridize to form a complex; or it means that at least 12, 15, 16, 17, 18, 19, 20 or more bases of the nucleic acid sequences of the target sequence and the gRNA can complementarily pair and hybridize to form a complex.

### Expression

Nucleic acid expression includes one or more of: producing an RNA template from a DNA sequence (e.g. transcription), processing of an RNA transcript (e.g., through splicing, editing, 5'-cap formation, and/or 3'-end processing), translating an RNA into a polypeptide or protein, or posttranslational modification of a polypeptide or protein.

### Delivery

"Delivery" refers to providing an entity (such as a drug) to a target place. For example, the components of the CRISPR-Cas system/composition of the disclosure can be delivered in various forms, such as combinations of DNA/RNA, RNA/RNA, or protein-RNA. For example, the Cas protein can be delivered as a polynucleotide encoding a DNA, a polynucleotide encoding an RNA, or as a protein.

In one aspect, the disclosure also provides a delivery system, which includes the Cas protein, the fusion protein, the polynucleotide, or the CRISPR-Cas composition.

In one embodiment, the delivery system further includes a delivery vehicle, and the delivery vehicle includes nanoparticles, liposomes, exosomes, microbubbles, a gene gun, or an electroporation device.

In addition, when the delivery target is a plant cell, a delivery method such as one using a cellpenetrating peptide (CPP) may also be employed. For example, in a specific embodiment, the Cas protein and/or at least one guide RNA is conjugated to one or more CPPs, so as to effectively transport the CPP conjugated with the Cas protein and/or guide RNA into a plant cell (e.g., into a protoplast). CPPs are short peptides with less than 35 amino acids, which are derived from proteins or from chimeric sequences and are capable of transporting biomolecules across cell membranes in a receptor-independent manner. CPPs can be cationic peptides, peptides with hydrophobic sequences, amphiphilic peptides, peptides with proline-rich and antimicrobial sequences, as well as chimeric or bipartite peptides. CPPs can penetrate biological membranes, thereby triggering cross-membrane movement of various biomolecules into the cytoplasm, and can improve their intracellular pathways, thereby promoting the interaction of biomolecules with targets.

Exemplarily, CPPs include Tat (a nuclear transcriptional activator protein required for viral replication by HIV type 1), penetratin, Kaposi fibroblast growth factor (FGF) signal peptide sequence, integrin β3 signal peptide sequence, poly-arginine peptide Arg sequence, guanine-rich molecular transporters, sweet arrow peptide, etc.

### Linker

Herein a "linker" refers to a chemical group or molecule that connects two molecules or parts, such as two domains of a fusion protein, for example, a Cas protein and a deaminase. In some connection methods, the linker is located between or besides two groups, molecules, or other parts, and covalently links the two.

In some examples, the linker is a linear polypeptide formed by connection of amino acids or multiple amino acid residues through peptide bonding. In some embodiments, the linker is an organic molecule, group, polymer, or chemical moiety. The length and type of the linker can be designed as needed. In some examples, the linker may be an artificially synthesized amino acid sequence or a naturally occurring polypeptide sequence.

### Detection

In one aspect, the disclosure also provides a method for targeting and editing a target nucleic acid, the method comprising contacting the target nucleic acid with the CRISPR-Cas system or composition according to any of the above aspects.

In one aspect, the disclosure also provides a method for non-specifically degrading a single-stranded DNA after recognizing a target nucleic acid, the method comprising contacting the target nucleic acid with the CRISPR-Cas composition described above.

In one aspect, the disclosure also provides a method for targeting and nicking a non-spacer complementary strand of a double-stranded target nucleic acid after recognizing the spacer complementary strand of the double-stranded target nucleic acid, the method comprising contacting the double-stranded target nucleic acid with the CRISPR-Cas system or composition described above.

In one aspect, the disclosure also provides a method for targeting and cleaving a double-stranded target nucleic acid, the method comprising contacting the double-stranded target nucleic acid with the CRISPR-Cas system or composition described above.

In one embodiment, before nicking the spacer complementary strand of the double-stranded DNA, the non-spacer sequence complementary strand of the double-stranded target nucleic acid is nicked.

In one aspect, the disclosure also provides a method for specifically editing a double-stranded nucleic acid, the method comprising bringing the following (1) and (2) into contact under sufficient conditions for a sufficient period of time:
(1) The Cas protein or fusion protein, another enzyme with sequence-specific nicking activity, and the guide RNA, wherein the guide RNA directs the Cas protein variant or the fusion protein to nick the opposite strand relative to the activity of the another sequence-specific nicking enzyme; and (2) the double-stranded nucleic acid;
wherein the method results in the formation of a double-strand break.

In one aspect, the disclosure also provides a method for editing a double-stranded nucleic acid, the method comprising bringing the following (1) and (2) into contact under sufficient conditions for a sufficient period of time:
(1) The Cas protein or fusion protein, a fusion protein with a protein domain having DNA-modifying activity, and the RNA guide targeting the double-stranded nucleic acid; and (2) the double-stranded nucleic acid;
wherein the Cas protein of the fusion protein is modified to nick the non-target strand of the double-stranded nucleic acid.

In one embodiment, the two strands of the double-stranded nucleic acid are cleaved at different sites, resulting in a staggered cut.

In one embodiment, the two strands of the double-stranded nucleic acid are cleaved at the same site, resulting in a blunt double-strand break.

In one aspect, the disclosure also provides a method for targeting and cleaving a single-stranded target nucleic acid, the method comprising contacting the target nucleic acid with the CRISPR-Cas composition according to any one of the above aspects.

In one aspect, the disclosure also provides a method for inducing a change in the state of a cell, the method comprising contacting the CRISPR-Cas composition with a target nucleic acid in a cell.

In one embodiment, the state of the cell includes apoptosis or dormancy;

In one embodiment, the cell includes a eukaryotic cell or a prokaryotic cell;

In one embodiment, the cell includes a mammalian cell or a plant diseased cell;

In one embodiment, the cell includes a cancer cell;

In one embodiment, the cell includes an infectious cell or a cell infected by a pathogen;

In one embodiment, the cell includes a virus-infected cell or a prion-infected cell.

In one embodiment, the cell includes a fungal cell, a protozoan or a parasite cell.

In one aspect, the disclosure also provides a method for detecting a target nucleic acid in a sample, the method comprising contacting the sample with the Cas protein, guide RNA and a non-target sequence described above; and detecting a detectable signal generated by the cleavage of the non-target sequence by the Cas protein, thereby detecting the target nucleic acid; wherein the non-target sequence does not hybridize to the guide RNA.

### Kit

In one aspect, the present disclosure provides a kit, comprising the aforementioned Cas protein, the aforementioned fusion protein, the aforementioned polynucleotide, the aforementioned CRISPR-Cas composition, or the aforementioned host cell for use in the preparation of a kit. The components of the kit are in the same or different containers.

In one aspect, the present disclosure also provides a container that contains the aforementioned kit.

In one embodiment, the container includes a sterile container;

In one embodiment, the container includes a syringe.

In some embodiments, the kit further includes instructions for using the kit, for example, instructions in more than one language. The kit may also contain one or more reagents for use in a process that utilizes one or more of the above-mentioned components. The reagents can be provided in any suitable container. For example, the kit may provide one or more reaction or storage buffers. The above-mentioned reagents may be provided in a form that requires the addition of one or more other components before use (for example, in concentrated or lyophilized form); the buffer may be any buffer, including but not limited to sodium carbonate buffer, sodium bicarbonate buffer, borate buffer, Tris buffer, MOPS buffer, HEPES buffer, and combinations thereof. The buffer may have a suitable pH value, for example, it may be alkaline. In some embodiments, the pH of the buffer is approximately 7-10.

### Treatment

"Treatment" refers to treating or curing a disorder of a subject, delaying the onset of symptoms of the disorder, and/or alleviating the severity of the disorder. The term "subject" includes but is not limited to various animals, plants, and microorganisms. Animals include mammals, such as bovines, equines, ovines, porcines, canines, felines, lagomorphs, rodents (e.g., mice or rats), non-human primates (e.g., rhesus monkeys or cynomolgus monkeys), or humans. In certain embodiments, the subject (e.g., a human) has a disorder (e.g., a disorder caused by a disease-related gene defect). "Plant" refers to any differentiated multicellular organism capable of photosynthesis, including crop plants at any stage of maturity or development.

In one aspect, the present disclosure also provides the use of the aforementioned Cas protein, the aforementioned fusion protein, the aforementioned polynucleotide, the aforementioned CRISPR-Cas composition, or the aforementioned host cell, in the preparation of a medicament for treating a disorder or disease in a subject in need thereof.

In one embodiment, the use includes administering the CRISPR-Cas composition to the subject or *ex vivo* cells from the subject.

In one embodiment, the spacer sequence is complementary to at least 15 nucleotides of the target nucleic acid related to the disorder or disease, and the Cas protein or the fusion protein cleaves the target nucleic acid.

In one embodiment, the condition or disease includes cancer or an infectious disease;

In one embodiment, the cancer includes one or more of Wilms' tumor, Ewing's sarcoma, neuroendocrine tumor, glioblastoma, neuroblastoma, melanoma, skin cancer, breast cancer, colon cancer, rectal cancer, prostate cancer, liver cancer, kidney cancer, pancreatic cancer, lung cancer, biliary tract cancer, cervical cancer, endometrial cancer, esophageal cancer, gastric cancer, head and neck cancer, medullary thyroid cancer, ovarian cancer, glioma, lymphoma, leukemia, myeloma, acute lymphoblastic leukemia, acute myeloid leukemia, chronic lymphocytic leukemia, chronic myeloid leukemia, Hodgkin's lymphoma, non-Hodgkin's lymphoma, or urinary bladder cancer;

In one embodiment, the disorder or disease includes one or more of cystic fibrosis, Duchenne muscular dystrophy, Becker muscular dystrophy, α-1-antitrypsin deficiency, Pompe disease, myotonic dystrophy, Huntington's disease, fragile X syndrome, Friedreich's ataxia, amyotrophic lateral sclerosis, frontotemporal dementia, hereditary chronic kidney disease, hyperlipidemia, hypercholesterolemia, Leber congenital amaurosis, sickle cell disease, hypercholesterolemia, transthyretin amyloidosis, or beta-thalassemia.

In one embodiment, the pathogen of the infectious disease includes one or more of human immunodeficiency virus, herpes simplex virus-1, or herpes simplex virus-2.

The main advantages of the disclosure include the following aspects.
(a) The present disclosure for the first time discovers a novel Cas protein which has excellent gene-editing activity, can effectively edit or cleave target genes, and can effectively treat disorders or diseases of a subject in need (such as one or more of cystic fibrosis, Duchenne muscular dystrophy, Becker muscular dystrophy, α-1-antitrypsin deficiency, Pompe disease, myotonic dystrophy, Huntington's disease, fragile X syndrome, Friedreich's ataxia, amyotrophic lateral sclerosis, frontotemporal dementia, hereditary chronic kidney disease, hyperlipidemia, hypercholesterolemia, Leber congenital amaurosis, sickle cell disease, hypercholesterolemia, transthyretin amyloidosis, or beta-thalassemia).
(b) The Cas protein of the disclosure is a brand-new Cas enzyme which exhibits good nuclease activity both *in vivo* and *in vitro,* and has broad application prospects.
(c) Compared with the Cas enzymes disclosed in the prior art, the Cas protein of the disclosure has advantageous editing efficiency, providing more options for base-editing tools.
(d) The base editor constructed with the Cas enzyme disclosed in the present disclosure can effectively perform base editing and has great potential for applications.
(e) By mutating the wild-type Cas protein, the disclosure unexpectedly obtains new Cas protein variants showing better gene-editing activity than the wild-type Cas protein.

The disclosure will be further elaborated hereinafter in combination with specific examples. It should be understood that these examples are provided to only illustrate the disclosure and not to limit its scope. The experimental methods in the following examples without detailed conditions specified are usually carried out under conventional conditions, such as the conditions described in Sambrook et al., Molecular Cloning: A Laboratory Manual (New York: Cold Spring Harbor Laboratory Press, 1989), or according to the conditions recommended by the manufacturers. Unless otherwise stated, percentages and parts are percentages by weight and parts by weight.

Unless otherwise specified, the reagents and materials in the examples of the disclosure are all commercially available products.

### Example 1. Obtaining Cas Protein

The inventors analyzed the metagenome of uncultured organisms, performed analyses such as redundancy removal and protein clustering, and identified one novel Cas protein. The Cas protein showed a low sequence identity to previously reported Cas proteins according to Blast analysis, and is named CasY6 in the disclosure.

The amino acid sequence of the above-mentioned CasY6 protein is SEQ ID NO: 1, and its nucleotide sequence optimized with human codons is SEQ ID NO: 2.

The direct repeat (DR) sequence of the guide RNA corresponding to the above-mentioned CasY6 protein was analyzed, and the results showed that the DNA sequence encoding the direct repeat (DR) sequence of the guide RNA corresponding to the CasY6 protein was:
TATCCATCGTGCCGCCTCTTGGCAC (SEQ ID NO: 5).

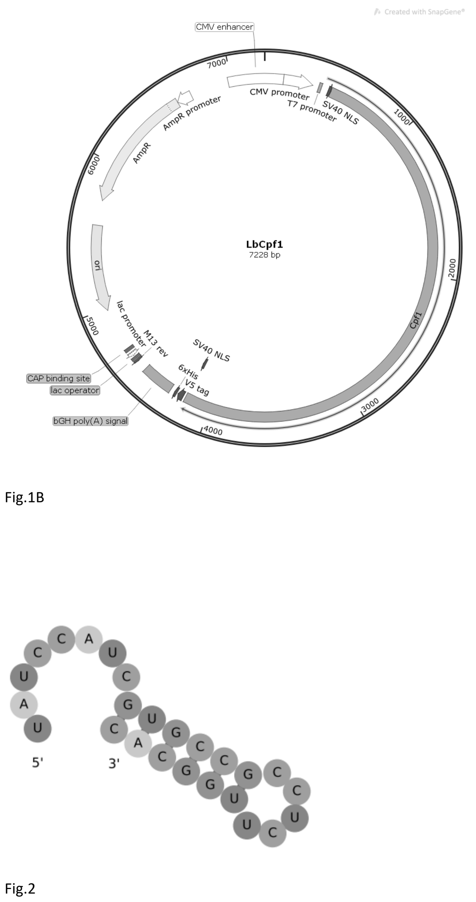

The inventors further analyzed the RNA secondary structure of the DR sequence in the precrRNA by RNAfold. The analysis results are shown in Figure 2. The analysis revealed that the PAM corresponding to CasY6 is TTN, where N is A/T/C/G. The gRNA (also known as crRNA) sequence of the CasY6 protein consists of a spacer sequence and a direct repeat (DR) sequence.

After identification, CasY6 of the disclosure belongs to the Cas12 protein family.

### Example 2. Verification of the Cleavage Activity of Cas Protein

### 1. Plasmid Construction

(1) The TTR gene was used as the target, and according to the target sequence of the target gene TTR a spacer sequence was designed: GCATCTCCCCATTCCATGAG (SEQ ID NO: 6).

Based on the DR sequences of the CasY6 protein (with the amino acid sequence of SEQ ID NO: 1) and the LbCpf1 protein (with the amino acid sequence of SEQ ID NO: 12), sgRNA sequences targeting the TTR target gene were designed, as shown in the following table.

| Sequence Name | Specific Sequence |
|---|---|
| CasY6-TTR-sgRNA sequence | TATCCATCGTGCCGCCTCTTGGCACGCATCTCCCCATTCCATGAG (SEQ ID NO: 8). The underlined part of the sequence is the DR sequence, and the remaining sequence is the spacer sequence targeting the TTR target sequence. |
| LbCpf1-TTR-sgRNAsequence | TAATTTCTACTAAGTGTAGATGCATCTCCCCATTCCATGAG (SEQ ID NO: 15). The underlined part of the sequence is the DR sequence, and the remaining sequence is the spacer sequence targeting the TTR target sequence. |

According to the requirements of vector expression, a T7 promoter and an rrnB T2 terminator were added to the 5'-end and 3'-end of the sgRNA sequence of each of CasY6 and LbCpf1 mentioned above, obtaining the CasY6-sgRNA expression fragment sequence:
CAATTG*TAATACGACTCACTATAGG*CACCGTATCCATCGTGCCGCCTCTTGGCACGCATCTCCCCATTCCA TGAGTTTTTTAAGCTTGGCGTagaaggccatcctgacggatggcctttt**ACGCGT** (SEQ ID NO: 7), wherein the singleunderlined sequence is the CasY6 DR sequence, the double-underlined sequence is the spacer sequence, the italic sequence is the T7 promoter, the wavy-underlined sequence is the rrnB T2 terminator sequence, the sequence between the spacer sequence and the rrnB T2 terminator sequence is a linker sequence, the dotted-line-underscored sequence is the Mfel restriction site, the sequence in bold is the Mlul restriction site, and the CACCG is a linker.

In the same way, the LbCpf1-sgRNA expression fragment sequence was synthesized:
CAATTG*TAATACGACTCACTATAGG*CACCGTAATTTCTACTAAGTGTAGATGCATCTCCCCATTCCATGAG TTTTTTAAGCTTGGCGTagaaggccatcctgacggatggcctttt**ACGCGT** (SEQ ID NO: 14), wherein the singleunderlined sequence is the LbCpf1 DR sequence, the double-underlined sequence is the spacer sequence, the italic sequence is the T7 promoter, the wavy-underlined sequence is the rrnB T2 terminator sequence, the sequence between the spacer sequence and the rrnB T2 terminator sequence is a linker sequence, the dotted-line-underscored sequence is the Mfel restriction site, the sequence in bold is the Mlul restriction site, and the CACCG is a linker.

To protect the integrity of the sequence, in the synthesis of the CasY6-sgRNA expression fragment sequence and the LbCpfl-sgRNA expression fragment sequence, AGC was introduced at the 5'-end and ATA was introduced at the 3'-end as protective bases.

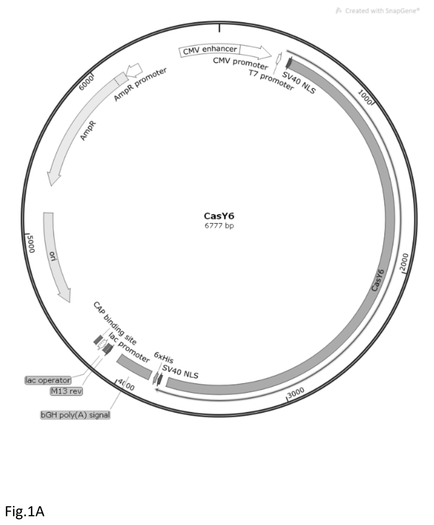

(2) A nucleotide sequence (SEQ ID NO: 2) fragment encoding the CasY6 protein was synthesized by Suzhou Synbio Technologies Co., Ltd. The synthesized nucleotide sequence fragment encoding the CasY6 protein was constructed into an ABE8e plasmid (Addgene, Plasmid #138489) at positions 466-5160, to construct a CasY6 recombinant expression plasmid (see Figure 1A for the plasmid map,).

An optimized nucleotide sequence (SEQ ID NO: 13) encoding LbCpf1 was synthesized by Suzhou Synbio Technologies Co., Ltd. and was constructed in the same way to obtain an LbCpf1 recombinant expression plasmid (see Figure 1B for the plasmid map).

(3) The sgRNA expression sequence fragments (CasY6-sgRNA expression fragment sequence and LbCpf1-sgRNA expression fragment sequence) described in step (1) were synthesized by Suzhou Synbio Technologies Co., Ltd. The sgRNA expression fragment sequence was double-digested (Mfel/Mlul) and inserted into a CasY6 recombinant expression plasmid vector that was also double-digested (Mfel/Mlul), to obtain a recombinant CasY6+sgRNA expression plasmid expressing CasY6 and sgRNA. The LbCpf1+sgRNA expression plasmid was constructed in the same way.

(4) A Target plasmid with the target sequence was constructed as follows.

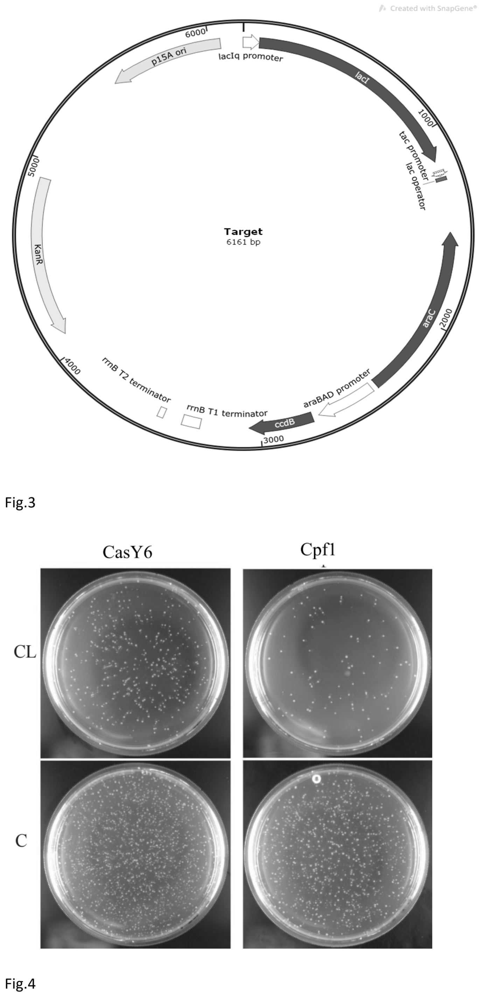

An araC-pBAD-CCDB fragment (SEQ ID NO: 11) with the TTR gene target sequence (SEQ ID NO: 6) was synthesized by Suzhou Synbio Technologies Co., Ltd, and was inserted into a pKESK22 plasmid (Addgene, Plasmid #64857) at positions 1284-1300 to obtain the Target plasmid. The sequence of the Target plasmid is of SEQ ID NO: 4, and the plasmid map is shown in Figure 3.

### 2. Preparation and Transformation of Escherichia coli Competent Cells

The Target plasmid was transferred into DH5α competent cells, and then streaked and inoculated on an LB solid medium containing 50 µg/ml kanamycin sulfate with an inoculating loop. The plate was placed in a biochemical incubator and cultured overnight at 37 °C. The next day, a single colony was picked from the plate and inoculated into a test tube containing 4 ml of LB liquid medium with 50 µg/ml kanamycin sulfate (Sangon Biotech, A100408-0100), and cultured overnight under shaking at 37 °C and 200 rpm. The following day, 4 ml of the bacterial liquid culture was inoculated into a 2-L Erlenmeyer flask containing 400 ml of LB liquid medium with 50 µg/ml kanamycin sulfate, and cultured under shaking at 37 °C and 200 rpm for 2-3 hours.

When the OD_{600 nm} value of the bacterial liquid culture reached 0.3-0.5, the Erlenmeyer flask was taken out and placed on ice for 10-15 min. Under a sterile condition, the bacterial liquid culture was poured into a pre-cooled 500-ml centrifuge bottle, centrifuged at 4 °C and 3000 rpm for 8 min. The supernatant was discarded, and approximately 200 ml of a pre-cooled CaCl₂ solution was added. The mixture was pipetted to resuspend the bacterial cells, and then placed in an ice-bath for 30 min. Subsequently, the bacterial liquid culture was centrifuged at 4 °C and 3000 rpm for 8 min. The supernatant was discarded, and approximately 8 ml of a pre-cooled CaCl₂ solution was added to resuspend the bacterial cells again. The resuspended bacterial cells were aliquoted into 1.5-ml EP tubes, 110 µl per tube, and stored in an ultra-low-temperature freezer at -80 °C for later use.

### 3. Determination of in vivo Editing Efficiency in Escherichia coli

The CasY6+sgRNA expression plasmid and the LbCpf1+sgRNA expression plasmid were respectively transferred into the competent cells prepared in Step 2. The detailed process is as follows.
(1) The competent cells were taken out of the -80 °C freezer and quickly buried in ice. After about 5 minutes, the cell mass thawed, and the CasY6+sgRNA expression plasmid was added. Then, they were gently mixed by tapping the bottom of the centrifuge tube by hand, let stand in ice for 25 minutes, heat-shocked in a 42 °C water-bath for 45 seconds, quickly put back on ice, and let stand for 2 minutes. 900 µl of sterile LB medium without antibiotics was added to the centrifuge tube, mixed well, and recovered at 37 °C and 220 rpm for 60 minutes. 100 µl of the bacterial liquid was spread onto each of an LB agar plate containing 30 µg/ml carbenicillin (Sangon Biotech, A100358-0001) (referred to as C-LB medium) and an LB agar plate containing both 30 µg/ml carbenicillin and 10 mM L-arabinose (Sangon Biotech, A610071-0100) (referred to as CL-LB medium). The two LB agar plates were placed upside-down in an incubator and cultured overnight at 37 °C.
(2) Measurement of *in vivo* Editing Efficiency in *Escherichia coli*

As shown in Figure 3, the Target plasmid carried a PBAD promoter inducible by L-arabinose and a CCDB gene whose expression is regulated by the PBAD promoter. The CCDB gene can express the CCDB toxic protein. As a DNA gyrase inhibitor, the CCDB toxic protein can lock the DNA gyrase and the broken double-stranded DNA complex, preventing the DNA gyrase from functioning and ultimately leading to cell death.

Based on this, the inventors designed a method for measuring the *in vivo* editing efficiency in *Escherichia coli.*

When L-arabinose is present in the medium, if the CasY6 protein or the LbCpf1 protein under the guidance by the sgRNA can specifically target the target sequence (SEQ ID NO: 6) of the TTR gene on the Target plasmid and exert a cleavage effect, then the regulatory expression pathway of the PBAD promoter on the CCDB toxic protein is shut down, and the host cells survive because no ccdB toxic protein is produced. Conversely, if the CasY6 protein or the LbCpf1 protein cannot specifically target the TTR target sequence on the Target plasmid, the host *Escherichia coli* cells die because of the expression of the CCDB toxic protein regulated by the PBAD promoter induced by L-arabinose.

Therefore, the editing efficiency of the CasY6 protein in specifically targeting and cleaving the TTR target gene in *Escherichia coli* can be calculated as the ratio of the number of bacterial clones on the CL-LB medium to the number of bacterial clones on the C-LB medium in Step (2).

The results are shown in Figure 4. After counting the number of *Escherichia coli* clones and calculating the ratio, it was found that the editing efficiency of the CasY6 protein was 16.2%, and the editing efficiency of the LbCpf1 protein was 6.4%. The editing efficiency of the CasY6 protein was significantly higher than that of the LbCpf1 protein.

### Example 3. Measurement of Editing Efficiency in HEK293T Cells

### 1. Construction of TTR-sgRNA Expression Plasmid

(1) A TTR-sgRNA sequence was designed according to the target sequence of the TTR gene (SEQ ID NO: 6), and oligonucleotides (oligos) were synthesized:
CasY6-TTR-sgRNA sequence:
   TATCCATCGTGCCGCCTCTTGGCACGCATCTCCCCATTCCATGAG (SEQ ID NO: 8). The underlined part is the DR sequence, and the remaining sequence is the spacer sequence.
LbCpf1-TTR-sgRNA sequence: TAATTTCTACTAAGTGTAGATGCATCTCCCCATTCCATGAG (SEQ ID NO: 15). The underlined part is the DR sequence, and the remaining sequence is the spacer sequence.

(2) A sequence CACC was added to the 5'-end of the upstream sequence of the TTR-sgRNA, and a sequence AAAA was added to the 5'-end of the downstream sequence, and the oligos were synthesized. The specific sequences are as follows:

| sgRNA Name | Up- and Down-stream Primer Sequences |
|---|---|
| CasY6-TTR-sgRNA | CasY6-TTR-sgRNA-Oligo-F: |
| | CACCTATCCATCGTGCCGCCTCTTGGCACGCATCTCCCCATTCCATGAG (SEQ ID NO: 16) |
| | AAAACTCATGGAATGGGGAGATGCGTGCCAAGAGGCGGCACGATGGAT A (SEQ ID NO: 17) CasY6-TTR-sgRNA-Oligo-R: |
| LbCpf1-TTR-sgRNA | LbCpf1-TTR-sgRNA-Oligo-F: |
| | CACCTAATTTCTACTAAGTGTAGATGCATCTCCCCATTCCATGAG (SEQ ID NO: 18) |
| | LbCpf1-TTR-sgRNA-Oligo-R: |
| | AAAACTCATGGAATGGGGAGATGCATCTACACTTAGTAGAAATTA (SEQ ID NO: 19) |

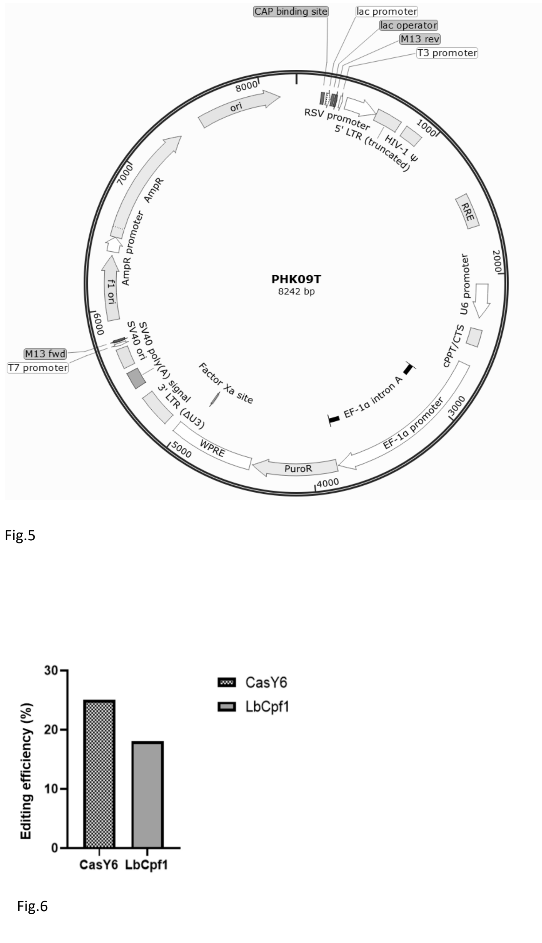

After the upstream and downstream primers of the aforementioned TTR-sgRNA were synthesized, annealing was carried out in a preset program (95 °C, 5 min; from 95 °C to 85 °C at a rate of -2 °C/s; from 85 °C to 25 °C at a rate of -0.1 °C/s; held at 4 °C). Then, the annealed product was ligated to a PHK09T vector linearized by BsmBI (NEB, #R0580L). The sequence of the PHK09T vector is SEQ ID NO: 3, and the plasmid map is shown in Figure 5.

The linearization of the PHK09T vector and its ligation with the annealed product of TTR-sgRNA were as follows.

First, the PHK09T vector was linearized in a linearization system: 3 µg of the PHK09T vector; 6 µL of a buffer (NEB: R0539L); 2 µL of BsmBl; and ddH₂O making up to 60 µL, which were digested at 50 °C overnight.

The ligation system of the annealed product of TTR-gRNA and the linearized vector:
1 µL of a T4 ligase buffer (NEB, #M0202L), 20 ng of the linearized vector, 5 µL of the annealed oligo fragment, 0.5 µL of T4 ligase (NEB, #M0202L), and ddH₂O making up to 10 µL, which were ligated at 16 °C overnight to obtain the CasY6-TTR-sgRNA expression plasmid and the LbCpf1-TTR-sgRNA expression plasmid.

(3) The CasY6-TTR-sgRNA expression plasmid and the LbCpf1-TTR-sgRNA expression plasmid obtained in step (2) were transferred into *Escherichia coli* DH5α competent cells (Vazyme, DL1001). The specific steps were as follows.

The DH5α competent cells were taken out from a -80 °C refrigerator and quickly buried in ice. After 5 minutes, the cell mass thawed, and the ligation product was added. They were gently mixed by tapping the bottom of the centrifuge tube by hand, let stand in ice for 25 minutes, heat-shocked in a 42 °C water-bath for 45 seconds, quickly put back on ice, and let stand for 2 minutes. 700 µl of sterile LB medium without antibiotics was added to the centrifuge tube, mixed well, and recovered at 37 °C and 200 rpm for 60 minutes. The mixture was centrifuged at 3,000 rpm for one minute to collect the bacteria. With about 100 µl of the supernatant left in the tube, the cell mass was gently resuspended by pipetting, and spread onto an LB medium plate with Amp antibiotic. The plate was placed upside-down in a 37 °C incubator and cultured overnight. Single colonies were picked. After confirmation by sequencing, positive clones were cultured, the plasmids were extracted (with the Endo-Free Plasmid Mega Kit, TIANGEN: DP120-01), and the concentration was measured, followed by storage in a -20 °C refrigerator for later use.

### 2. Measurement of Editing Efficiency at the Cellular Level

### (1) HEK293T Cell Culture

HEK293T cells (purchased from ATCC) were inoculated into DMEM medium (Gibco, 11965092) supplemented with 10% FBS (v/v) and containing 1% Penicillin Streptomycin (v/v) (Gibco, 15140122), and cultured in a 37 °C cell incubator with 5% CO₂. For cells used for transfection, they were inoculated into a 24 - well cell culture plate one day before and cultured. The cells were observed the next day, and transfection was performed when the cell density reached approximately 80%..

(2) The CasY6 recombinant expression plasmid (see the plasmid map in Figure 1A), the CasY6-TTR-sgRNA expression plasmid, the LbCpf1 recombinant expression plasmid (see the plasmid map in Figure 1B), and the LbCpf1-TTR-sgRNA expression plasmid were each used together with the EGFP-C1 (Addgene, Plasmid, #54759) plasmid to co-transfect HEK293T cells.

The amounts of plasmids used to transfect the cells per well in the 24-well plate were 0.3 µg of the nuclease expression plasmid (CasY6 recombinant expression plasmid or LbCpf1 recombinant expression plasmid), 0.3 µg of the sgRNA expression plasmid (CasY6-TTR-sgRNA expression plasmid or LbCpf1-TTR-sgRNA expression plasmid), and 0.3 µg of the EGFP-C1 plasmid. The specific transfection procedure was as follows.

The CasY6 expression plasmid, the CasY6-TTR-sgRNA expression plasmid, and the EGFP-C1 plasmid were mixed and diluted with 25 µl of UltraFectin^{®} transfection-specific serum-free medium (Yuanpei Biology, L530KJ), then 2 µl of Lipofectamine 3000 (Invitrogen, L3000015) reagent was added, and the mixture was pipetted gently and mixed until uniform to obtain Reagent A, which was left to stand for 5 minutes. At the same time, 2 µl of Lipofectamine 3000 transfection reagent (Invitrogen, L3000015) was diluted and mixed with 25 µl of UltraFectin^{®} transfection-specific serum-free medium (Yuanpei Biology, L530KJ) to obtain Reagent B, which was left to stand for 5 minutes.

Reagent A and Reagent B were mixed and pipetted until uniform, and left to stand for 20 minutes. After the standing, the mixed reagent was added dropwise to the cells to be transfected in a 24-well plate, and then the plate was returned to a 37 °C, 5% CO₂ incubator for culturing. Six hours after transfection, the medium was replaced with DMEM medium containing 10% FBS.

In the same way, the LbCpf1 recombinant expression plasmid, the LbCpf1-TTR-sgRNA expression plasmid, and the EGFP-C1 plasmid were transfected into HEK293T cells.

### (3) Measurement of Editing Efficiency

Forty-eight hours after transfection, the expression of the EGFP fluorescent protein indicated successful cell transfection. EGFP-positive cells were sorted for the measurement of editing efficiency. Genomic DNA was extracted from these cells (using the Genomic DNA Extraction Kit, TIANGEN, DP304-03). Identification primers were designed according to experimental requirements, and the sequences of the identification primers used are shown in the following table:

| Primer Name | Sequence |
|---|---|
| TTR-F | aactgaggaggaatttgtag (SEQ ID NO: 9) |
| TTR-R | caaaagcaaaaaccaaaacc (SEQ ID NO: 10) |

With the genomic DNA as a template, the sequence near the target site was amplified by PCR using the primers shown in the above table. The PCR amplification system was as follows:
2× Taq Master Mix (Vazyme, P112-03) 25 µL; Primer-F (TTR-F) (10 pmol/µL) 1 µL; Primer-R (TTR-R) (10 pmol/µL) 1 µL; template 1 µL; ddH₂O making up to 50 µL.

The amplified PCR products were subjected to high-throughput deep sequencing (GENEWIZ, Inc.) or Sanger sequencing (Biosune Biotechnology (Shanghai) Co., Ltd.) to determine the editing efficiency.

The results of measuring and determining the editing efficiency of CasY6 and LbCpf1 are shown in Figure 6. In 293T cells, the editing efficiency of CasY6 was 25%, while that of LbCpf1 was only 18%. The editing efficiency of the CasY6 protein was much higher than that of LbCpf1.

### Example 4. Use of CasY6 in Base Editing

### (1) Obtaining Catalytically Inactive CasY6

To obtain catalytically inactive (i.e., cleavage-inactive) dCasY6, the inventors constructed CasY6 mutants with single-point mutations D659A, D711A, E895A, and D1069A, namely D659A-dCasY6, D711A-dCasY6, E895A-dCasY6, and D1069A-dCasY6. The specific construction processes were as follows.

The CasY6+sgRNA expression plasmid obtained in Step 1(3) of Example 2 was subjected to sitedirected mutagenesis to modify the amino acids at four positions of CasY6, which were aspartic acid (Asp, D) at position 659, aspartic acid (Asp, D) at position 711, glutamic acid (Glu, E) at position 895, and aspartic acid (Asp, D) at position 1069 of SEQ ID NO: 1. These amino acids were mutated to alanine (Ala, A). The codons before and after the mutation of each of the above-mentioned amino acids are shown in the following table:

| Amino Acid before Mutation | Codon | Amino Acid after Mutation | Codon |
|---|---|---|---|
| Aspartic acid (Asp, D) | GAC | Alanine (Ala, A) | GCA |
| Aspartic acid (Asp, D) | GAC | Alanine (Ala, A) | GCA |
| Glutamic acid (Glu, E) | GAG | Alanine (Ala, A) | GCA |
| Aspartic acid (Asp, D) | GAC | Alanine (Ala, A) | GCA |

Forward and reverse primers were designed and synthesized for the amino acids and their codons shown in the above table, and PCR amplification was carried out with the CasY6+sgRNA expression plasmid as a template. After amplification, the Universal DNA Purification and Recovery Kit (TIANGEN Biotech (Beijing) Co., Ltd., DP214) was used to recover and purify the amplified products. The purified products were transformed into Escherichia coli Dh5α competent cells (Vazyme, DL1001) and cultured at 37 °C overnight. The next day, single colonies were picked and subjected to sequencing. After confirmation by sequencing, the positive clones were cultured, the plasmids were extracted (TIANGEN, DP120-01), and the concentration was measured, followed by storage in a -20 °C refrigerator for later use.

The obtained recombinant plasmids with point mutations were named as: D659A-dCasY6+sgRNA expression plasmid, D711A-dCasY6+sgRNA expression plasmid, E895A-dCasY6+sgRNA expression plasmid, and D1069A-dCasY6+sgRNA expression plasmid.

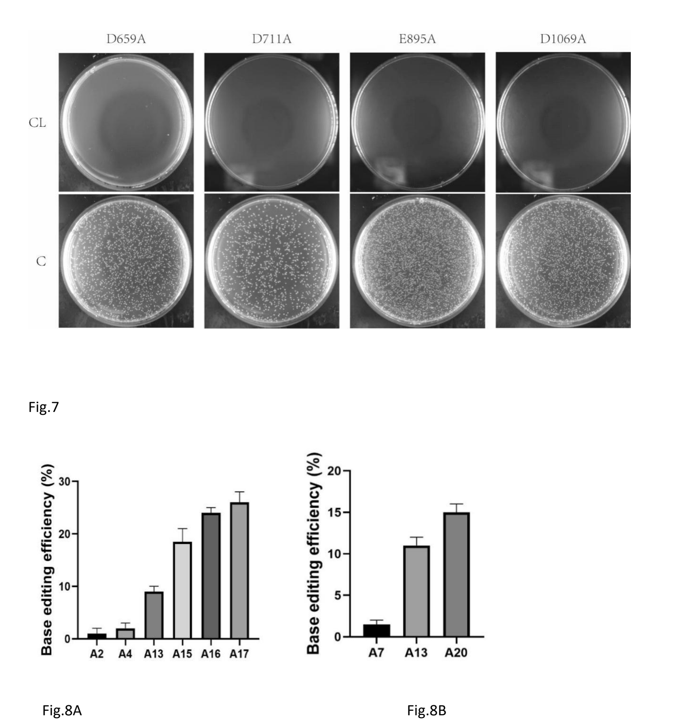

Subsequently, the constructed D659A-dCasY6+sgRNA expression plasmid, D711A-dCasY6+sgRNA expression plasmid, E895A-dCasY6+sgRNA expression plasmid, and D1069A-dCasY6+sgRNA expression plasmid were each measured for *in vivo* editing efficiency in *Escherichia coli.* The measurement method and calculation method were the same as those in Steps 2 and 3 of Example 2. The experimental results are shown in Figure 7. By the counting of the number of *Escherichia coli* clones and the calculation of the ratio, it was considered that D659A-dCasY6, D711A-dCasY6, E895A-dCasY6, and D1069A-dCasY6 had lost their catalytic activity (cleavage activity). That is to say, the point mutations D659A, D711A, E895A, and D1069A caused the CasY6 protein to lose its cleavage activity.

### 2. Measurement of Base-Editing Efficiency at the Cellular Level

### (1) Construction of CasY6-TTR-sgRNA' and CasY6-TTR-sgRNA" Plasmids

sgRNAs were designed according to the target sequence of the TTR gene: CasY6-TTR-sgRNA' and CasY6-TTR-sgRNA" sequences, and oligonucleotides (oligos) were synthesized:
CasY6-TTR-sgRNA': TATCCATCGTGCCGCCTCTTGGCACtatatcccttctacaaattc (SEQ ID NO: 20);
CasY6-TTR-sgRNA": TATCCATCGTGCCGCCTCTTGGCACgtgtctatttccactttgta (SEQ ID NO: 21),
wherein the underlined part is the DR sequence, and the remaining sequence is the spacer sequence.

(2) A sequence CACC was added to the 5'-end of the upstream sequence of each sgRNA and a sequence AAAA was added to the 5'-end of the downstream sequence. The specific forms are as follows:

| sgRNA Name | Up- and Down-stream Sequences |
|---|---|
| CasY6-TTR-sgRNA' | CasY6-TTR-sgRNA'-Oligo-F: |
| | CACCTATCCATCGTGCCGCCTCTTGGCACtatatcccttctacaaattc (SEQ ID NO: 22) |

| | |
|---|---|
| | CasY6-TTR-sgRNA'-Oligo-R: |
| | **AAAA**GAATTTGTAGAAGGGATATAGTGCCAAGAGGCGGCACGATGGATA (SEQ ID NO: 23) |
| CasY6-TTR-sgRNA" | CasY6-TTR-sgRNA"-Oligo-F: |
| | **CACC**TATCCATCGTGCCGCCTCTTGGCACgtgtctatttccactttgta (SEQ ID NO: 24) |
| | CasY6-TTR-sgRNA"-Oligo-R: |
| | **AAAA**TACAAAGTGGAAATAGACACGTGCCAAGAGGCGGCACGATGGATA (SEQ ID NO: 25) |

According to the method in Step 1 of Example 3, the upstream and downstream sequences of CasY6-TTR-sgRNA' and CasY6-TTR-sgRNA" were annealed and then ligated into PHK09T vectors to obtain the CasY6-TTR-sgRNA' expression plasmid and the CasY6-TTR-sgRNA" expression plasmid. These plasmids were then transferred into *Escherichia coli* DH5α competent cells which were cultured for plasmid amplification. After sequencing to confirm correctness and measuring the concentration, they were stored for future use.

### (3) Construction of Base Editor Plasmid (taking 005V1-10-3 as an example)

The adenosine deaminase catalytic domain selected by the inventors was a mutant of the amino-acid sequence of SEQ ID NO:28 (named 005V1-10-3): Q148G+Q149M+P150R. The amino-acid sequence of this mutant is SEQ ID NO: 29, and the nucleotide sequence encoding the deaminase 005V1-10-3 is SEQ ID NO: 30. A base-editor fusion protein (having the amino-acid sequence of SEQ ID NO: 43) composed of the deaminase 005V10-3 and the dCasY6 protein was constructed by homologous recombination. The specific operations were as follows.

First, the 005V1-10-3 nucleotide fragment with homologous-arm sequences and a linker was synthesized by Suzhou Synbio Technologies Co., Ltd.:
*ccaaagaagaagcggaaagtc***ATGAGTGAGCTGAATGATGCTTACTGGATGAAACAGGCACTCGCTTTAGC TCAGAAGGCCcggGAACAGGGAGAAGTTCCAGTGGGCGCTATTCTGGTGctgGATGATGAAGTGATAGGACA GGGATGGAATAGAgccATCACCctgCACGACCCCACCGCCCACGCCGAGATCATGGCCCTGCAGCAGGGCGGC CAGATCGTGCAGAACTACCGGCTGCTGAACGCCACCCTGTACGTGACCttcGAGCCCTGCGTGATGTGCGCCG GCGCCATGGTGCACAGCAGGATCAAGAGACTGGTCTACGGCgtgAGCaactcaAAAagaGGCGCCGCCGGCAG CCTGCTGaacGTGCTGaactacCCCGGCATGAACCACCAGATCGAGATCACCGCCGGCGTGATGGCCAACGAG TGCAGCGAGATGCTGtgcCAGTTCtatGGTATGCGCAGAGAAgtgttcaatGCTGAGCGTGAGGCTAGGCGGCTG AACCAACCTGATAGAGCTGAC**tccggaggatctagcggaggctcctctggctctgagacacctggcacaagcgagagcgccaacacct gaaagcagcgggggcagcagcggggggtca*ATCAAGAACCAAATCATCGG* (SEQ ID NO: 31), wherein the bold part is the 005V1-10-3 nucleotide sequence, the italic part is the left and right homologous-arm regions, and the wavy-line part is the linker sequence.

The D1069A-dCasY6+sgRNA expression plasmid obtained in step (1) was amplified by PCR for linearization to obtain a linearized expression vector. The primers used are shown in the following table:

| Primer Name | Specific Sequence |
|---|---|
| D1069A-dCasY6-F | ATCAAGAACCAAATCATCGG (SEQ ID NO:32) |
| D1069A-dCasY6-R | Gactttccgcttcttctttgg (SEQ ID NO:33) |

The nucleotide fragment 005V1-10-3 with homologous-arm sequences and a linker (SEQ ID NO:31) and the linearized D1069A-dCasY6+sgRNA expression vector were subjected to homologous recombination. The reaction was carried out with Gibson Assembly Master Mix (NEB, E2611S). After the reaction, the ligation product was transformed into Escherichia coli DH5α competent cells (Vazyme, DL1001). The specific process was as follows.

DH5α competent cells were taken out from a -80 °C refrigerator and quickly buried in ice. After 5 minutes, the cell mass thawed, and the ligation product was added. They were gently mixed by tapping the bottom of the centrifuge tube by hand, let stand in ice for 25 minutes, heat-shocked in a 42 °C water-bath for 45 seconds, quickly put back on ice, and let stand for 2 minutes. 700 µl of sterile LB medium was added to the centrifuge tube, mixed well, and recovered at 37 °C and 200 rpm for 60 minutes. The mixture was centrifuged at 5,000 rpm for one minute to collect the bacteria. With about 100 µl of the supernatant left in the tube, the cell mass was gently resuspended by pipetting, and spread on an LB medium plate with Amp antibiotic. The plate was placed upside-down in a 37 °C incubator and cultured overnight. Single colonies were picked. After confirmation by sequencing, the positive clones were cultured, and the base-editor plasmids were extracted with the Endo-Free Plasmid Mega Kit (TIANGEN: DP120-01), measured for the concentration, and stored in a -20°C refrigerator for later use. The nucleotide sequence encoding the base-editor fusion protein 005V1-10-3-D1069A-dCasY6 is SEQ ID NO:34.

(4) According to the method in Step 2 of Example 3, the base-editor plasmid and the EGFP-C1 (Addgene, Plasmid #54759) plasmid were used together with the CasY6-TTR-sgRNA' or CasY6-TTR-sgRNA" expression plasmid to co-transfect 293T cells.

Forty-eight hours after transfection, the expression of EGFP fluorescent protein indicated successful cell transfection. EGFP-positive cells were sorted for the measurement of editing efficiency. The genome of the 293T cells was extracted with a kit (TIANGEN, DP304-03).

(5) The base-editing efficiency was measured according to the method in Step (3) of Example 3.

Primers were designed according to experimental requirements. The sequences of the identification primers used are shown in the following table:

| Primer Name | Specific Sequence |
|---|---|
| TTR-F' | gggtgtattactttgccatg (SEQ ID NO: 26) |
| TTR-R' | aacctttggtcattcatcaccttc (SEQ ID NO: 27) |

The results are shown in Figures 8A and 8B. The base editor made from D1069A-dCasY6 can achieve effective editing at multiple sites. As can be seen from Figure 8A, at the +2, +4, +13, +15, +16, and +17 sites of the target site mediated by CasY6-TTR-sgRNA', there was effective editing. The editing efficiency at the +13, +15, +16, and +17 sites was about 10% to about 30%.

As can be seen from Figure 8B, at the +7, +13, and +20 sites of the target site mediated by CasY6-TTR-sgRNA", there was effective editing. The base-editing efficiency at the +13 site was over 10%, and the editing efficiency at the +20 site was over 15%.

### Example 5. Obtaining CasY6 Protein Mutants

For the known Cas protein (having the amino-acid sequence of SEQ ID NO: 1, the nucleotide-coding sequence of SEQ ID NO: 2, and the corresponding direct repeat (DR) sequence of SEQ ID NO: 5), in order to improve the cleavage activity of the CasY6 protein, CasY6 mutant proteins with enhanced editing activity were obtained through directed mutagenesis and screening.

Specifically, the methods for obtaining each CasY6 mutant were as follows.

The nucleotide sequence (SEQ ID NO: 2) fragment encoding the CasY6 protein was synthesized by Suzhou Synbio Technologies Co., Ltd. The synthesized nucleotide sequence fragment encoding the CasY6 protein was constructed into an ABE8e plasmid (Addgene, Plasmid#138489) at positions 466-5160, to construct a CasY6 recombinant expression plasmid (for the plasmid map, see Figure 1A). Subsequently, based on the nucleotide-coding sequence of CasY6, PCR primers with mutation sites were designed, and expression plasmids expressing the mutants were obtained through homologous recombination.

The construction method of the CasY6-V1.1 recombinant expression plasmid was as follows.
1.The designed primers are as follows:

| Primer Name | Primer Sequence | Mutation Sites |
|---|---|---|
| 1.1-F | CACTTCGTAAGGTCGCTTGACAAGACCCTGAAGCAAAA (SEQ ID NO: 56) | L272H+S275R+C277L |
| 1.1-R | AAGCGACCTTACGAAGTGTTCCAGCTCCTTCTCGCCGA (SEQ ID NO: 57) | |

2. The CasY6 recombinant expression plasmid was amplified using the primers through the following steps.
2×Phanta Flash Master Mix (Dye Plus) high-fidelity enzyme was used for amplification. The amplification system was as follows.

| | |
|---|---|
| Component | 100 µl System |
| 2×Phanta Flash Master Mix (Dye Plus) | 50 µl |
| 1.1-F | 5 µl |
| 1.1-R | 5 µl |
| CasY6 recombinant expression plasmid | 5 ng |
| Nuclease-Free Water | to 100µl |

3. After the amplified product was purified, 100 ng of the purified amplified product was added to Dh5α competent cells for heat-shock and plating. The next day, single colonies were picked for sequencing. The plasmid with the correct sequence was the CasY6-V1.1 recombinant expression plasmid.

A total of 52 variants were obtained by the above method. The mutants are shown in the following table:

| Mutant Name | Mutations | Mutated Codons (Relative to Nucleotide Sequence Encoding Wild-type CasY6) |
|---|---|---|
| CasY6-V1.1 | L272H+S275R+C277L | CAC+AGG+CTT |
| CasY6-V2.1 | L272H+S275R+C277L+E289A+I292L | CAC+AGG+CTT+GCC+CTG |
| CasY6-V2.2 | L272H+S275R+C277L+E289L+A290N+I292L | CAC+AGG+CTT+TTG+AAT+TTG |
| CasY6-V2.3 | L272H+S275R+C277L+E289L+A290H+I2 92V | CAC+AGG+CTT+CTT+CAT+GTC |
| CasY6-V2.4 | L272H+S275R+C277L+K399P+E400F+D4 01S+E402Y+D403P | CAC+AGG+CTT+CCG+TTC+TCG+TAT +CCG |
| CasY6-V2.5 | L272H+S275R+C277L+K399I+E400C+D4 01T+E402A+D403P | CAC+AGG+CTT+ATA+TGC+ACG+GCG +CCC |
| CasY6-V2.6 | L272H+S275R+C277L+K399V+E400P+D4 01V+E402L+D403S | CAC+AGG+CTT+GTT+CCG+GTC+TTG +TCT |
| CasY6-V2.7 | L272H+S275R+C277L+K399E+E400A+D4 01C+E402R+D403P | CAC+AGG+CTT+GAA+GCG+TGC+CG G+CCG |
| CasY6-V2.8 | L272H+S275R+C277L+K399R+E400V+D4 01G+E402T+D403R | CAC+AGG+CTT+AGA+GTC+GGA+AC G+AGA |
| CasY6-V2.9 | L272H+S275R+C277L+K399T+E400P+D4 01R+E402F+D403S | CAC+AGG+CTT+ACT+CCA+AGA+TTT +TCA |
| CasY6-V2.10 | L272H+S275R+C277L+K399P+E400A+D4 01R+E402F+D403T | CAC+AGG+CTT+CCT+GCC+CGA+TTT +ACA |
| CasY6-V2.11 | L272H+S275R+C277L+K399S+E400P+D4 01H+E402T+D403T | CAC+AGG+CTT+AGC+CCC+CAC+ACG +ACT |
| CasY6-V2.12 | L272H+S275R+C277L+S404Q+C405A+E4 08I | CAC+AGG+CTT+CAG+GCG+ATT |
| CasY6-V2.13 | L272H+S275R+C277L+S404R+C405R+R4 06T+E408L | CAC+AGG+CTT+CGC+CGA+ACC+CTC |
| CasY6-V2.14 | L272H+S275R+C277L+S404Y+C405K+R4 06G+F407P+E408V | CAC+AGG+CTT+TAT+AAG+GGG+CCC +GTA |
| CasY6-V2.15 | L272H+S275R+C277L+D403R+S404K+C4 05R+R406P+F407L+E408R | CAC+AGG+CTT+CGC+AAA+CGA+CCT +TTG+CGC |
| CasY6-V2.16 | L272H+S275R+C277L+D403S+C405P+R4 06P+F407R+E408V | CAC+AGG+CTT+TCG+CCG+CCC+CGG +GTG |
| CasY6-V2.17 | L272H+S275R+C277L+L394H+D395C+R3 96Y+D397F+R398S | CAC+AGG+CTT+CAT+TGT+TAC+TTC+ TCT |
| CasY6-V2.18 | L272H+S275R+C277L+L394A+D395V+D 397S+R398A | CAC+AGG+CTT+GCT+GTC+TCT+GCT |
| CasY6-V2.19 | L272H+S275R+C277L+D397S+R398I+K3 99D+E400S+D401K+E402I | CAC+AGG+CTT+TCA+ATA+GAC+TCT +AAG+ATC |
| CasY6-V2.20 | L272H+S275R+C277L+D397N+R398G+E 400R+D401T+E402S | CAC+AGG+CTT+AAC+GGA+CGG+AC T+TCA |
| CasY6-V2.21 | L272H+S275R+C277L+D397H+R398A+K 399S+E400A+D401M+E402S | CAC+AGG+CTT+CAT+GCA+TCT+GCA +ATG+TCG |
| CasY6-V2.22 | L272H+S275R+C277L+D397R+R398D+K 399R+E400S+D401P+E402S | CAC+AGG+CTT+CGA+GAT+CGA+TCA +CCC+TCT |
| CasY6-V2.23 | L272H+S275R+C277L+D397N+R398G+E 400R+D401T+E402S | CAC+AGG+CTT+AAC+GGA+CGG+AC T+TCA |
| CasY6-V2.24 | S242Y+S243A+S246R+L272H+S275R+C2 77L | TAT+GCA+CGA+CAC+AGG+CTT |
| CasY6-V2.25 | G119P+D120L+V1215+Q122K+Y123F+L2 72H+S275R+C277L | CCT+CTG+TCG+AAA+TTC+CAC+AGG +CTT |
| CasY6-V2.26 | E118D+G119S+D120K+V121S+Q122G+Y 123F+L272H+S275R+C277L | GAC+TCA+AAA+TCA+GGT+TTT+CAC +AGG+CTT |
| CasY6-V2.27 | E118Y+G119S+D120G+V121P+Q122G+Y 123L+L272H+S275R+C277L | TAC+AGT+GGC+CCA+GGC+CTA+CAC +AGG+CTT |
| CasY6-V2.28 | E118S+G119D+D120S+V121A+Q122F+Y 123L+L272H+S275R+C277L | TCG+GAT+TCT+GCT+TTC+CTC+CAC+ AGG+CTT |
| CasY6-V2.29 | E118K+G119S+D120T+V121G+Q122G+Y 1231+L272H+S275R+C277L | AAA+AGC+ACA+GGG+GGT+ATT+CA C+AGG+CTT |
| CasY6-V2.30 | E118Q+G119S+D120S+V121T+Q122G+Y 123F+L272H+S275R+C277L | CAG+TCA+TCC+ACG+GGG+TTC+CAC +AGG+CTT |
| CasY6-V2.31 | E118T+G119D+D120T+V121S+Q122K+Y 123I+L272H+S275R+C277L | ACA+GAC+ACA+TCA+AAG+ATC+CAC +AGG+CTT |
| CasY6-V2.32 | E118I+G119P+D120E+V121P+Q122H+Y 123F+L272H+S275R+C277L | ATT+CCC+GAA+CCC+CAC+TTC+CAC+ AGG+CTT |
| CasY6-V2.33 | E118P+G119R+D120T+V121G+Q122R+L 272H+S275R+C277L | CCT+AGG+ACC+GGC+CGA+CAC+AG G+CTT |
| CasY6-V2.34 | E118P+G119P+D120N+V121R+Q122R+Y 123I+L272H+S275R+C277L | CCC+CCG+AAC+CGA+AGA+ATA+CAC +AGG+CTT |
| CasY6-V2.35 | E118s+G119M+D120s+Q122s+Y123T+L 272H+S275R+C277L | TCG+ATG+TCC+TCG+ACG+CAC+AGG +CTT |
| CasY6-V2.36 | G119S+V121S+Q122K+Y123S+L272H+S2 75R+C277L | AGC+TCG+AAG+TCT+CAC+AGG+CTT |
| CasY6-V2.37 | E118S+G119D+V121E+Q122Y+Y123T+L272H+S275R+C277L | TCC+GAC+GAG+TAC+ACA+CAC+AGG+CTT |
| CasY6-V2.38 | G119L+D120S+V121S+Q122T+L272H+S 275R+C277L | TTA+TCA+AGT+ACA+CAC+AGG+CTT |
| CasY6-V3.1 | L272H+S275R+C277L+D397N+R398G+E 400R+D401T+E402S+L789S | CAC+AGG+CTT+AAC+GGA+CGG+AC T+TCA+AGC |
| CasY6-V3.2 | L272H+S275R+C277L+T285R+S286F+C2 87I+D288W+D403S+C405P+R406P+F40 7R+E408V+K502R+K557N | CAC+AGG+CTT+CGC+TTC+ATA+TGG +TCG+CCG+CCC+CGG+GTG+CGG+A AC |
| CasY6-V3.3 | L272H+S275R+C277L+E289R+I292L+K39 9S+E400P+D401H+E402T+D403T | CAC+AGG+CTT+CGT+TTG+AGC+CCC +CAC+ACG+ACT |
| CasY6-V3.4 | L272H+S275R+C277L+E289S+A290V+I2 92L+P758L+V860L | CAC+AGG+CTT+TCC+GTC+CTT+CTC+ CTC |
| CasY6-V3.5 | L272H+S275R+C277L+K299R+R300D+W 301F+C672R+P758L | CAC+AGG+CTT+CGG+GAC+TTC+CGG +CTC |
| CasY6-V3.6 | L272H+S275R+C277L+D397N+R398G+E 400R+D401T+E402S+L657F+L789S | CAC+AGG+CTT+AAC+GGA+CGG+AC T+TCA+TTC+AGC |
| CasY6-V3.7 | E118P+G119R+D120T+V121G+Q122R+L 272H+S275R+C277L+C672R+P758L | CCT+AGG+ACC+GGC+CGA+CAC+AG G+CTT+CGG+CTC |
| CasY6-V3.8 | L272H+S275R+C277L+K399L+E400T+D4 01F+E402G+D403G+P758L+K827E | CAC+AGG+CTT+CTG+ACG+TTC+GGC +GGT+CTC+GAG |
| CasY6-V3.9 | E118P+G119R+D120T+V121G+Q122R+L 272H+S275R+C277L+L394R+D395S+R39 6T+D397T+R398N+C672R+P758L | CCT+AGG+ACC+GGC+CGA+CAC+AG G+CTT+CGT+AGC+ACC+ACG+AAC+C GG+CTC |
| CasY6-V3.10 | E118P+G119R+D120T+V121G+Q122R+L 272H+S275R+C277L+L394P+D395P+R39 6G+D397F+C672R+P758L | CCT+AGG+ACC+GGC+CGA+CAC+AG G+CTT+CCT+CCT+GGA+TTT+CGG+CT C |
| CasY6-V3.11 | E118P+G119R+D120T+V121G+Q122R+L 272H+S275R+C277L+D403S+C405R+R40 6L+F407S+E408R+C672R+P758L | CCT+AGG+ACC+GGC+CGA+CAC+AG G+CTT+AGC+AGA+CTG+TCG+CGC+C GG+CTC |
| CasY6-V3.12 | E118P+G119R+D120T+V121G+Q122R+L 272H+S275R+C277L+D403S+S404P+C40 5L+R406H+F407M+E408V+C672R+P758 L | CCT+AGG+ACC+GGC+CGA+CAC+AG G+CTT+TCT+CCC+CTA+CAT+ATG+GT A+CGG+CTC |
| CasY6-V3.13 | K241A+S242F+S243C+S246R+L272H+S2 75R+C277L+D397N+R398G+E400R+D401T+E402S+L789S | GCT+TTT+TGT+CGA+CAC+AGG+CTT +AAC+GGA+CGG+ACT+TCA+AGC |

### Example 6. Evaluation of the Cleavage Activity of CasY6 Mutants

### 1. Construction of TTR-sgRNA Expression Plasmid

(1) Based on the target sequence of the TTR-3 locus of the TTR gene, tagaagggatatacaaagtg (SEQ ID NO: 48), a TTR-3-sgRNA sequence was designed, and oligonucleotides (oligos) were synthesized:
CasY6-TTR-3-sgRNA sequence:
TATCCATCGTGCCGCCTCTTGGCACtagaagggatatacaaagtg (SEQ ID NO: 49), wherein the underlined part of the sequence is the DR sequence, and the remaining is the spacer sequence.
(2) A sequence CACC was added to the 5'-end of the upstream sequence of the TTR-sgRNA, and a sequence AAAA was added to the 5'-end of the downstream sequence, and the oligos were synthesized. The specific sequences are as follows:

| sgRNA Name | Up- and Down-stream Primer Sequences |
|---|---|
| | CasY6-TTR-3-sgRNA-Oligo-F : |
| CasY6-TTR-3-sgRNA | CACCTATCCATCGTGCCGCCTCTTGGCAC tagaagggatatacaaagtg(SEQ ID NO: 50) |
| | CasY6-TTR-3-sgRNA-Oligo-R: |
| | AAAACACTTTGTATATCCCTTCTAGTGCCAAGAGGCGGCACGATGGATA (SEQ ID NO: 51) |

After the upstream and downstream primers of the aforementioned TTR-sgRNA were synthesized, annealing was carried out through a preset program (95 °C, 5 min; from 95 °C to 85 °C at a rate of -2 °C/s; from 85 °C to 25 °C at a rate of -0.1 °C/s; held at 4 °C). Then, the annealed product was ligated to a PHK09T vector linearized by BsmBI (NEB, # R0739L). The sequence of the PHK09T vector is SEQ ID NO: 3, and the plasmid map is shown in Figure 5.

The linearization of the PHK09T vector and its ligation with the annealed product of TTR-3-sgRNA were performed as follows.

First, the PHK09T vector was linearized in a linearization system as follows:
3 µg of the PHK09T vector; 6 µL of a buffer (NEB: R0739L); 2 µL of BsmBl; ddH₂O making up to 60 µL, which were digested at 55 °C overnight.

The system for ligation of the annealed product of TTR-gRNA and the linearized vector:
1 µL of a T4 ligase buffer (NEB, #M0202L), 20 ng of the linearized vector, 5 µL of the annealed oligo fragment, 0.5 µL of T4 ligase (NEB, #M0202L), ddH₂O making up to 10 µL, which was subjected to ligation at 16 °C overnight to obtain the CasY6-TTR-3-sgRNA expression plasmid.

(3) The CasY6-TTR-3-sgRNA expression plasmid obtained in step (2) was transferred into Escherichia coli DH5α competent cells (Vazyme, DL1001). The specific steps are as follows:
DH5α competent cells were taken out from a -80 °C refrigerator and quickly buried in ice. After 5 minutes, the cell mass thawed, and the ligation product was added. They were gently mixed by tapping the bottom of the centrifuge tube by hand, let stand in ice for 25 minutes, heat-shocked in a 42 °C water-bath for 45 seconds, quickly put back on ice, and let stand for 2 minutes. 700 µl of a sterile LB medium without antibiotics was added to the centrifuge tube, mixed well, and recovered at 37 °C and 200 rpm for 60 minutes. The mixture was centrifuged at 3,000 rpm for one minute to collect the bacteria. With about 100 µl of the supernatant left in the tube, the cell mass was gently resuspended by pipetting, and spread on an LB medium plate with Amp antibiotic. The plate was placed upside-down in a 37 °C incubator and cultured overnight. Single colonies were picked. After confirmation by sequencing, the positive clones were cultured, and the plasmids were extracted (with the Endo-Free Plasmid Mega Kit,TIANGEN: DP120-01), measured for the concentration, and stored in a -20°C refrigerator for later us.

### 2. Measurement of the Cleavage Activity of Each Variant Using a Dual-Luciferase Reporter System

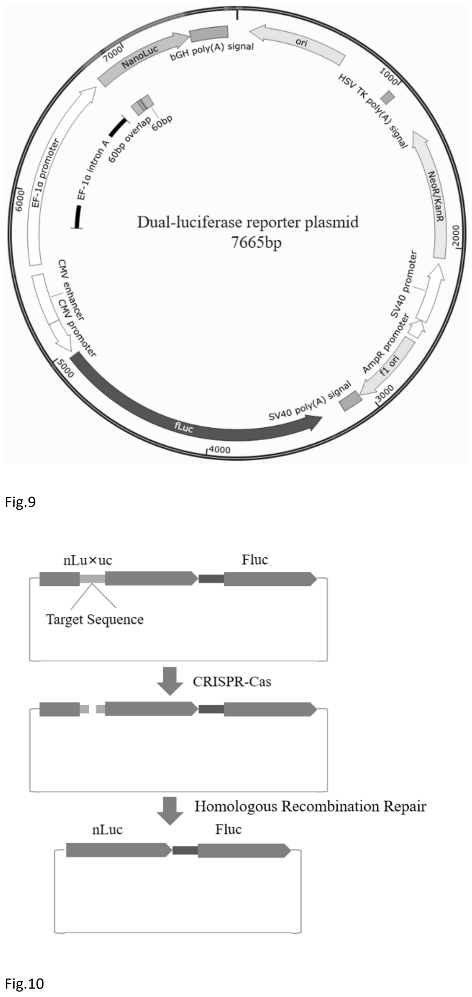

To sensitively detect the cleavage activity of the CasY6 variants, a dual-luciferase reporter system was constructed (for the plasmid map, see Figure 9, and the plasmid sequence is SEQ ID NO: 52). Firefly luciferase (Fluc) was used as the internal reference, and NanoLuc (nLuc) was used as the reporter gene. Luciferase can catalyze the oxidation of luciferin to oxyluciferin, and during the oxidation of luciferin, bioluminescence is emitted.

Fluc normally catalyzed the substrate to emit light, indicating successful transfer of the reporter vector. In nLu×uc, the LU and UC sequences were the 119-nt N-terminal sequence and the 469-nt C-terminal sequence encoding nLuc, respectively, and these two sequences had an overlap of 60 nt. The middle of nLu×uc had an inserted fragment (SEQ ID NO: 53) which contains the target sequence (SEQ ID NO: 48) targeted by the CRISPR/Cas system. There was a TAG premature stop codon in the middle of the target sequence. When it was cleaved, nLu×uc used the recombination mechanism to generate the correct nLuc coding frame, the nLuc gene was expressed such that the cells changed from a state not expressing NanoLuc into a state expressing it, and then it catalyzed the substrate to emit bioluminescence (Figure 10).

In a 96-well plate, the TTR-3-sgRNA expression plasmid (50 ng), the dual-luciferase reporter system plasmid (50 ng), and each of the CasY6 mutant recombinant plasmids (50 ng) obtained in Example 5 were used to co-transfect HEK293 cells (purchased from ATCC) in each well by the PEI method. After 24 hours of culturing, the expression intensities of Fluc and nLuc were measured on a microplate reader by using a dual-luciferase reporter gene detection kit (Beyotime, RG028), and the cleavage activity of each CasY6 variant was characterized by the fluorescence intensity of nLuc.

The editing efficiencies of wild-type CasY6 and each mutant are as follows:

| Mutant | Editing Efficien cy(%) | Mutant | Editin g Efficie ncy(%) | Mutant | Editing Efficie ncy(%) | Mutant | Editing Efficienc y(%) |
|---|---|---|---|---|---|---|---|
| CasY6 | 11.00 | CasY6-V2.13 | 23.37 | CasY6-V2.27 | 25.02 | CasY6-V3.3 | 40.05 |
| CasY6-V1.1 | 19.28 | CasY6-V2.14 | 25.83 | CasY6-V2.28 | 27.84 | CasY6-V3.4 | 36.47 |
| CasY6-V2.1 | 26.94 | CasY6-V2.15 | 27.27 | CasY6-V2.29 | 27.50 | CasY6-V3.5 | 35.95 |
| CasY6-V2.2 | 20.50 | CasY6-V2.16 | 26.25 | CasY6-V2.30 | 31.70 | CasY6-V3.6 | 38.09 |
| CasY6-V2.3 | 30.32 | CasY6-V2.17 | 23.89 | CasY6-V2.31 | 28.40 | CasY6-V3.7 | 35.82 |
| CasY6-V2.4 | 24.84 | CasY6-V2.18 | 25.61 | CasY6-V2.32 | 30.87 | CasY6-V3.8 | 36.36 |
| CasY6-V2.5 | 24.22 | CasY6-V2.19 | 20.50 | CasY6-V2.33 | 30.79 | CasY6-V3.9 | 37.69 |
| CasY6-V2.6 | 25.61 | CasY6-V2.20 | 32.31 | CasY6-V2.34 | 26.32 | CasY6-V3.10 | 42.47 |
| CasY6-V2.7 | 22.49 | CasY6-V2.21 | 22.59 | CasY6-V2.35 | 26.58 | CasY6-V3.11 | 36.53 |
| CasY6-V2.8 | 24.64 | CasY6-V2.22 | 28.82 | CasY6-V2.36 | 21.59 | CasY6-V3.12 | 39.58 |
| CasY6-V2.9 | 26.40 | CasY6-V2.23 | 25.57 | CasY6-V2.37 | 24.61 | CasY6-V3.13 | 36.45 |
| CasY6-V2.10 | 31.98 | CasY6-V2.24 | 20.66 | CasY6-V2.38 | 31.90 | | |
| CasY6-V2.11 | 28.41 | CasY6-V2.25 | 30.58 | CasY6-V3.1 | 39.80 | | |
| CasY6-V2.12 | 21.54 | CasY6-V2.26 | 30.62 | CasY6-V3.2 | 36.89 | | |

Through analysis, it can be seen that, compared with CasY6, the variants showed various degrees of increase in cleavage activity. In particular, the cleavage activities of CasY6-V3.1, CasY6-V3.3, and CasY6-V3.12 were close to or exceed 40%. CasY6-V3.3 exhibited the highest cleavage activity.

### Example 7. Measurement of the Effective Spacer Sequence Length of CasY6

The dual-plasmid fluorescence reporter system in Example 6 was used. In order to measure the effective spacer sequence length of CasY6, spacer sequences of different lengths from 15 to 25 nt targeting the TTR gene locus (SEQ ID NO: 48, 58-64) were designed to target the LUxUC intermediate inserted fragment (SEQ ID NO:53) of the dual-luciferase reporter plasmid in Example 6. The LUxUC intermediate inserted fragment contains the same target sequence as the 25-nt spacer sequence (SEQ ID NO:64). According to the construction method in Example 6, crRNA expression vectors containing spacer sequences of different lengths (any one of SEQ ID NO:48 or SEQ ID NO:58-64) were constructed respectively, while the other components of the fluorescence reporter system remained unchanged.

Through the experimental procedures in Example 6, crRNA expression plasmids with spacer sequences of different lengths and the CasY6-V3.3 mutant plasmid were prepared. In a 96-well plate, the crRNA expression plasmids of different lengths (50 ng), the dual-luciferase reporter system plasmid (50 ng), and the CasY6-V3.3 mutant plasmid (50 ng) were used to co-transfect HEK293 cells in each well by the PEI method. After 24 hours of culturing, the expression intensities of Fluc and nLuc were measured on a microplate reader by using a dual-luciferase reporter gene detection kit (Beyotime, RG028), and the cleavage activity of each CasY6 variant was characterized by the fluorescence intensity of nLuc. It was observed that the spacer sequence length in the range of 15-25 nt was effective for the spacer-sequence-specific cleavage activity of CasY6-V3.3, with the best cleavage activity at 18-20 nt. The spacer sequences of different lengths and the corresponding cleavage activities are shown in the following table (the 20-nt spacer sequence in the table is the spacer sequence (SEQ ID NO:48) targeting the TTR-3 locus in Example 6).

| Length (nt) | Spacer Sequence | SEQ ID NO: | Editing Efficiency (%) |
|---|---|---|---|
| 15 | tagaagggatataca | 58 | 38 |
| 18 | tagaagggatatacaaag | 59 | 48 |
| 19 | tagaagggatatacaaagt | 60 | 49 |
| 20 | tagaagggatatacaaagtg | 48 | 47 |
| 21 | tagaagggatatacaaagtgg | 61 | 38 |
| 22 | tagaagggatatacaaagtgga | 62 | 38 |
| 23 | tagaagggatatacaaagtggaa | 63 | 30 |
| 25 | tagaagggatatacaaagtggaaat | 64 | 21 |

The above examples demonstrate that the CRISPR-CasY6 system can achieve specific and efficient gene editing in prokaryotic cells and mammalian cells, and outperforms other Cas12 systems (such as the LbCpf1 system). The CasY6 protein of the present disclosure has a relatively small size, and the crRNA has a relatively short length. It is suitable for multiplex gene editing applications in subjects and can achieve delivery in various ways (such as LNP, AAV), thus showing great potential in therapeutic gene-editing applications.

Through the engineering design of the CasY6 protein, it has been confirmed that the CasY6 system can be used for base-editing applications. Regarding the base editors, the D1069A-dCasY6 system effectively edits at multiple sites of the TTR locus. At the target site mediated by CasY6-TTR-sgRNA', the editing efficiency at the +13, +15, +16, and +17 sites was about 10% to about 30%. At the target site mediated by CasY6-TTR-sgRNA", the base-editing efficiency at the +13 site was over 10%, and the editing efficiency at the +20 site was over 15%, indicating that it has greater potential as a base editor.

Through directed mutagenesis and screening, 52 variants including CasY6-V1.1 to CasY6-V3.13 were obtained, among which multiple variants exhibit specific and more efficient editing activities.

In summary, the CasY6 system of the present disclosure has robust editing activity and high specificity, serving as a versatile platform for genome editing or base editing in mammalian cells, and can be used for *in vivo* or *ex vivo* therapeutic applications in the future.

### SEQUENCE INFORMATION:

**SEQ ID NO: 1, Amino acid sequence of CasY6 protein** 
MIKNQIIGYEVKLDLNDRKRAFVRDTLRYFAQTSDVFRKGLMCLFAGVDPEMISGYLPEGESGDLWCAVNWFRLV
DLSEGLVSEERVVDGSELCRRFQDYAGHAPDRQAEIYLTRRSEGDVQYVWADCRAGYRSFCREVGLSPDQMGFDV
LALVARGYIKFDKGENDAWGFVSAVFGDGVKEDTKNKIRMLEGMRRAAHAKPATYEELQESALRETGCSDIKQMI
REYKSGQQDDKEGVRKSSGGSHAFFVKYFLRGADRVCGPIGEKELELFVSSCDKTLKQKTSCDEAPITHSAVWKRWF
KRRIPHTVGSIQEPYSVACDQAMADISSKNCLNARMVSCPDSGSVAERRKKGTKAKKVGSSGVLEMQDKVSSLSSK
GVDLAKSVFDSFNDALDRDRKEDEDSCRFEVLRHHLGKLSDLYEIWVKNDCDSGIGLYLEEYEGSIKKQPKEELLRAIY
PHRESIKAKEFMDAAHRNDLIQKIESRKAHPTVRGNRGVGYGKSKMRGKIMPQSNMHNGRKAGLNPEMWLEAD
LLNGERWEKHHLKFHSSQFFDQVYCHKDGLPYTKIPGLPRLARHGMPIGLSISPDDVPLVNRVSGNGRRCPRANKR
NLRAVANITHNVELHPETAFEIRSNGEYASISHRVQDVAKKSIEAGDRSLGYDRNQTAQDTYAVLCVVNEGGYAHR
GNLVELVAVGKISSKSMKRRTDGSMYECDDLAYDGLPYAEFSEWREERKAFMHRLIPLLPPATKKLGSSREPMDLPK
ELEALTHCKSLYVWNRRFLIIFRVALKRALKEQASNLFRQEILDICHANLSPLRRSSLSKSGVESLQKLKSLVNAYLSKM
 
KAKREDPLLEADPEFAELFKVITERLKAKRLEKAKRTASDIVRLANLHGCSFVSGESDLPTTSKVNKKFQNASNTDWM
ARKVEDLVKSMCIRSGIAFRGIDPSYTSLRDPLQFETDGRSVIRCRFWKAENGSAPEYVIDGLVRYTKHAVKNMEQS
RPLSSFHRGGLAFCRKYGIDPARLTPKVVRGVLARHEVVYLPRAGGRYFLATLPLVAGAPVFEWDGQQMYVLDADE
VAAINIALEAIPSKLP
**SEQ ID NO: 2, Coding sequence of CasY6** 
ATGATCAAGAACCAAATCATCGGATATGAAGTGAAGCTTGACCTCAACGACAGGAAGAGGGCTTTTGTTAGG
GATACCTTGCGGTACTTCGCCCAGACATCGGATGTGTTCCGAAAGGGCCTGATGTGCCTGTTCGCTGGCGTCG
ACCCCGAGATGATAAGCGGCTACCTGCCCGAGGGGGAATCCGGCGACCTGTGGTGCGCCGTCAACTGGTTCC
GCCTCGTGGATCTGTCGGAGGGTCTTGTCTCCGAGGAGCgGGTGGTAGACGGGTCGGAGCTGTGCCGCAGGT
TCCAAGACTACGCAGGTCATGCGCCCGACCGGCAGGCGGAGATATACCTGACTAGGAGGAGCGAGGGCGAT
GTCCAGTACGTTTGGGCCGACTGCCGTGCTGGCTACCGCAGCTTCTGCCGTGAGGTGGGACTGAGCCCAGACC
AGATGGgATTTGACGTGCTTGCGCTTGTGGCAAGGGGCTACATCAAGTTCGACAAGGGCGAAAACGATGCCT
GGGGGTTTGTATCCGCCGTCTTCGGCGACGGCGTCAAGGAGGACACCAAGAACAAGATTCGCATGCTCGAGG
GCATGAGGAGAGCcGCACACGCCAAGCCGGCCACCTACGAGGAGCTTCAGGAATCGGCGCTGAGGGAGACG
GGATGCAGCGACATCAAGCAAATGATCAgGGAGTACAAGTCCGGTCAGCAGGACGACAAGGAAGGCGTCAG
AAAAAGCAGCGgCGGCAGTCACGCATTCTTCGTGAAATACTTCTTGAGGGGAGCCGATCGGGTATGCGGCCCC
ATCGGCGAGAAGGAGCTGGAATTGTTcGTCAGTAGCTGCGACAAGACCCTGAAGCAAAAAACATCGTGCGAC
GAGGCGCCGATCACGCACAGcGCCGTGTGGAAGAGGTGGTTCAAGAGGAGGATACCGCACACCGTtGGTTCA
ATCCAGGAACCATACAGCGTTGCGTGCGACCAAGCCATGGCCGACATAAGCTCTAAGAACTGCCTGAACGCCC
GCATGGTGAGCTGCCCAGACAGCGGCTCGGTGGCCGAGCGCAGGAAGAAGGGCACCAAGGCGAAGAAGGTC
GGGTCATCCGGCGTGCTCGAGATGCAGGACAAGGTTTCGTCCCTGTCGAGCAAGGGCGTTGATCTTGCGAAG
TCCGTATTCGACAGCTTCAACGATGCTCTGGACAGgGACCGCAAGGAGGACGAGGATTCCTGCCGGTTCGAGG
TCCTCAGGCATCACCTCGGCAAGCTTTCCGACCTTTACGAGATTTGGGTGAAGAACGACTGCGACTCCGGCATA
GGTCTaTATCTGGAGGAATACGAAGGTTCGATTAAAAAACAGCCGAAAGAGGAACTGCTGCGTGCGATCTACC
CGCATCGAGAGTCAATAAAGGCCAAGGAGTTCATGGACGCAGCGCACCGCAACGACCTCATCCAAAAGATCG
AGTCCCGCAAGGCGCACCCGACTGTGCGTGGCAACAGGGGCGTCGGTTACGGCAAaTCCAAGaTGAGGGGCA
AGATAATGCCGCAGTCCAACATGCACAAcGGTCGCAAgGCCGGCCTGAACCCCGAGATGTGGCTCGAGGCGG
ATCTCCTGAACGGGGAGAGGTGGGAGAAaCATCACCTGAAGTTCCACTCGTCGCAGTTCTTCGATCAGGTCTA
CTGCCACAAGGACGGACTGCCCTACACGAAAATCCCTGGCCTGCCGAGGTTGGCACGGCACGGCATGCCCATC
GGGCTGTCGATAAGCCCCGATGACGTTCCTCTGGTGAACCGTGTGTCCGGCAACGGCAGGCGTTGCCCGAGG
GCGAACAAGAGAAACCTgAGGGCCGTCGCCAACATCACTCACAACGTGGAACTCCACCCGGAaACAGCCTTCG
AGATACGCAGCAACGGCGAATACGCAAGCATCAGCCACAGGGTGCAAGATGTCGCCAAGAAGAGCATCGAG
GCCGGCGATCGAAGTCTTGGCTACGACCGAAATCAGACCGCACAGGACACCTACGCAGTACTGTGTGTCGTCA
ACGAGGGAGGTTATGCCCACAGGGGCAACTTGGTCGAGCTCGTCGCCGTCGGCAAGATCTCGAGCAAGAGCA
TGAAGCGCAGGACGGACGGCTCGATGTACGAGTGCGACGACCTGGCCTATGACGGCTTGCCTTATGCGGAGT
 
TCTCCGAGTGGAGGGAGGAACGGAAAGCATTCATGCATCGCCTGATCCCGCTGCTGCCTCCGGCAACCAAGA
AGCTTGGCAGCAGCAGGGAGCCaATGGATCTGCCGAAGGAACTCGAGGCCCTGACGCACTGCAAGTCGCTgT
ACGTGTGGAACCGCAGGTTCCTGAtCATCTTCCGGgTCGCACTGAAGAGGGCCttGAAGGAACAGGCGtCCaAT
CTGTTCCGCCAGGAGATACTCGACATCTGTCACGCCAATCTTAGCCCGCTGAGACGAAGCAGCtTGAGCAAGtc
CGGAGTTGAATCtCTTCAGAAGCTGAAgaGcCTCGTCAACGCCTACCTCAGCAAAATGAAGGCGAAGAgGgAgG
AcCCACTGCTTGAGGCCGACCCAGAGTTCGCCGAGCTGTTCAAGGTAATCAcCGAgCGTCTCAAGGCGAAAAG
GCTCGAGAAGGCCAAACGCACAGCCAGCGATATCGTCCGGCTGGCCAACCTGCACGGCTGCTCCTTCGTCTCC
GGCGAGTCCGACCTGCCGACGACCAGCAAGGTCAACAAGAAATTCCAGAACGCAAGCAACAcGGACTGGATG
GCTCGCAAGGTCGAGGACCTCGTCAAGAGCATGTGCATTAGGTCTGGCATCGCCTTTAGAGGCATCGACCCGT
CCTACACGAGCCTGCGTGATCCGTTACAGTTCGAaACCGACGGCAGGAGCGTCATCCGATGCCGTTTCTGGAA
GGCGGAAAATGGCTCGGCTCCGGAATATGTCATCGATGGTCTGGTGCGTTACACGAAGCACGCCGTCAAGAA
CATGGAGCAGTCAAGGCCCCTGTCGTCCTTCCACAGAGGCGGGCTGGCCTTCTGCCGCAAGTACGGCATCGAC
CCAGCGAGACTGACGCCCAAGGTCGTCCGTGGCGTGCTGGCGAGGCACGAGGTGGTATACTTGCCACGTGCC
GGCGGCAGGTACTTCCTCGCCACGCTGCCGCTGGTGGCGGGGGCTCCCGTCTTCGAGTGGGATGGCCAGCAG
ATGTATGTCCTCGACGCCGACGAGGTCGCTGCGATCAACATCGCACTCGAAGCCATACCTTCCAAGCTACCG
**SEQ ID NO: 3, PHK09T** 
TGAGCTGATACCGCTCGCCGCAGCCGAACGACCGAGCGCAGCGAGTCAGTGAGCGAGGAAGCGGAAGAGCG
CCCAATACGCAAACCGCCTCTCCCCGCGCGTTGGCCGATTCATTAATGCAGCTGGCACGACAGGTTTCCCGACT
GGAAAGCGGGCAGTGAGCGCAACGCAATTAATGTGAGTTAGCTCACTCATTAGGCACCCCAGGCTTTACACTT
TATGCTTCCGGCTCGTATGTTGTGTGGAATTGTGAGCGGATAACAATTTCACACAGGAAACAGCTATGACCAT
GATTACGCCAAGCGCGCAATTAACCCTCACTAAAGGGAACAAAAGCTGGAGCTGCAAGCTTAATGTAGTCTTA
TGCAATACTCTTGTAGTCTTGCAACATGGTAACGATGAGTTAGCAACATGCCTTACAAGGAGAGAAAAAGCAC
CGTGCATGCCGATTGGTGGAAGTAAGGTGGTACGATCGTGCCTTATTAGGAAGGCAACAGACGGGTCTGACA
TGGATTGGACGAACCACTGAATTGCCGCATTGCAGAGATATTGTATTTAAGTGCCTAGCTCGATACATAAACG
GGTCTCTCTGGTTAGACCAGATCTGAGCCTGGGAGCTCTCTGGCTAACTAGGGAACCCACTGCTTAAGCCTCAA
TAAAGCTTGCCTTGAGTGCTTCAAGTAGTGTGTGCCCGTCTGTTGTGTGACTCTGGTAACTAGAGATCCCTCAG
ACCCTTTTAGTCAGTGTGGAAAATCTCTAGCAGTGGCGCCCGAACAGGGACTTGAAAGCGAAAGGGAAACCA
GAGGAGCTCTCTCGACGCAGGACTCGGCTTGCTGAAGCGCGCACGGCAAGAGGCGAGGGGCGGCGACTGGT
GAGTACGCCAAAAATTTTGACTAGCGGAGGCTAGAAGGAGAGAGATGGGTGCGAGAGCGTCAGTATTAAGC
GGGGGAGAATTAGATCGCGATGGGAAAAAATTCGGTTAAGGCCAGGGGGAAAGAAAAAATATAAATTAAAA
CATATAGTATGGGCAAGCAGGGAGCTAGAACGATTCGCAGTTAATCCTGGCCTGTTAGAAACATCAGAAGGCT
GTAGACAAATACTGGGACAGCTACAACCATCCCTTCAGACAGGATCAGAAGAACTTAGATCATTATATAATAC
AGTAGCAACCCTCTATTGTGTGCATCAAAGGATAGAGATAAAAGACACCAAGGAAGCTTTAGACAAGATAGA
GGAAGAGCAAAACAAAAGTAAGACCACCGCACAGCAAGCGGCCGCTGATCTTCAGACCTGGAGGAGGAGAT
ATGAGGGACAATTGGAGAAGTGAATTATATAAATATAAAGTAGTAAAAATTGAACCATTAGGAGTAGCACCCA
 
CCAAGGCAAAGAGAAGAGTGGTGCAGAGAGAAAAAAGAGCAGTGGGAATAGGAGCTTTGTTCCTTGGGTTC
TTGGGAGCAGCAGGAAGCACTATGGGCGCAGCGTCAATGACGCTGACGGTACAGGCCAGACAATTATTGTCT
GGTATAGTGCAGCAGCAGAACAATTTGCTGAGGGCTATTGAGGCGCAACAGCATCTGTTGCAACTCACAGTCT
GGGGCATCAAGCAGCTCCAGGCAAGAATCCTGGCTGTGGAAAGATACCTAAAGGATCAACAGCTCCTGGGGA
TTTGGGGTTGCTCTGGAAAACTCATTTGCACCACTGCTGTGCCTTGGAATGCTAGTTGGAGTAATAAATCTCTG
GAACAGATTTGGAATCACACGACCTGGATGGAGTGGGACAGAGAAATTAACAATTACACAAGCTTAATACACT
CCTTAATTGAAGAATCGCAAAACCAGCAAGAAAAGAATGAACAAGAATTATTGGAATTAGATAAATGGGCAA
GTTTGTGGAATTGGTTTAACATAACAAATTGGCTGTGGTATATAAAATTATTCATAATGATAGTAGGAGGCTTG
GTAGGTTTAAGAATAGTTTTTGCTGTACTTTCTATAGTGAATAGAGTTAGGCAGGGATATTCACCATTATCGTTT
CAGACCCACCTCCCAACCCCGAGGGGACCCAGAGAGGGCCTATTTCCCATGATTCCTTCATATTTGCATATACG
ATACAAGGCTGTTAGAGAGATAATTAGAATTAATTTGACTGTAAACACAAAGATATTAGTACAAAATACGTGA
CGTAGAAAGTAATAATTTCTTGGGTAGTTTGCAGTTTTAAAATTATGTTTTAAAATGGACTATCATATGCTTACC
GTAACTTGAAAGTATTTCGATTTCTTGGCTTTATATATCTTGTGGAAAGGACGAAACACCGGAGACGGACGTCT
CTTTTTTTAAGCTTGGCGTAACTAGATCTTGAGACAAATGGCAGTATTCATCCACAATTTTAAAAGAAAAGGGG
GGATTGGGGGGTACAGTGCAGGGGAAAGAATAGTAGACATAATAGCAACAGACATACAAACTAAAGAATTAC
AAAAACAAATTACAAAAATTCAAAATTTTCGGGTTTATTACAGGGACAGCAGAGATCCACTTTGGCGCCGGCTC
GAGGGGGCCCGGGTGCAAAGATGGATAAAGTTTTAAACAGAGAGGAATCTTTGCAGCTAATGGACCTTCTAG
GTCTTGAAAGGAGTGGGAATTGGCTCCGGTGCCCGTCAGTGGGCAGAGCGCACATCGCCCACAGTCCCCGAG
AAGTTGGGGGGAGGGGTCGGCAATTGATCCGGTGCCTAGAGAAGGTGGCGCGGGGTAAACTGGGAAAGTG
ATGTCGTGTACTGGCTCCGCCTTTTTCCCGAGGGTGGGGGAGAACCGTATATAAGTGCAGTAGTCGCCGTGAA
CGTTCTTTTTCGCAACGGGTTTGCCGCCAGAACACAGGTAAGTGCCGTGTGTGGTTCCCGCGGGCCTGGCCTCT
TTACGGGTTATGGCCCTTGCGTGCCTTGAATTACTTCCACCTGGCTGCAGTACGTGATTCTTGATCCCGAGCTTC
GGGTTGGAAGTGGGTGGGAGAGTTCGAGGCCTTGCGCTTAAGGAGCCCCTTCGCCTCGTGCTTGAGTTGAGG
CCTGGCCTGGGCGCTGGGGCCGCCGCGTGCGAATCTGGTGGCACCTTCGCGCCTGTCTCGCTGCTTTCGATAA
GTCTCTAGCCATTTAAAATTTTTGATGACCTGCTGCGACGCTTTTTTTCTGGCAAGATAGTCTTGTAAATGCGGG
CCAAGATCTGCACACTGGTATTTCGGTTTTTGGGGCCGCGGGCGGCGACGGGGCCCGTGCGTCCCAGCGCAC
ATGTTCGGCGAGGCGGGGCCTGCGAGCGCGGCCACCGAGAATCGGACGGGGGTAGTCTCAAGCTGGCCGGC
CTGCTCTGGTGCCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGACGCGGCGCTC
GGGAGAGCGGGCGGGTGAGTCACCCACACAAAGGAAAAGGGCCTTTCCGTCCTCAGCCGTCGCTTCATGTGA
CTCCACGGAGTACCGGGCGCCGTCCAGGCACCTCGATTAGTTCTCGAGCTTTTGGAGTACGTCGTCTTTAGGTT
GGGGGGAGGGGTTTTATGCGATGGAGTTTCCCCACACTGAGTGGGTGGAGACTGAAGTTAGGCCAGCTTGGC
ACTTGATGTAATTCTCCTTGGAATTTGCCCTTTTTGAGTTTGGATCTTGGTTCATTCTCAAGCCTCAGACAGTGG
TTCAAAGTTTTTTTCTTCCATTTCAGGTGTCGTGACGTACGGCCACCATGACCGAGTACAAGCCCACGGTGCGC
CTCGCCACCCGCGACGACGTCCCCAGGGCCGTACGCACCCTCGCCGCCGCGTTCGCCGACTACCCCGCCACGC
GCCACACCGTCGATCCGGACCGCCACATCGAGCGGGTCACCGAGCTGCAAGAACTCTTCCTCACGCGCGTCGG
 
GCTCGACATCGGCAAGGTGTGGGTCGCGGACGACGGCGCCGCCGTGGCGGTCTGGACCACGCCGGAGAGCG
TCGAAGCGGGGGCGGTGTTCGCCGAGATCGGCCCGCGCATGGCCGAGTTGAGCGGTTCCCGGCTGGCCGCGC
AGCAACAGATGGAAGGCCTCCTGGCGCCGCACCGGCCCAAGGAGCCCGCGTGGTTCCTGGCCACCGTCGGAG
TCTCGCCCGACCACCAGGGCAAGGGTCTGGGCAGCGCCGTCGTGCTCCCCGGAGTGGAGGCGGCCGAGCGC
GCCGGGGTGCCCGCCTTCCTGGAGACCTCCGCGCCCCGCAACCTCCCCTTCTACGAGCGGCTCGGCTTCACCGT
CACCGCCGACGTCGAGGTGCCCGAAGGACCGCGCACCTGGTGCATGACCCGCAAGCCCGGTGCCTGAACGCG
TTAAGTCGACAATCAACCTCTGGATTACAAAATTTGTGAAAGATTGACTGGTATTCTTAACTATGTTGCTCCTTT
TACGCTATGTGGATACGCTGCTTTAATGCCTTTGTATCATGCTATTGCTTCCCGTATGGCTTTCATTTTCTCCTCC
TTGTATAAATCCTGGTTGCTGTCTCTTTATGAGGAGTTGTGGCCCGTTGTCAGGCAACGTGGCGTGGTGTGCAC
TGTGTTTGCTGACGCAACCCCCACTGGTTGGGGCATTGCCACCACCTGTCAGCTCCTTTCCGGGACTTTCGCTTT
CCCCCTCCCTATTGCCACGGCGGAACTCATCGCCGCCTGCCTTGCCCGCTGCTGGACAGGGGCTCGGCTGTTGG
GCACTGACAATTCCGTGGTGTTGTCGGGGAAATCATCGTCCTTTCCTTGGCTGCTCGCCTGTGTTGCCACCTGG
ATTCTGCGCGGGACGTCCTTCTGCTACGTCCCTTCGGCCCTCAATCCAGCGGACCTTCCTTCCCGCGGCCTGCTG
CCGGCTCTGCGGCCTCTTCCGCGTCTTCGCCTTCGCCCTCAGACGAGTCGGATCTCCCTTTGGGCCGCCTCCCCG
CGTCGACTTTAAGACCAATGACTTACAAGGCAGCTGTAGATCTTAGCCACTTTTTAAAAGAAAAGGGGGGACT
GGAAGGGCTAATTCACTCCCAACGAAGACAAGATCTGCTTTTTGCTTGTACTGGGTCTCTCTGGTTAGACCAGA
TCTGAGCCTGGGAGCTCTCTGGCTAACTAGGGAACCCACTGCTTAAGCCTCAATAAAGCTTGCCTTGAGTGCTT
CAAGTAGTGTGTGCCCGTCTGTTGTGTGACTCTGGTAACTAGAGATCCCTCAGACCCTTTTAGTCAGTGTGGAA
AATCTCTAGCAGTACGTATAGTAGTTCATGTCATCTTATTATTCAGTATTTATAACTTGCAAAGAAATGAATATC
AGAGAGTGAGAGGAACTTGTTTATTGCAGCTTATAATGGTTACAAATAAAGCAATAGCATCACAAATTTCACA
AATAAAGCATTTTTTTCACTGCATTCTAGTTGTGGTTTGTCCAAACTCATCAATGTATCTTATCATGTCTGGCTCT
AGCTATCCCGCCCCTAACTCCGCCCATCCCGCCCCTAACTCCGCCCAGTTCCGCCCATTCTCCGCCCCATGGCTG
ACTAATTTTTTTTATTTATGCAGAGGCCGAGGCCGCCTCGGCCTCTGAGCTATTCCAGAAGTAGTGAGGAGGCT
TTTTTGGAGGCCTAGGGACGTACCCAATTCGCCCTATAGTGAGTCGTATTACGCGCGCTCACTGGCCGTCGTTT
TACAACGTCGTGACTGGGAAAACCCTGGCGTTACCCAACTTAATCGCCTTGCAGCACATCCCCCTTTCGCCAGC
TGGCGTAATAGCGAAGAGGCCCGCACCGATCGCCCTTCCCAACAGTTGCGCAGCCTGAATGGCGAATGGGAC
GCGCCCTGTAGCGGCGCATTAAGCGCGGCGGGTGTGGTGGTTACGCGCAGCGTGACCGCTACACTTGCCAGC
GCCCTAGCGCCCGCTCCTTTCGCTTTCTTCCCTTCCTTTCTCGCCACGTTCGCCGGCTTTCCCCGTCAAGCTCTAA
ATCGGGGGCTCCCTTTAGGGTTCCGATTTAGTGCTTTACGGCACCTCGACCCCAAAAAACTTGATTAGGGTGAT
GGTTCACGTAGTGGGCCATCGCCCTGATAGACGGTTTTTCGCCCTTTGACGTTGGAGTCCACGTTCTTTAATAG
TGGACTCTTGTTCCAAACTGGAACAACACTCAACCCTATCTCGGTCTATTCTTTTGATTTATAAGGGATTTTGCC
GATTTCGGCCTATTGGTTAAAAAATGAGCTGATTTAACAAAAATTTAACGCGAATTTTAACAAAATATTAACGC
TTACAATTTAGGTGGCACTTTTCGGGGAAATGTGCGCGGAACCCCTATTTGTTTATTTTTCTAAATACATTCAAA
TATGTATCCGCTCATGAGACAATAACCCTGATAAATGCTTCAATAATATTGAAAAAGGAAGAGTATGAGTATTC
AACATTTCCGTGTCGCCCTTATTCCCTTTTTTGCGGCATTTTGCCTTCCTGTTTTTGCTCACCCAGAAACGCTGGT
GAAAGTAAAAGATGCTGAAGATCAGTTGGGTGCACGAGTGGGTTACATCGAACTGGATCTCAACAGCGGTAA
 
GATCCTTGAGAGTTTTCGCCCCGAAGAACGTTTTCCAATGATGAGCACTTTTAAAGTTCTGCTATGTGGCGCGG
TATTATCCCGTATTGACGCCGGGCAAGAGCAACTCGGTCGCCGCATACACTATTCTCAGAATGACTTGGTTGAG
TACTCACCAGTCACAGAAAAGCATCTTACGGATGGCATGACAGTAAGAGAATTATGCAGTGCTGCCATAACCA
TGAGTGATAACACTGCGGCCAACTTACTTCTGACAACGATCGGAGGACCGAAGGAGCTAACCGCTTTTTTGCA
CAACATGGGGGATCATGTAACTCGCCTTGATCGTTGGGAACCGGAGCTGAATGAAGCCATACCAAACGACGA
GCGTGACACCACGATGCCTGTAGCAATGGCAACAACGTTGCGCAAACTATTAACTGGCGAACTACTTACTCTA
GCTTCCCGGCAACAATTAATAGACTGGATGGAGGCGGATAAAGTTGCAGGACCACTTCTGCGCTCGGCCCTTC
CGGCTGGCTGGTTTATTGCTGATAAATCTGGAGCCGGTGAGCGTGGGTCTCGCGGTATCATTGCAGCACTGGG
GCCAGATGGTAAGCCCTCCCGTATCGTAGTTATCTACACGACGGGGAGTCAGGCAACTATGGATGAACGAAAT
AGACAGATCGCTGAGATAGGTGCCTCACTGATTAAGCATTGGTAACTGTCAGACCAAGTTTACTCATATATACT
TTAGATTGATTTAAAACTTCATTTTTAATTTAAAAGGATCTAGGTGAAGATCCTTTTTGATAATCTCATGACCAA
AATCCCTTAACGTGAGTTTTCGTTCCACTGAGCGTCAGACCCCGTAGAAAAGATCAAAGGATCTTCTTGAGATC
CTTTTTTTCTGCGCGTAATCTGCTGCTTGCAAACAAAAAAACCACCGCTACCAGCGGTGGTTTGTTTGCCGGATC
AAGAGCTACCAACTCTTTTTCCGAAGGTAACTGGCTTCAGCAGAGCGCAGATACCAAATACTGTTCTTCTAGTG
TAGCCGTAGTTAGGCCACCACTTCAAGAACTCTGTAGCACCGCCTACATACCTCGCTCTGCTAATCCTGTTACCA
GTGGCTGCTGCCAGTGGCGATAAGTCGTGTCTTACCGGGTTGGACTCAAGACGATAGTTACCGGATAAGGCG
CAGCGGTCGGGCTGAACGGGGGGTTCGTGCACACAGCCCAGCTTGGAGCGAACGACCTACACCGAACTGAGA
TACCTACAGCGTGAGCTATGAGAAAGCGCCACGCTTCCCGAAGGGAGAAAGGCGGACAGGTATCCGGTAAGC
GGCAGGGTCGGAACAGGAGAGCGCACGAGGGAGCTTCCAGGGGGAAACGCCTGGTATCTTTATAGTCCTGTC
GGGTTTCGCCACCTCTGACTTGAGCGTCGATTTTTGTGATGCTCGTCAGGGGGGCGGAGCCTATGGAAAAACG
CCAGCAACGCGGCCTTTTTACGGTTCCTGGCCTTTTGCTGGCCTTTTGCTCACATGTTCTTTCCTGCGTTATCCCC
TGATTCTGTGGATAACCGTATTACCGCCTTTGAG
**SEQ ID NO: 4, Target plasmid** 
acaccatcgaatggtgcaaaacctttcgcggtatggcatgatagcgcccggaagagagtcaattcagggtggtgaatgtgaaaccagtaacgtt
atacgatgtcgcagagtatgccggtgtctcttatcagaccgtttcccgcgtggtgaaccaggccagccacgtttctgcgaaaacgcgggaaaaag
tggaagcggcgatggcggagctgaattacattcccaaccgcgtggcacaacaactggcgggcaaacagtcgttgctgattggcgttgccacctcc
agtctggccctgcacgcgccgtcgcaaattgtcgcggcgattaaatctcgcgccgatcaactgggtgccagcgtggtggtgtcgatggtagaacg
aagcggcgtcgaagcctgtaaagcggcggtgcacaatcttctcgcgcaacgcgtcagtgggctgatcattaactatccgctggatgaccaggatg
ccattgctgtggaagctgcctgcactaatgttccggcgttatttcttgatgtctctgaccagacacccatcaacagtattattttctcccatgaagac
ggtacgcgactgggcgtggagcatctggtcgcattgggtcaccagcaaatcgcgctgttagcgggcccattaagttctgtctcggcgcgtctgcgt
ctggctggctggcataaatatctcactcgcaatcaaattcagccgatagcggaacgggaaggcgactggagtgccatgtccggttttcaacaaac
catgcaaatgctgaatgagggcatcgttcccactgcgatgctggttgccaacgatcagatggcgctgggcgcaatgcgcgccattaccgagtccg
ggctgcgcgttggtgcggatatctcggtagtgggatacgacgataccgaagacagctcatgttatatcccgccgttaaccaccatcaaacaggat
tttcgcctgctggggcaaaccagcgtggaccgcttgctgcaactctctcagggccaggcggtgaagggcaatcagctgttgcccgtctcactggtg
aaaagaaaaaccaccctggcgcccaatacgcaaaccgcctctccccgcgcgttggccgattcattaatgcagctggcacgacaggtttcccgact
 
ggaaagcgggcagtgagcgcaacgcaattaatgtaagttagctcaaatattctgaaatgagctgttgacaattaatcatcggctcgtataatgtgt
ggaattgtgagcggataacaatttcacacaggaaacagaattcTTTGCATCTCCCCATTCCATGAGcATATGCgatcttccccatcgg
tgatgtcggcgatataggcgccagcaaccgcacctgtggcgccggtgatgccggccacgatgcgtccggcgtagaggatctgctcatgtttgaca
gcttatcatcgatgcataatgtgcctgtcaaatggacgaagcagggattctgcaaaccctatgctactccgtcaagccgtcaattgtctgattcgtt
accaattatgacaacttgacggctacatcattcactttttcttcacaaccggcacggaactcgctcgggctggccccggtgcattttttaaatacccg
cgagaaatagagttgatcgtcaaaaccaacattgcgaccgacggtggcgataggcatccgggtggtgctcaaaagcagcttcgcctggctgata
cgttggtcctcgcgccagcttaagacgctaatccctaactgctggcggaaaagatgtgacagacgcgacggcgacaagcaaacatgctgtgcga
cgctggcgatatcaaaattgctgtctgccaggtgatcgctgatgtactgacaagcctcgcgtacccgattatccatcggtggatggagcgactcgtt
aatcgcttccatgcgccgcagtaacaattgctcaagcagatttatcgccagcagctccgaatagcgcccttccccttgcccggcgttaatgatttgc
ccaaacaggtcgctgaaatgcggctggtgcgcttcatccgggcgaaagaaccccgtattggcaaatattgacggccagttaagccattcatgcca
gtaggcgcgcggacgaaagtaaacccactggtgataccattcgcgagcctccggatgacgaccgtagtgatgaatctctcctggcgggaacagc
aaaatatcacccggtcggcaaacaaattctcgtccctgatttttcaccaccccctgaccgcgaatggtgagattgagaatataacctttcattccca
gcggtcggtcgataaaaaaatcgagataaccgttggcctcaatcggcgttaaacccgccaccagatgggcattaaacgagtatcccggcagcag
gggatcattttgcgcttcagccatacttttcatactcccgccattcagagaagaaaccaattgtccatattgcatcagacattgccgtcactgcgtct
tttactggctcttctcgctaaccaaaccggtaaccccgcttattaaaagcattctgtaacaaagcgggaccaaagccatgacaaaaacgcgtaac
aaaagtgtctataatcacggcagaaaagtccacattgattatttgcacggcgtcacactttgctatgccatagcatttttatccataagattagcgg
atcctacctgacgctttttatcgcaactctctactgtttctccatacccgtttttttgggctagcgaattcgagctcatgcagtttaaggtttacaccta
taaaagagagagccgttatcgtctgtttgtggatgtacagagtgatattattgacacgcccgggcgacggatggtgatccccctggccagtgcac
gtctgctgtcagataaagtctcccgtgaactttacccggtggtgcatatcggggatgaaagctggcgcatgatgaccaccgatatggccagtgtgc
cggtatccgttatcggggaagaagtggctgatctcagccaccgcgaaaatgacatcaaaaacgccattaacctgatgttctggggaatataatct
agagtcgacctgcaggcatgcaagcttggctgttttggcggatgagagaagattttcagcctgatacagattaaatcagaacgcagaagcggtct
gataaaacagaatttgcctggcggcagtagcgcggtggtcccacctgaccccatgccgaactcagaagtgaaacgccgtagcgccgatggtagt
gtggggtctccccatgcgagagtagggaactgccaggcatcaaataaaacgaaaggctcagtcgaaagactgggcctttcgttttatctgttgttt
gtcggtgaacgctctcctgagtaggacaaatccgccgggagcggatttgaacgttgcgaagcaacggcccggagggtggcgggcaggacgccc
gccataaactgccaggcatcaaattaagcagaaggccatcctgacggatggcctttttgcgtttctacaaactctggatcctccggcgttcagcct
gtgccacagccgacaggatggtgaccaccatttgccccatatcaccgtcggtactgatcccgtcgtcaataaaccgaaccgctacaccctgagca
tcaaactcttttatcagttggatcatgtcggcggtgtcgcggccaagacggtcgagcttcttcaccagaatgacatcaccttcctccaccttcatcct
cagcaaatccagcccttcccgatctgttgaactgccggatgccttgtcggtaaagatgcggttagcttttacccctgcatctttgagcgctgaggtct
gcctcgtgaagaaggtgttgctgactcataccaggcctgaatcgccccatcatccagccagaaagtgagggagccacggttgatgagagctttgt
tgtaggtggaccagttggtgattttgaacttttgctttgccacggaacggtctgcgttgtcgggaagatgcgtgatctgatccttcaactcagcaaa
agttcgatttattcaacaaagccgccgtcccgtcaagtcagcgtaatgctctgccagtgttacaaccaattaaccaattctgattagaaaaactcat
cgagcatcaaatgaaactgcaatttattcatatcaggattatcaataccatatttttgaaaaagccgtttctgtaatgaaggagaaaactcaccga
ggcagttccataggatggcaagatcctggtatcggtctgcgattccgactcgtccaacatcaatacaacctattaatttcccctcgtcaaaaataa
ggttatcaagtgagaaatcaccatgagtgacgactgaatccggtgagaatggcaaaagcttatgcatttctttccagacttgttcaacaggccagc
cattacgctcgtcatcaaaatcactcgcatcaaccaaaccgttattcattcgtgattgcgcctgagcgagacgaaatacgcgatcgctgttaaaag
gacaattacaaacaggaatcgaatgcaaccggcgcaggaacactgccagcgcatcaacaatattttcacctgaatcaggatattcttctaatacc
 
tggaatgctgttttcccggggatcgcagtggtgagtaaccatgcatcatcaggagtacggataaaatgcttgatggtcggaagaggcataaattc
cgtcagccagtttagtctgaccatctcatctgtaacatcattggcaacgctacctttgccatgtttcagaaacaactctggcgcatcgggcttcccat
acaatcgatagattgtcgcacctgattgcccgacattatcgcgagcccatttatacccatataaatcagcatccatgttggaatttaatcgcggcct
cgagcaagacgtttcccgttgaatatggctcataacaccccttgtattactgtttatgtaagcagacagttttattgttcatgatgatatatttttatct
tgtgcaatgtaacatcagagattttgagacacaacgtggctttgttgaataaatcgaacttttgctgagttgaaggatcagatcacgcatcttcccg
acaacgcagaccgttccgtggcaaagcaaaagttcaaaatcaccaactggtccacctacaacaaagctctcatcaaccgtggctccctcactttc
tggctggatgatggggcgattcaggcctggtatgagtcagcaacaccttcttcacgaggcagacctcagcgctagcggagtgtatactggcttact
atgttggcactgatgagggtgtcagtgaagtgcttcatgtggcaggagaaaaaaggctgcaccggtgcgtcagcagaatatgtgatacaggata
tattccgcttcctcgctcactgactcgctacgctcggtcgttcgactgcggcgagcggaaatggcttacgaacggggcggagatttcctggaagat
gccaggaagatacttaacagggaagtgagagggccgcggcaaagccgtttttccataggctccgcccccctgacaagcatcacgaaatctgacg
ctcaaatcagtggtggcgaaacccgacaggactataaagataccaggcgtttccccctggcggctccctcgtgcgctctcctgttcctgcctttcgg
tttaccggtgtcattccgctgttatggccgcgtttgtctcattccacgcctgacactcagttccgggtaggcagttcgctccaagctggactgtatgc
acgaaccccccgttcagtccgaccgctgcgccttatccggtaactatcgtcttgagtccaacccggaaagacatgcaaaagcaccactggcagca
gccactggtaattgatttagaggagttagtcttgaagtcatgcgccggttaaggctaaactgaaaggacaagttttggtgactgcgctcctccaag
ccagttacctcggttcaaagagttggtagctcagagaaccttcgaaaaaccgccctgcaaggcggttttttcgttttcagagcaagagattacgcg
cagaccaaaacgatctcaagaagatcatcttattaaggggtctgacgctcagtggaacgaaaactcacgttaagggattttggtcatgagattat
caaaaaggatcttcacctagatccttttggtcg
**SEQ ID NO: 5, Direct Repeat sequence of CasY6**
   TATCCATCGTGCCGCCTCTTGGCAC
**SEQ ID NO: 6, Target-TTR-spacer**
   gcatctccccattccatgag
**SEQ ID NO: 7, Sequence of CasY6-sgRNA expression fragment** 
CAATTGTAATACGACTCACTATAGGCACCGTATCCATCGTGCCGCCTCTTGGCACGCATCTCCCCATTCCATGAG
TTTTTTAAGCTTGGCGTagaaggccatcctgacggatggccttttACGCGT
**SEQ ID NO: 8, CasY6-TTR-sgRNA**
   TATCCATCGTGCCGCCTCTTGGCACGCATCTCCCCATTCCATGAG
**SEQ ID NO: 9, TTR-F**
   aactgaggaggaatttgtag
**SEQ ID NO: 10, TTR-R**
   caaaagcaaaaaccaaaacc
**SEQ ID NO: 11, araC-pBAD-CCDB** 
TTTGCATCTCCCCATTCCATGAGcATATGCgatcttccccatcggtgatgtcggcgatataggcgccagcaaccgcacctgtggcgcc
ggtgatgccggccacgatgcgtccggcgtagaggatctgctcatgtttgacagcttatcatcgatgcataatgtgcctgtcaaatggacgaagcag
ggattctgcaaaccctatgctactccgtcaagccgtcaattgtctgattcgttaccaattatgacaacttgacggctacatcattcactttttcttcac
aaccggcacggaactcgctcgggctggccccggtgcattttttaaatacccgcgagaaatagagttgatcgtcaaaaccaacattgcgaccgacg
gtggcgataggcatccgggtggtgctcaaaagcagcttcgcctggctgatacgttggtcctcgcgccagcttaagacgctaatccctaactgctgg
cggaaaagatgtgacagacgcgacggcgacaagcaaacatgctgtgcgacgctggcgatatcaaaattgctgtctgccaggtgatcgctgatgt
actgacaagcctcgcgtacccgattatccatcggtggatggagcgactcgttaatcgcttccatgcgccgcagtaacaattgctcaagcagattta
tcgccagcagctccgaatagcgcccttccccttgcccggcgttaatgatttgcccaaacaggtcgctgaaatgcggctggtgcgcttcatccgggc
gaaagaaccccgtattggcaaatattgacggccagttaagccattcatgccagtaggcgcgcggacgaaagtaaacccactggtgataccattc
gcgagcctccggatgacgaccgtagtgatgaatctctcctggcgggaacagcaaaatatcacccggtcggcaaacaaattctcgtccctgattttt
caccaccccctgaccgcgaatggtgagattgagaatataacctttcattcccagcggtcggtcgataaaaaaatcgagataaccgttggcctcaa
tcggcgttaaacccgccaccagatgggcattaaacgagtatcccggcagcaggggatcattttgcgcttcagccatacttttcatactcccgccatt
cagagaagaaaccaattgtccatattgcatcagacattgccgtcactgcgtcttttactggctcttctcgctaaccaaaccggtaaccccgcttatt
aaaagcattctgtaacaaagcgggaccaaagccatgacaaaaacgcgtaacaaaagtgtctataatcacggcagaaaagtccacattgattatt
tgcacggcgtcacactttgctatgccatagcatttttatccataagattagcggatcctacctgacgctttttatcgcaactctctactgtttctccata
cccgtttttttgggctagcgaattcgagctcatgcagtttaaggtttacacctataaaagagagagccgttatcgtctgtttgtggatgtacagagtg
atattattgacacgcccgggcgacggatggtgatccccctggccagtgcacgtctgctgtcagataaagtctcccgtgaactttacccggtggtgc
atatcggggatgaaagctggcgcatgatgaccaccgatatggccagtgtgccggtatccgttatcggggaagaagtggctgatctcagccaccgc
gaaaatgacatcaaaaacgccattaacctgatgttctggggaatataatctagagtcgacctgcaggcatgcaagcttggctgttttggcggatg
agagaagattttcagcctgatacagattaaatcagaacgcagaagcggtctgataaaacagaatttgcctggcggcagtagcgcggtggtccca
cctgaccccatgccgaactcagaagtgaaacgccgtagcgccgatggtagtgtggggtctccccatgcgagagtagggaactgccaggcatcaa
ataaaacgaaaggctcagtcgaaagactgggcctttcgttttatctgttgtttgtcggtgaacgctctcctgagtaggacaaatccgccgggagcg
gatttgaacgttgcgaagcaacggcccggagggtggcgggcaggacgcccgccataaactgccaggcatcaaattaagcagaaggccatcctg
acggatggcctttttgcgtttctacaaactct
SEQ ID NO: 12, Amino acid sequence of LbCpf1 
MSKLEKFTNCYSLSKTLRFKAIPVGKTQENIDNKRLLVEDEKRAEDYKGVKKLLDRYYLSFINDVLHSIKLKNLNNYISLF
RKKTRTEKENKELENLEINLRKEIAKAFKGNEGYKSLFKKDIIETILPEFLDDKDEIALVNSFNGFTTAFTGFFDNRENMF
SEEAKSTSIAFRCINENLTRYISNMDIFEKVDAIFDKHEVQEIKEKILNSDYDVEDFFEGEFFNFVLTQEGIDVYNAIIGG
FVTESGEKIKGLNEYINLYNQKTKQKLPKFKPLYKQVLSDRESLSFYGEGYTSDEEVLEVFRNTLNKNSEIFSSIKKLEKLF
KNFDEYSSAGIFVKNGPAISTISKDIFGEWNVIRDKWNAEYDDIHLKKKAVVTEKYEDDRRKSFKKIGSFSLEQLQEYA
DADLSVVEKLKEIIIQKVDEIYKVYGSSEKLFDADFVLEKSLKKNDAVVAIMKDLLDSVKSFENYIKAFFGEGKETNRDE
SFYGDFVLAYDILLKVDHIYDAIRNYVTQKPYSKDKFKLYFQNPQFMGGWDKDKETDYRATILRYGSKYYLAIMDKKY
AKCLQKIDKDDVNGNYEKINYKLLPGPNKMLPKVFFSKKWMAYYNPSEDIQKIYKNGTFKKGDMFNLNDCHKLIDF
 
FKDSISRYPKWSNAYDFNFSETEKYKDIAGFYREVEEQGYKVSFESASKKEVDKLVEEGKLYMFQIYNKDFSDKSHGT
PNLHTMYFKLLFDENNHGQIRLSGGAELFMRRASLKKEELVVHPANSPIANKNPDNPKKTTTLSYDVYKDKRFSEDQ
YELHIPIAINKCPKNIFKINTEVRVLLKHDDNPYVIGIDRGERNLLYIVVVDGKGNIVEQYSLNEIINNFNGIRIKTDYHSL
LDKKEKERFEARQNWTSIENIKELKAGYISQVVHKICELVEKYDAVIALEDLNSGFKNSRVKVEKQVYQKFEKMLIDKL
NYMVDKKSNPCATGGALKGYQITNKFESFKSMSTQNGFIFYIPAWLTSKIDPSTGFVNLLKTKYTSIADSKKFISSFDRI
MYVPEEDLFEFALDYKNFSRTDADYIKKWKLYSYGNRIRIFRNPKKNNVFDWEEVCLTSAYKELFNKYGINYQQGDI
RALLCEQSDKAFYSSFMALMSLMLQMRNSITGRTDVDFLISPVKNSDGIFYDSRNYEAQENAILPKNADANGAYNI
ARKVLWAIGQFKKAEDEKLDKVKIAISNKEWLEYAQTSVKHGS
**SEQ ID NO: 13, Nucleotide sequence of LbCpf1** 
atgtcaaagctggagaaattcaccaactgttatagcctgtctaagaccctgcgcttcaaggcaatcccagtgggcaagacacaagagaacattg
acaacaaacggctcctggtggaggatgagaagagggctgaagattacaagggcgttaagaagctgctggataggtactatctgtcattcatcaa
cgatgtcctccacagtatcaagctgaagaatctgaacaattacatttctctgttccggaagaagacacggaccgagaaggagaacaaagagctg
gagaatctggagatcaacctgaggaaagaaatagctaaggctttcaaagggaacgagggttacaagtccctgttcaagaaagacattatcgag
actattctgcctgagttcctggacgataaagatgagatcgccctcgtcaattccttcaatgggtttaccacagcctttaccggcttcttcgacaatag
agagaatatgttctctgaagaggccaaatccactagcatcgcctttcgctgcataaacgagaacctgactaggtacatcagcaatatggacatctt
tgagaaagtcgatgccatattcgacaaacatgaggtgcaggagattaaggagaagatcctgaactcagattacgatgtcgaagatttcttcgag
ggagagttcttcaacttcgtgctcacacaagagggcattgatgtgtacaatgcaatcattggagggttcgtgacagagagtggcgagaagataa
agggcctgaacgagtatatcaacctctacaaccagaaaaccaagcagaaactgcctaagttcaagccactgtacaaacaagtgctctcagatag
ggaaagcctgagcttctacggtgaagggtatacatcagatgaagaagtgctcgaagtgttccgcaacaccctcaataagaacagtgaaatcttct
cttcaatcaagaagctggagaaactgttcaagaatttcgatgagtactcctctgccggaatctttgtgaagaatggccctgcaatatccactattag
caaagacatctttggcgagtggaacgttatcagggataagtggaatgccgagtacgatgatattcatctcaagaagaaagccgtggttacagag
aaatacgaggatgatagacgcaagagctttaagaagattggtagcttctctctcgaacagctgcaggagtacgccgacgctgacctgtcagtcgt
ggagaaactcaaggagatcataatccagaaggtggatgaaatctacaaagtgtatggaagctctgagaaactcttcgatgcagactttgttctgg
agaagagtctgaagaagaacgacgcagtggttgctatcatgaaggacctgctggattctgttaagtctttcgagaattacattaaggcattctttg
gtgaagggaaggagacaaatagggacgagagcttctatggcgactttgttctggcctacgacatcctcctcaaggttgaccacatctatgacgct
atacggaattacgttacccagaagccctatagcaaagacaagttcaagctgtatttccagaatccacagtttatgggtgggtgggataaagacaa
agaaacagattacagggccactatcctgcggtacggcagcaaatactatctggctatcatggataagaagtacgccaaatgcctccagaagatc
gacaaggacgacgtgaacggtaactacgagaagatcaattacaagctcctgccaggacctaacaagatgctgcccaaggtgttcttctccaaga
aatggatggcctactataacccaagcgaggacattcagaagatatacaagaatgggacattcaagaagggcgatatgttcaacctcaacgactg
ccacaagctgattgatttcttcaaggatagcatttctcgctatcccaagtggtctaatgcatacgatttcaacttcagcgagactgagaagtacaaa
gacatcgctggcttctaccgggaggtggaagagcaaggctataaggtgtcattcgaatccgcttctaagaaggaagtggataagctcgtggaag
agggtaagctgtacatgttccagatatacaacaaagacttcagcgataagagccacggcactccaaacctccatactatgtatttcaagctgctgt
ttgacgagaacaaccacggacagattaggctgtcaggaggcgcagaactcttcatgcgcagagcttcactgaagaaggaggaactcgttgtcca
cccagccaatagccctatagccaataagaatccagacaatcctaagaaaaccactactctgtcttacgatgtgtataaggataagagattctctg
aagatcagtacgaactgcacatacccattgccattaacaagtgccctaagaacatcttcaagattaacacagaggttagagtgctcctgaaacac
 
gacgataacccttatgttataggcattgatcgcggagagagaaacctgctgtacatcgtcgtggtggacggcaaaggcaacatcgtggaacagt
acagtctcaatgaaatcattaacaatttcaacggaatccgcattaagaccgactaccattctctcctcgacaagaaggagaaagaaaggttcga
agcaagacagaattggacaagtatagagaatatcaaagaactgaaggctgggtacatctctcaggttgtgcacaagatatgtgagctggtggag
aagtacgacgctgttatcgccctcgaggacctgaatagcggcttcaagaactccagggtgaaggtggagaagcaggtgtatcagaagttcgaga
agatgctgatcgacaagctcaactatatggtggacaagaaatccaatccttgcgctactggtggagccctgaagggctatcaaatcaccaataag
ttcgaatctttcaagtctatgagcacccagaatggcttcatcttctacatacccgcatggctgacatccaagattgatccctctaccggatttgttaa
tctgctcaagactaagtacacctctattgctgactcaaagaagttcatatcatcatttgaccgcatcatgtacgtgccagaagaggacctgttcgag
tttgccctggattacaagaatttctctcggactgacgccgactacatcaagaagtggaagctctactcttatggtaatcggattcgcatattccgca
atcccaagaagaataacgtgttcgattgggaggaagtttgcctcaccagcgcttacaaggagctgttcaataagtatgggattaactaccagcag
ggcgacataagagccctgctgtgcgaacaatctgataaggcattctattcctctttcatggcactgatgtcactgatgctgcaaatgcgcaattcca
tcaccggaagaacagacgtggactttctgatctctcctgtcaagaactcagatggcatcttctacgattcccgcaactatgaagcacaggagaat
gctatcctgcctaagaatgccgatgcaaatggagcctataacatcgccagaaaggtcctctgggccataggacaattcaagaaagctgaagatg
agaagctggacaaggtgaagatcgccatttcaaacaaagagtggctcgaatatgctcagacctcagtgaagcatggatca
**SEQ ID NO: 14, Sequence of LbCpf1-sgRNA expression fragment** 
CAATTGTAATACGACTCACTATAGGCACCGTAATTTCTACTAAGTGTAGATGCATCTCCCCATTCCATGAGTTTT
TTAAGCTTGGCGTagaaggccatcctgacggatggcctttt ACGCGT
**SEQ ID NO: 15, LbCpf1-TTR-sgRNA**
   TAATTTCTACTAAGTGTAGATGCATCTCCCCATTCCATGAG
**SEQ ID NO: 16, CasY6-TTR-sgRNA-Oligo-F**
   CACCTATCCATCGTGCCGCCTCTTGGCACGCATCTCCCCATTCCATGAG
**SEQ ID NO: 17, CasY6-TTR-sgRNA-Oligo-R**
   AAAACTCATGGAATGGGGAGATGCGTGCCAAGAGGCGGCACGATGGATA
**SEQ ID NO: 18, LbCpf1-TTR-sgRNA-Oligo-F**
   CACCTAATTTCTACTAAGTGTAGATGCATCTCCCCATTCCATGAG
**SEQ ID NO: 19, LbCpf1-TTR-sgRNA-Oligo-R**
   AAAACTCATGGAATGGGGAGATGCATCTACACTTAGTAGAAATTA
**SEQ ID NO: 20, CasY6-TTR-sgRNA'**
   TATCCATCGTGCCGCCTCTTGGCACtatatcccttctacaaattc
**SEQ ID NO: 21, CasY6-TTR-sgRNA"**
   TATCCATCGTGCCGCCTCTTGGCACgtgtctatttccactttgta
**SEQ ID NO: 22, CasY6-TTR-sgRNA'-Oligo-F**
   CACCTATCCATCGTGCCGCCTCTTGGCACtatatcccttctacaaattc
**SEQ ID NO: 23, CasY6-TTR-sgRNA'-Oligo-R**
   AAAAGAATTTGTAGAAGGGATATAGTGCCAAGAGGCGGCACGATGGATA
**SEQ ID NO: 24, CasY6-TTR-sgRNA"-Oligo-F**
   CACCTATCCATCGTGCCGCCTCTTGGCACgtgtctatttccactttgta
**SEQ ID NO: 25, CasY6-TTR-sgRNA"-Oligo-R**
   AAAATACAAAGTGGAAATAGACACGTGCCAAGAGGCGGCACGATGGATA
**SEQ ID NO: 26, TTR-F'**
   Gggtgtattactttgccatg
**SEQ ID NO: 27, TTR-R'**
   Aacctttggtcattcatcaccttc
**SEQ ID NO: 28, Amino acid sequence of 005V1** 
MSELNDAYWMKQALALAQKAREQGEVPVGAILVLDDEVIGQGWNRAITLHDPTAHAEIMALQQGGQIVQNYRL
LNATLYVTFEPCVMCAGAMVHSRIKRLVYGVSNSKRGAAGSLLNVLNYPGMNHQIEITAGVMANECSEMLCQFY
QQPREVFNAEREARRLNQPDRAD
**SEQ ID NO: 29, Amino acid sequence of 005V1-10-3** 
MSELNDAYWMKQALALAQKAREQGEVPVGAILVLDDEVIGQGWNRAITLHDPTAHAEIMALQQGGQIVQNYRL
LNATLYVTFEPCVMCAGAMVHSRIKRLVYGVSNSKRGAAGSLLNVLNYPGMNHQIEITAGVMANECSEMLCQFY
GMRREVFNAEREARRLNQPDRAD
**SEQ ID NO: 30, Nucleotide sequence of 005V1-10-3** 
ATGAGTGAGCTGAATGATGCTTACTGGATGAAACAGGCACTCGCTTTAGCTCAGAAGGCCcggGAACAGGGAG
AAGTTCCAGTGGGCGCTATTCTGGTGctgGATGATGAAGTGATAGGACAGGGATGGAATAGAgccATCACCctg
CACGACCCCACCGCCCACGCCGAGATCATGGCCCTGCAGCAGGGCGGCCAGATCGTGCAGAACTACCGGCTG
 
CTGAACGCCACCCTGTACGTGACCttcGAGCCCTGCGTGATGTGCGCCGGCGCCATGGTGCACAGCAGGATCAA
GAGACTGGTCTACGGCgtgAGCaactcaAAAagaGGCGCCGCCGGCAGCCTGCTGaacGTGCTGaactacCCCGGC
ATGAACCACCAGATCGAGATCACCGCCGGCGTGATGGCCAACGAGTGCAGCGAGATGCTGtgcCAGTTCtatGG
TATGCGCAGAGAAgtgttcaatGCTGAGCGTGAGGCTAGGCGGCTGAACCAACCTGATAGAGCTGAC
**SEQ ID NO: 31, Nucleotide fragment of 005V1-10-3 with homologous-arm sequence and linker** 
ccaaagaagaagcggaaagtcATGAGTGAGCTGAATGATGCTTACTGGATGAAACAGGCACTCGCTTTAGCTCAG
AAGGCCcggGAACAGGGAGAAGTTCCAGTGGGCGCTATTCTGGTGctgGATGATGAAGTGATAGGACAGGG
ATGGAATAGAgccATCACCctgCACGACCCCACCGCCCACGCCGAGATCATGGCCCTGCAGCAGGGCGGCCAG
ATCGTGCAGAACTACCGGCTGCTGAACGCCACCCTGTACGTGACCttcGAGCCCTGCGTGATGTGCGCCGGCG
CCATGGTGCACAGCAGGATCAAGAGACTGGTCTACGGCgtgAGCaactcaAAAagaGGCGCCGCCGGCAGCCT
GCTGaacGTGCTGaactacCCCGGCATGAACCACCAGATCGAGATCACCGCCGGCGTGATGGCCAACGAGTGC
AGCGAGATGCTGtgcCAGTTCtatGGTATGCGCAGAGAAgtgttcaatGCTGAGCGTGAGGCTAGGCGGCTGAA
CCAACCTGATAGAGCTGACtccggaggatctagcggaggctcctctggctctgagacacctggcacaagcgagagcgcaacacctgaa
agcagcgggggcagcagcggggggtcaATCAAGAACCAAATCATCGG
**SEQ ID NO: 32, D1069A-dCasY6-F**
   ATCAAGAACCAAATCATCGG
**SEQ ID NO: 33, D1069A-dCasY6-R**
   Gactttccgcttcttctttgg
**SEQ ID NO: 34, Nucleotide sequence of 005V1-10-3-dCasY6 fusion protein** 
atgaaacggacagccgacggaagcgagttcgagtcaccaaagaagaagcggaaagtcATGAGTGAGCTGAATGATGCTTACTGG
ATGAAACAGGCACTCGCTTTAGCTCAGAAGGCCcggGAACAGGGAGAAGTTCCAGTGGGCGCTATTCTGGTGct
gGATGATGAAGTGATAGGACAGGGATGGAATAGAgccATCACCctgCACGACCCCACCGCCCACGCCGAGATCA
TGGCCCTGCAGCAGGGCGGCCAGATCGTGCAGAACTACCGGCTGCTGAACGCCACCCTGTACGTGACCttcGA
GCCCTGCGTGATGTGCGCCGGCGCCATGGTGCACAGCAGGATCAAGAGACTGGTCTACGGCgtgAGCaactcaA
AAagaGGCGCCGCCGGCAGCCTGCTGaacGTGCTGaactacCCCGGCATGAACCACCAGATCGAGATCACCGCCG
GCGTGATGGCCAACGAGTGCAGCGAGATGCTGtgcCAGTTCtatGGTATGCGCAGAGAAgtgttcaatGCTGAGCG
TGAGGCTAGGCGGCTGAACCAACCTGATAGAGCTGACtccggaggatctagcggaggctcctctggctctgagacacctggca
caagcgagagcgcaacacctgaaagcagcgggggcagcagcggggggtcaATCAAGAACCAAATCATCGGATATGAAGTGAA
GCTTGACCTCAACGACAGGAAGAGGGCTTTTGTTAGGGATACCTTGCGGTACTTCGCCCAGACATCGGATGTG
TTCCGAAAGGGCCTGATGTGCCTGTTCGCTGGCGTCGACCCCGAGATGATAAGCGGCTACCTGCCCGAGGGG
GAATCCGGCGACCTGTGGTGCGCCGTCAACTGGTTCCGCCTCGTGGATCTGTCGGAGGGTCTTGTCTCCGAGG
AGCAGGTGGTAGACGGGTCGGAGCTGTGCCGCAGGTTCCAAGACTACGCAGGTCATGCGCCCGACCGGCAG
 
GCGGAGATATACCTGACTAGGAGGAGCGAGGGCGATGTCCAGTACGTTTGGGCCGACTGCCGTGCTGGCTAC
CGCAGCTTCTGCCGTGAGGTGGGACTGAGCCCAGACCAGATGGCATTTGACGTGCTTGCGCTTGTGGCAAGG
GGCTACATCAAGTTCGACAAGGGCGAAAACGATGCCTGGGGGTTTGTATCCGCCGTCTTCGGCGACGGCGTC
AAGGAGGACACCAAGAACAAGATTCGCATGCTCGAGGGCATGAGGAGAGCAGCACACGCCAAGCCGGCCAC
CTACGAGGAGCTTCAGGAATCGGCGCTGAGGGAGACGGGATGCAGCGACATCAAGCAAATGATCATGGAGT
ACAAGTCCGGTCAGCAGGACGACAAGGAAGGCGTCAGAAAAAGCAGCGCCGGCAGTCACGCATTCTTCGTGA
AATACTTCTTGAGGGGAGCCGATCGGGTATGCGGCCCCATCGGCGAGAAGGAGCTGGAATTGTTTGTCAGTA
GCTGCGACAAGACCCTGAAGCAAAAAACATCGTGCGACGAGGCGCCGATCACGCACAGTGCCGTGTGGAAGA
GGTGGTTCAAGAGGAGGATACCGCACACCGTCGGTTCAATCCAGGAACCATACAGCGTTGCGTGCGACCAAG
CCATGGCCGACATAAGCTCTAAGAACTGCCTGAACGCCCGCATGGTGAGCTGCCCAGACAGCGGCTCGGTGG
CCGAGCGCAGGAAGAAGGGCACCAAGGCGAAGAAGGTCGGGTCATCCGGCGTGCTCGAGATGCAGGACAAG
GTTTCGTCCCTGTCGAGCAAGGGCGTTGATCTTGCGAAGTCCGTATTCGACAGCTTCAACGATGCTCTGGACAG
AGACCGCAAGGAGGACGAGGATTCCTGCCGGTTCGAGGTCCTCAGGCATCACCTCGGCAAGCTTTCCGACCTT
TACGAGATTTGGGTGAAGAACGACTGCGACTCCGGCATAGGTCTCTATCTGGAGGAATACGAAGGTTCGATTA
AAAAACAGCCGAAAGAGGAACTGCTGCGTGCGATCTACCCGCATCGAGAGTCAATAAAGGCCAAGGAGTTCA
TGGACGCAGCGCACCGCAACGACCTCATCCAAAAGATCGAGTCCCGCAAGGCGCACCCGACTGTGCGTGGCA
ACAGGGGCGTCGGTTACGGCAACTCCAAGTTGAGGGGCAAGATAATGCCGCAGTCCAACATGCACAAAGGTC
GCAAAGCCGGCCTGAACCCCGAGATGTGGCTCGAGGCGGATCTCCTGAACGGGGAGAGGTGGGAGAAGCAT
CACCTGAAGTTCCACTCGTCGCAGTTCTTCGATCAGGTCTACTGCCACAAGGACGGACTGCCCTACACGAAAAT
CCCTGGCCTGCCGAGGTTGGCACGGCACGGCATGCCCATCGGGCTGTCGATAAGCCCCGATGACGTTCCTCTG
GTGAACCGTGTGTCCGGCAACGGCAGGCGTTGCCCGAGGGCGAACAAGAGAAACCTTAGGGCCGTCGCCAAC
ATCACTCACAACGTGGAACTCCACCCGGAGACAGCCTTCGAGATACGCAGCAACGGCGAATACGCAAGCATCA
GCCACAGGGTGCAAGATGTCGCCAAGAAGAGCATCGAGGCCGGCGATCGAAGTCTTGGCTACGACCGAAATC
AGACCGCACAGGACACCTACGCAGTACTGTGTGTCGTCAACGAGGGAGGTTATGCCCACAGGGGCAACTTGG
TCGAGCTCGTCGCCGTCGGCAAGATCTCGAGCAAGAGCATGAAGCGCAGGACGGACGGCTCGATGTACGAGT
GCGACGACCTGGCCTATGACGGCTTGCCTTATGCGGAGTTCTCCGAGTGGAGGGAGGAACGGAAAGCATTCA
TGCATCGCCTGATCCCGCTGCTGCCTCCGGCAACCAAGAAGCTTGGCAGCAGCAGGGAGCCGATGGATCTGCC
GAAGGAACTCGAGGCCCTGACGCACTGCAAGTCGCTTTACGTGTGGAACCGCAGGTTCCTGACCATCTTCCGG
ATCGCACTGAAGAGGGCCACGAAGGAACAGGCGCCCGATCTGTTCCGCCAGGAGATACTCGACATCTGTCAC
GCCAATCTTAGCCCGCTGAGACGAAGCAGCCTGAGCAAGGACGGAGTTGAATCACTTCAGAAGCTGAAAGGA
CTCGTCAACGCCTACCTCAGCAAAATGAAGGCGAAGAAAGATCCACTGCTTGAGGCCGACCCAGAGTTCG
CCGAGCTGTTCAAGGTAATCAACGATCGTCTCAAGGCGAAAAGGCTCGAGAAGGCCAAACGCACAGCCAGCG
ATATCGTCCGGCTGGCCAACCTGCACGGCTGCTCCTTCGTCTCCGGCGAGTCCGACCTGCCGACGACCAGCAA
GGTCAACAAGAAATTCCAGAACGCAAGCAACATGGACTGGATGGCTCGCAAGGTCGAGGACCTCGTCAAGAG
CATGTGCATTAGGTCTGGCATCGCCTTTAGAGGCATCGACCCGTCCTACACGAGCCTGCGTGATCCGTTACAGT
TCGAGACCGACGGCAGGAGCGTCATCCGATGCCGTTTCTGGAAGGCGGAAAATGGCTCGGCTCCGGAATATG
 
TCATCGATGGTCTGGTGCGTTACACGAAGCACGCCGTCAAGAACATGGAGCAGTCAAGGCCCCTGTCGTCCTT
CCACAGAGGCGGGCTGGCCTTCTGCCGCAAGTACGGCATCGACCCAGCGAGACTGACGCCCAAGGTCGTCCG
TGGCGTGCTGGCGAGGCACGAGGTGGTATACTTGCCACGTGCCGGCGGCAGGTACTTCCTCGCCACGCTGCC
GCTGGTGGCGGGGGCTCCCGTCTTCGAGTGGGATGGCCAGCAGATGTATGTCCTCGACGCCGCAGAGGTCGC
TGCGATCAACATCGCACTCGAAGCCATACCTTCCAAGCTACCGtctggcggctcaaaaagaaccgccgacggcagcgaattc
gagcccaagaagaagaggaaagtctaa
**SEQ ID NO: 35, Nuclear localization signal (NLS)**
   KRTADGSEFESPKKKRKV
**SEQ ID NO: 36, Nuclear localization signal (NLS)**
   AVKRPAATKKAGQAKKKKLD
**SEQ ID NO: 37, Nuclear localization signal (NLS)**
   KRPAATKKAGQAKKKK
**SEQ ID NO: 38, Nuclear localization signal (NLS)**
   KKTELQTTNAENKTKKL
**SEQ ID NO: 39, Nuclear localization signal (NLS)**
   KRGINDRNFWRGENGRKTR
**SEQ ID NO: 40, Nuclear localization signal (NLS)**
   RKSGKIAAIVVKRPRK
**SEQ ID NO: 41, Nuclear localization signal (NLS)**
   PKKKRKV
**SEQ ID NO: 42, Nuclear localization signal (NLS)**
   MDSLLMNRRKFLYQFKNVRWAKGRRETYLC
**SEQ ID NO: 43, 005V1-10-3-dCasY6 fusion protein** 
MKRTADGSEFESPKKKRKVMSELNDAYWMKQALALAQKAREQGEVPVGAILVLDDEVIGQGWNRAITLHDPTAH
AEIMALQQGGQIVQNYRLLNATLYVTFEPCVMCAGAMVHSRIKRLVYGVSNSKRGAAGSLLNVLNYPGMNHQIEI
TAGVMANECSEMLCQFYGMRREVFNAEREARRLNQPDRADSGGSSGGSSGSETPGTSESATPESSGGSSGGSIKN
QIIGYEVKLDLNDRKRAFVRDTLRYFAQTSDVFRKGLMCLFAGVDPEMISGYLPEGESGDLWCAVNWFRLVDLSEG
 
LVSEEQVVDGSELCRRFQDYAGHAPDRQAEIYLTRRSEGDVQYVWADCRAGYRSFCREVGLSPDQMAFDVLALVA
RGYIKFDKGENDAWGFVSAVFGDGVKEDTKNKIRMLEGMRRAAHAKPATYEELQESALRETGCSDIKQMIMEYKS
GQQDDKEGVRKSSAGSHAFFVKYFLRGADRVCGPIGEKELELFVSSCDKTLKQKTSCDEAPITHSAVWKRWFKRRIP
HTVGSIQEPYSVACDQAMADISSKNCLNARMVSCPDSGSVAERRKKGTKAKKVGSSGVLEMQDKVSSLSSKGVDL
AKSVFDSFNDALDRDRKEDEDSCRFEVLRHHLGKLSDLYEIWVKNDCDSGIGLYLEEYEGSIKKQPKEELLRAIYPHRE
SIKAKEFMDAAHRNDLIQKIESRKAHPTVRGNRGVGYGNSKLRGKIMPQSNMHKGRKAGLNPEMWLEADLLNGE
RWEKHHLKFHSSQFFDQVYCHKDGLPYTKIPGLPRLARHGMPIGLSISPDDVPLVNRVSGNGRRCPRANKRNLRAV
ANITHNVELHPETAFEIRSNGEYASISHRVQDVAKKSIEAGDRSLGYDRNQTAQDTYAVLCVVNEGGYAHRGNLVEL
VAVGKISSKSMKRRTDGSMYECDDLAYDGLPYAEFSEWREERKAFMHRLIPLLPPATKKLGSSREPMDLPKELEALT
HCKSLYVWNRRFLTIFRIALKRATKEQAPDLFRQEILDICHANLSPLRRSSLSKDGVESLQKLKGLVNAYLSKMKAKKK
DPLLEADPEFAELFKVINDRLKAKRLEKAKRTASDIVRLANLHGCSFVSGESDLPTTSKVNKKFQNASNMDWMARK
VEDLVKSMCIRSGIAFRGIDPSYTSLRDPLQFETDGRSVIRCRFWKAENGSAPEYVIDGLVRYTKHAVKNMEQSRPLS
SFHRGGLAFCRKYGIDPARLTPKVVRGVLARHEVVYLPRAGGRYFLATLPLVAGAPVFEWDGQQMYVLDAAEVAAI
NIALEAIPSKLPSGGSKRTADGSEFEPKKKRKV*
**SEQ ID NO: 44, D659A-CasY6** 
MIKNQIIGYEVKLDLNDRKRAFVRDTLRYFAQTSDVFRKGLMCLFAGVDPEMISGYLPEGESGDLWCAVNWFRLV
DLSEGLVSEERVVDGSELCRRFQDYAGHAPDRQAEIYLTRRSEGDVQYVWADCRAGYRSFCREVGLSPDQMGFDV
LALVARGYIKFDKGENDAWGFVSAVFGDGVKEDTKNKIRMLEGMRRAAHAKPATYEELQESALRETGCSDIKQMI
REYKSGQQDDKEGVRKSSGGSHAFFVKYFLRGADRVCGPIGEKELELFVSSCDKTLKQKTSCDEAPITHSAVWKRWF
KRRIPHTVGSIQEPYSVACDQAMADISSKNCLNARMVSCPDSGSVAERRKKGTKAKKVGSSGVLEMQDKVSSLSSK
GVDLAKSVFDSFNDALDRDRKEDEDSCRFEVLRHHLGKLSDLYEIWVKNDCDSGIGLYLEEYEGSIKKQPKEELLRAIY
PHRESIKAKEFMDAAHRNDLIQKIESRKAHPTVRGNRGVGYGKSKMRGKIMPQSNMHNGRKAGLNPEMWLEAD
LLNGERWEKHHLKFHSSQFFDQVYCHKDGLPYTKIPGLPRLARHGMPIGLSISPDDVPLVNRVSGNGRRCPRANKR
NLRAVANITHNVELHPETAFEIRSNGEYASISHRVQDVAKKSIEAGDRSLGYARNQTAQDTYAVLCVVNEGGYAHR
GNLVELVAVGKISSKSMKRRTDGSMYECDDLAYDGLPYAEFSEWREERKAFMHRLIPLLPPATKKLGSSREPMDLPK
ELEALTHCKSLYVWNRRFLIIFRVALKRALKEQASNLFRQEILDICHANLSPLRRSSLSKSGVESLQKLKSLVNAYLSKM
KAKREDPLLEADPEFAELFKVITERLKAKRLEKAKRTASDIVRLANLHGCSFVSGESDLPTTSKVNKKFQNASNTDWM
ARKVEDLVKSMCIRSGIAFRGIDPSYTSLRDPLQFETDGRSVIRCRFWKAENGSAPEYVIDGLVRYTKHAVKNMEQS
RPLSSFHRGGLAFCRKYGIDPARLTPKVVRGVLARHEVVYLPRAGGRYFLATLPLVAGAPVFEWDGQQMYVLDADE
VAAINIALEAIPSKLP
**SEQ ID NO: 45, D711A-CasY6** 
MIKNQIIGYEVKLDLNDRKRAFVRDTLRYFAQTSDVFRKGLMCLFAGVDPEMISGYLPEGESGDLWCAVNWFRLV
DLSEGLVSEERVVDGSELCRRFQDYAGHAPDRQAEIYLTRRSEGDVQYVWADCRAGYRSFCREVGLSPDQMGFDV
LALVARGYIKFDKGENDAWGFVSAVFGDGVKEDTKNKIRMLEGMRRAAHAKPATYEELQESALRETGCSDIKQMI
 
REYKSGQQDDKEGVRKSSGGSHAFFVKYFLRGADRVCGPIGEKELELFVSSCDKTLKQKTSCDEAPITHSAVWKRWF
KRRIPHTVGSIQEPYSVACDQAMADISSKNCLNARMVSCPDSGSVAERRKKGTKAKKVGSSGVLEMQDKVSSLSSK
GVDLAKSVFDSFNDALDRDRKEDEDSCRFEVLRHHLGKLSDLYEIWVKNDCDSGIGLYLEEYEGSIKKQPKEELLRAIY
PHRESIKAKEFMDAAHRNDLIQKIESRKAHPTVRGNRGVGYGKSKMRGKIMPQSNMHNGRKAGLNPEMWLEAD
LLNGERWEKHHLKFHSSQFFDQVYCHKDGLPYTKIPGLPRLARHGMPIGLSISPDDVPLVNRVSGNGRRCPRANKR
NLRAVANITHNVELHPETAFEIRSNGEYASISHRVQDVAKKSIEAGDRSLGYDRNQTAQDTYAVLCVVNEGGYAHR
GNLVELVAVGKISSKSMKRRTDGSMYECADLAYDGLPYAEFSEWREERKAFMHRLIPLLPPATKKLGSSREPMDLPK
ELEALTHCKSLYVWNRRFLIIFRVALKRALKEQASNLFRQEILDICHANLSPLRRSSLSKSGVESLQKLKSLVNAYLSKM
KAKREDPLLEADPEFAELFKVITERLKAKRLEKAKRTASDIVRLANLHGCSFVSGESDLPTTSKVNKKFQNASNTDWM
ARKVEDLVKSMCIRSGIAFRGIDPSYTSLRDPLQFETDGRSVIRCRFWKAENGSAPEYVIDGLVRYTKHAVKNMEQS
RPLSSFHRGGLAFCRKYGIDPARLTPKVVRGVLARHEVVYLPRAGGRYFLATLPLVAGAPVFEWDGQQMYVLDADE
VAAINIALEAIPSKLP
**SEQ ID NO: 46, E895A-CasY6** 
MIKNQIIGYEVKLDLNDRKRAFVRDTLRYFAQTSDVFRKGLMCLFAGVDPEMISGYLPEGESGDLWCAVNWFRLV
DLSEGLVSEERVVDGSELCRRFQDYAGHAPDRQAEIYLTRRSEGDVQYVWADCRAGYRSFCREVGLSPDQMGFDV
LALVARGYIKFDKGENDAWGFVSAVFGDGVKEDTKNKIRMLEGMRRAAHAKPATYEELQESALRETGCSDIKQMI
REYKSGQQDDKEGVRKSSGGSHAFFVKYFLRGADRVCGPIGEKELELFVSSCDKTLKQKTSCDEAPITHSAVWKRWF
KRRIPHTVGSIQEPYSVACDQAMADISSKNCLNARMVSCPDSGSVAERRKKGTKAKKVGSSGVLEMQDKVSSLSSK
GVDLAKSVFDSFNDALDRDRKEDEDSCRFEVLRHHLGKLSDLYEIWVKNDCDSGIGLYLEEYEGSIKKQPKEELLRAIY
PHRESIKAKEFMDAAHRNDLIQKIESRKAHPTVRGNRGVGYGKSKMRGKIMPQSNMHNGRKAGLNPEMWLEAD
LLNGERWEKHHLKFHSSQFFDQVYCHKDGLPYTKIPGLPRLARHGMPIGLSISPDDVPLVNRVSGNGRRCPRANKR
NLRAVANITHNVELHPETAFEIRSNGEYASISHRVQDVAKKSIEAGDRSLGYDRNQTAQDTYAVLCVVNEGGYAHR
GNLVELVAVGKISSKSMKRRTDGSMYECDDLAYDGLPYAEFSEWREERKAFMHRLIPLLPPATKKLGSSREPMDLPK
ELEALTHCKSLYVWNRRFLIIFRVALKRALKEQASNLFRQEILDICHANLSPLRRSSLSKSGVESLQKLKSLVNAYLSKM
KAKREDPLLEADPEFAELFKVITERLKAKRLEKAKRTASDIVRLANLHGCSFVSGASDLPTTSKVNKKFQNASNTDW
MARKVEDLVKSMCIRSGIAFRGIDPSYTSLRDPLQFETDGRSVIRCRFWKAENGSAPEYVIDGLVRYTKHAVKNME
QSRPLSSFHRGGLAFCRKYGIDPARLTPKVVRGVLARHEVVYLPRAGGRYFLATLPLVAGAPVFEWDGQQMYVLD
ADEVAAINIALEAIPSKLP
**SEQ ID NO: 47, D1069A-CasY6** 
MIKNQIIGYEVKLDLNDRKRAFVRDTLRYFAQTSDVFRKGLMCLFAGVDPEMISGYLPEGESGDLWCAVNWFRLV
DLSEGLVSEERVVDGSELCRRFQDYAGHAPDRQAEIYLTRRSEGDVQYVWADCRAGYRSFCREVGLSPDQMGFDV
LALVARGYIKFDKGENDAWGFVSAVFGDGVKEDTKNKIRMLEGMRRAAHAKPATYEELQESALRETGCSDIKQMI
REYKSGQQDDKEGVRKSSGGSHAFFVKYFLRGADRVCGPIGEKELELFVSSCDKTLKQKTSCDEAPITHSAVWKRWF
KRRIPHTVGSIQEPYSVACDQAMADISSKNCLNARMVSCPDSGSVAERRKKGTKAKKVGSSGVLEMQDKVSSLSSK
 
GVDLAKSVFDSFNDALDRDRKEDEDSCRFEVLRHHLGKLSDLYEIWVKNDCDSGIGLYLEEYEGSIKKQPKEELLRAIY
PHRESIKAKEFMDAAHRNDLIQKIESRKAHPTVRGNRGVGYGKSKMRGKIMPQSNMHNGRKAGLNPEMWLEAD
LLNGERWEKHHLKFHSSQFFDQVYCHKDGLPYTKIPGLPRLARHGMPIGLSISPDDVPLVNRVSGNGRRCPRANKR
NLRAVANITHNVELHPETAFEIRSNGEYASISHRVQDVAKKSIEAGDRSLGYDRNQTAQDTYAVLCVVNEGGYAHR
GNLVELVAVGKISSKSMKRRTDGSMYECDDLAYDGLPYAEFSEWREERKAFMHRLIPLLPPATKKLGSSREPMDLPK
ELEALTHCKSLYVWNRRFLIIFRVALKRALKEQASNLFRQEILDICHANLSPLRRSSLSKSGVESLQKLKSLVNAYLSKM
KAKREDPLLEADPEFAELFKVITERLKAKRLEKAKRTASDIVRLANLHGCSFVSGESDLPTTSKVNKKFQNASNTDWM
ARKVEDLVKSMCIRSGIAFRGIDPSYTSLRDPLQFETDGRSVIRCRFWKAENGSAPEYVIDGLVRYTKHAVKNMEQS
RPLSSFHRGGLAFCRKYGIDPARLTPKVVRGVLARHEVVYLPRAGGRYFLATLPLVAGAPVFEWDGQQMYVLDAAE
VAAINIALEAIPSKLP
**SEQ ID NO: 48, Target sequence of TTR-3 locus of TTR gene**
   tagaagggatatacaaagtg
**SEQ ID NO: 49, Sequence of CasY6-TTR-sgRNA-3**
   TATCCATCGTGCCGCCTCTTGGCACtagaagggatatacaaagtg
**SEQ ID NO: 50, CasY6-TTR-3-sgRNA-Oligo-F**
   CACCTATCCATCGTGCCGCCTCTTGGCACtagaagggatatacaaagtg
**SEQ ID NO: 51, CasY6-TTR-3-sgRNA-Oligo-R**
   AAAACACTTTGTATATCCCTTCTAGTGCCAAGAGGCGGCACGATGGATA
**SEQ ID NO: 52, Sequence of dual-luciferase reporter plasmid** 
taacgcaggaaagaacatgtgagcaaaaggccagcaaaaggccaggaaccgtaaaaaggccgcgttgctggcgtttttccataggctccgccc
ccctgacgagcatcacaaaaatcgacgctcaagtcagaggtggcgaaacccgacaggactataaagataccaggcgtttccccctggaagctcc
ctcgtgcgctctcctgttccgaccctgccgcttaccggatacctgtccgcctttctcccttcgggaagcgtggcgctttctcatagctcacgctgtagg
tatctcagttcggtgtaggtcgttcgctccaagctgggctgtgtgcacgaaccccccgttcagcccgaccgctgcgccttatccggtaactatcgtct
tgagtccaacccggtaagacacgacttatcgccactggcagcagccactggtaacaggattagcagagcgaggtatgtaggcggtgctacagag
ttcttgaagtggtggcctaactacggctacactagaaggacagtatttggtatctgcgctctgctgaagccagttaccttcggaaaaagagttggt
agctcttgatccggcaaacaaaccaccgctggtagcggtggtttttttgtttgcaagcagcagattacgcgcagaaaaaaaggatctcaagaaga
tcctttgatcttttctacggggtctgacgctcagtggaacgaaaactcacgttaagggattttggtcatgagattatcaaaaaggatcttcacctag
atccttttaaattaaaaatgaagttttaaatcaatctaaagtatatatgagtaacctgaggctatggcagggcctgccgccccgacgttggctgcg
agccctgggccttcacccgaacttggggggtggggtggggaaaaggaagaaacgcgggcgtattggccccaatggggtctcggtggggtatcg
acagagtgccagccctgggaccgaaccccgcgtttatgaacaaacgacccaacaccgtgcgttttattctgtctttttattgccgtcatagcgcggg
ttccttccggtattgtctccttccgtgtttcagttagcctccccctagggtgggcgaagaactccagcatgagatccccgcgctggaggatcatcca
 
gccggcgtcccggaaaacgattccgaagcccaacctttcatagaaggcggcggtggaatcgaaatctcgtgatggcaggttgggcgtcgcttggt
cggtcatttcgaaccccagagtcccgctcagaagaactcgtcaagaaggcgatagaaggcgatgcgctgcgaatcgggagcggcgataccgta
aagcacgaggaagcggtcagcccattcgccgccaagctcttcagcaatatcacgggtagccaacgctatgtcctgatagcggtccgccacaccc
agccggccacagtcgatgaatccagaaaagcggccattttccaccatgatattcggcaagcaggcatcgccatgggtcacgacgagatcctcgc
cgtcgggcatgctcgccttgagcctggcgaacagttcggctggcgcgagcccctgatgctcttcgtccagatcatcctgatcgacaagaccggctt
ccatccgagtacgtgctcgctcgatgcgatgtttcgcttggtggtcgaatgggcaggtagccggatcaagcgtatgcagccgccgcattgcatcag
ccatgatggatactttctcggcaggagcaaggtgagatgacaggagatcctgccccggcacttcgcccaatagcagccagtcccttcccgcttca
gtgacaacgtcgagcacagctgcgcaaggaacgcccgtcgtggccagccacgatagccgcgctgcctcgtcttgcagttcattcagggcaccgg
acaggtcggtcttgacaaaaagaaccgggcgcccctgcgctgacagccggaacacggcggcatcagagcagccgattgtctgttgtgcccagtc
atagccgaatagcctctccacccaagcggccggagaacctgcgtgcaatccatcttgttcaatcatgcgaaacgatcctcatcctgtctcttgatcg
atctttgcaaaagcctaggcctccaaaaaagcctcctcactacttctggaatagctcagaggccgaggcggcctcggcctctgcataaataaaaa
aaattagtcagccatggggcggagaatgggcggaactgggcggagttaggggcgggatgggcggagttaggggcgggactatggttgctgact
aattgagatgcatgctttgcatacttctgcctgctggggagcctggggactttccacacctggttgctgactaattgagatgcatgctttgcatactt
ctgcctgctggggagcctggggactttccacaccctaactgacacacattccacagctggttctttccgcctcaggactcttcctttttcaatattatt
gaagcatttatcagggttattgtctcatgagcggatacatatttgaatgtatttagaaaaataaacaaataggggttccgcgcacatttccccgaa
aagtgccacctgacgcgccctgtagcggcgcattaagcgcggcgggtgtggtggttacgcgcagcgtgaccgctacacttgccagcgccctagcg
cccgctcctttcgctttcttcccttcctttctcgccacgttcgccggctttccccgtcaagctctaaatcgggggctccctttagggttccgatttagtgc
tttacggcacctcgaccccaaaaaacttgattagggtgatggttcacgtagtgggccatcgccctgatagacggtttttcgccctttgacgttggag
tccacgttctttaatagtggactcttgttccaaactggaacaacactcaaccctatctcggtctattcttttgatttataagggattttgccgatttcgg
cctattggttaaaaaatgagctgatttaacaaaaatttaacgcgaattttaacaaaatattaacgcttacaatttacgcgttaagatacattgatga
gtttggacaaaccacaactagaatgcagtgaaaaaaatgctttatttgtgaaatttgtgatgctattgctttatttgtaaccattataagctgcaata
aacaagttaacaacaacaattgcattcattttatgtttcaggttcagggggaggtgtgggaggttttttaaagcaagtaaaacctctacaaatgtg
gtatggctgattatgatcagttatctagatccggtggatcccgggttacaatttggactttccgcccttcttggcctttatgaggatctctctgattttt
cttgcgtcgagttttccggtaagacctttcggtacttcgtccacaaacacaactcctccgcgcaactttttcgcggttgttacttgactggcgacgta
atccacgatctctttttccgtcatcgtctttccgtgctccaaaacaacaacggcggcgggaagttcaccggcgtcatcgtcgggaagacctgccac
gcccgcgtcgaagatgttggggtgttgtaacaatatcgattccaattcagcgggggccacctgatatcctttgtatttaattaaagacttcaagcgg
tcaactatgaagaagtgttcgtcttcgtcccagtaagctatgtctccagaatgtagccatccatccttgtcaatcaaggcgttggtcgcttccggatt
gtttacataaccggacataatcataggtcctctgacacataattcgcctctctgattaacgcccagcgttttcccggtatccagatccacaaccttcg
cttcaaaaaatggaacaactttaccgaccgcgcccggtttatcatccccctcgggtgtaatcagaatagctgatgtagtctcagtgagcccatatc
cttgtcgtatccctggaagatggaagcgttttgcaaccgcttccccgacttctttcgaaagaggtgcgcccccagaagcaatttcgtgtaaattaga
taaatcgtatttgtcaatcagagtgcttttggcgaagaatgaaaatagggttggtactagcaacgcactttgaattttgtaatcctgaagggatcgt
aaaaacagctcttcttcaaatctatacattaagacgactcgaaatccacatatcaaatatccgagtgtagtaaacattccaaaaccgtgatggaa
tggaacaacacttaaaatcgcagtatccggaatgatttgattgccaaaaataggatctctggcatgcgagaatctgacgcaggcagttctatgcg
gaagggccacacccttaggtaacccagtagatccagaggaattcattatcagtgcaattgttttgtcacgatcaaaggactctggtacaaaatcgt
attcattaaaaccgggaggtagatgagatgtgacgaacgtgtacatcgactgaaatccctggtaatccgttttagaatccatgataataattttctg
gattattggtaattttttttgcacgttcaaaattttttgcaacccctttttggaaacaaacactacggtaggctgcgaaatgttcatactgttgagcaa
 
ttcacgttcattataaatgtcgttcgcgggcgcaactgcaactccgataaataacgcgcccaacaccggcataaagaattgaagagagttttcact
gcatacgacgattctgtgatttgtattcagcccatatcgtttcatagcttctgccaaccgaacggacatttcgaagtattccgcgtacgtgatgttca
cctcgatatgtgcatctgtaaaagcaattgttccaggaaccagggcgtatctcttcatagccttatgcagttgctctccagcggttccatcctctaga
ggatagaatggcgccgggcctttctttatgtttttggcgtcttccatgctagcggatctgacggttcactaaaccagctctgcttatatagacctccc
accgtacacgcctaccgcccatttgcgtcaatggggcggagttgttacgacattttggaaagtcccgttgattttggtgccaaaacaaactcccatt
gacgtcaatggggtggagacttggaaatccccgtgagtcaaaccgctatccacgcccattgatgtactgccaaaaccgcatcaccatggtaatag
cgatgactaatacgtagatgtactgccaagtaggaaagtcccataaggtcatgtactgggcataatgccaggcgggccatttaccgtcattgacg
tcaatagggggcgtacttggcatatgatacacttgatgtactgccaagtgggcagtttaccgtaaatactccacccattgacgtcaatggaaagtc
cctattggcgttactatgggaacatacgtcattattgacgtcaatgggcgggggtcgttgggcggtcagccaggcgggccatttaccgtaagttat
gtaacgcggaactccatatatgggctatgaactaatgaccccgtaattgattactatTAGGcacgtgggctccggtgcccgtcagtgggcagag
cgcacatcgcccacagtccccgagaagttggggggaggggtcggcaattgaaccggtgcctagagaaggtggcgcggggtaaactgggaaagt
gatgtcgtgtactggctccgcctttttcccgagggtgggggagaaccgtatataagtgcagtagtcgccgtgaacgttctttttcgcaacgggtttg
ccgccagaacacaggtaagtgccgtgtgtggttcccgcgggcctggcctctttacgggttatggcccttgcgtgccttgaattacttccacctggct
gcagtacgtgattcttgatcccgagcttcgggttggaagtgggtgggagagttcgaggccttgcgcttaaggagccccttcgcctcgtgcttgagtt
gaggcctggcctgggcgctggggccgccgcgtgcgaatctggtggcaccttcgcgcctgtctcgctgctttcgataagtctctagccatttaaaatt
tttgatgacctgctgcgacgctttttttctggcaagatagtcttgtaaatgcgggccaagatctgcacactggtatttcggtttttggggccgcgggc
ggcgacggggcccgtgcgtcccagcgcacatgttcggcgaggcggggcctgcgagcgcggccaccgagaatcggacgggggtagtctcaagct
ggccggcctgctctggtgcctggcctcgcgccgccgtgtatcgccccgccctgggcggcaaggctggcccggtcggcaccagttgcgtgagcgga
aagatggccgcttcccggccctgctgcagggagctcaaaatggaggacgcggcgctcgggagagcgggcgggtgagtcacccacacaaagga
aaagggcctttccgtcctcagccgtcgcttcatgtgactccactgagtaccgggcgccgtccaggcacctcgattagttctcgagcttttggagtac
gtcgtctttaggttggggggaggggttttatgcgatggagtttccccacactgagtgggtggagactgaagttaggccagcttggcacttgatgta
attctccttggaatttgccctttttgagtttggatcttggttcattctcaagcctcagacagtggttcaaagtttttttcttccatttcaggtgtcgtgatc
tagagACTAGTCCCCTAGCCATGGGATCCCAGTCCGGCgtcttcacactcgaagatttcgttggggactggcgacagacagccgg
ctacaacctggaccaagtccttgaacagggaggtgtgtccagtttgtttcagaatcttttgtagaagggatatacaaagtgcGGgtgacggctac
aacctggaccaagtccttgaacagggaggtgtgtccagtttgtttcagaatctcggggtgtccgtaactccgatccaaaggattgtcctgagcggt
gaaaatgggctgaagatcgacatccatgtcatcatcccgtatgaaggtctgagcggcgaccaaatgggccagatcgaaaaaatttttaaggtgg
tgtaccctgtggatgatcatcactttaaggtgatcctgcactatggcacactggtaatcgacggggttacgccgaacatgatcgactatttcggacg
gccgtatgaaggcatcgccgtgttcgacggcaaaaagatcactgtaacagggaccctgtggaacggcaacaaaattatcgacgagcgcctgatc
aaccccgacggctccctgctgttccgagtaaccatcaacggagtgaccggctggcggctgtgcgaacgcattctggcgtaaGTCGACTGTGC
CTTCTAGTTGCCAGCCATCTGTTGTTTGCCCCTCCCCCGTGCCTTCCTTGACCCTGGAAGGTGCCACTCCCACTG
TCCTTTCCTAATAAAATGAGGAAATTGCATCGCATTGTCTGAGTAGGTGTCATTCTATTCTGGGGGGTGGGGTG
GGGCAGGACAGCAAGGGGGAGGATTGGGAAGACAATAGCAGGCATGCTGGGGATGCGGTGGGCTCTATGG
ctcaagcttataactaatgcatggcggtaatacggttatccacagaatcagggga
**SEQ ID NO: 53, Intermediate inserted fragment of LUxUC**
   tttgtagaagggatatacaaagtggaaatcGGgtga
**SEQ ID NO: 54, Amino acid sequence of 004V1 deaminase** 
MYNAPRFFCRSAAVSDHEFNDEYWMRHALTLAKRAREEGEVPVGAVLVLNNQVIGEGWNRAIGLHDPTAHAEI
MALRQGGLVLQNYRLIDATLYVTFEPCVMCAGAMVHSRISRLVFGVRNSKRGAAGSLINVLNYPGMNHRVEITEGI
LAESCSAMLCDFYRWPREVFNALKKARQEEG
**SEQ ID NO: 55, Amino acid sequence of TadA8e** 
MSEVEFSHEYWMRHALTLAKRARDEREVPVGAVLVLNNRVIGEGWNRAIGLHDPTAHAEIMALRQGGLVMQNY
RLIDATLYVTFEPCVMCAGAMIHSRIGRVVFGVRNSKRGAAGSLMNVLNYPGMNHRVEITEGILADECAALLCDFY
RMPRQVFNAQKKAQSSIN
**SEQ ID NO: 56, 1.1-F**
   CACTTCGTAAGGTCGCTTGACAAGACCCTGAAGCAAAA
**SEQ ID NO: 57, 1.1-R**
   AAGCGACCTTACGAAGTGTTCCAGCTCCTTCTCGCCGA
**SEQ ID NO: 58, 15-nt TTR-3 spacer**
   tagaagggatataca
**SEQ ID NO: 59, 18-nt TTR-3 spacer**
   tagaagggatatacaaag
**SEQ ID NO: 60, 19-nt TTR-3 spacer**
   tagaagggatatacaaagt
**SEQ ID NO: 61, 21-nt TTR-3 spacer**
   tagaagggatatacaaagtgg
**SEQ ID NO: 62, 22-nt TTR-3 spacer**
   tagaagggatatacaaagtgga
**SEQ ID NO: 63, 23-nt TTR-3 spacer**
   tagaagggatatacaaagtggaa
**SEQ ID NO: 64, 25-nt TTR-3 spacer**
   Tagaagggatatacaaagtggaaat

All the documents mentioned in the disclosure are incorporated by reference in this application, as if each document were individually cited as a reference. Additionally, it should be understood that after reading the above-mentioned teachings of the disclosure, those skilled in the art can make various changes or modifications to the disclosure, and these equivalent forms also fall within the scope defined by the appended claims of this application.

## Claims

1. A Cas protein selected from the group consisting of:
(a) a polypeptide having the amino acid sequence of SEQ ID NO: 1;
(b) a polypeptide having a homology (or identity) of ≥ 80%, 81%, 82%, 83%, 84%, 85%, 86%, 87%, 88%, 89%, 90%, 91%, 92%, 93%, 94%, 95%, 96%, 97%, 98%, 99% or 99.5% to the amino acid sequence of SEQ ID NO: 1, and having the biological function of SEQ ID NO: 1;
(c) a derivative polypeptide generated by substitution, deletion, or addition of one or more (preferably 1-20, more preferably 1-10, even more preferably 1-5) amino acid residues in the amino acid sequence of SEQ ID NO: 1, and retaining the biological function of SEQ ID NO: 1;
preferably, wherein the Cas protein comprises mutations at one or more key amino acid sites related to cleavage activity in SEQ ID NO: 1, said sites being selected from the group consisting of E118, G119, D120, V121, Q122, Y123, K241, S242, S243, S246, L272, S275, C277, T285, S286, C287, D288, E289, A290, 1292, K299, R300, W301, L394, D395, R396, D397, R398, K399, E400, D401, E402, D403, S404, C405, R406, F407, E408, K502, K557, L657, C672, P758, L789, K827, and V860;
preferably, wherein the Cas protein comprises mutations at key amino acid sites related to cleavage activity in SEQ ID NO: 1, said sites being selected from the group consisting of L272, S275, and C277;
preferably, wherein the Cas protein further comprises mutations at two or more key amino acid sites related to cleavage activity in SEQ ID NO: 1, said sites being selected from the group consisting of E289, A290, and I292;
preferably, wherein the Cas protein further comprises mutations at key amino acid sites related to cleavage activity in SEQ ID NO: 1, said sites being selected from the group consisting of K399, E400, D401, E402, and D403;
preferably, wherein the Cas protein further comprises mutations at three or more key amino acid sites related to cleavage activity in SEQ ID NO: 1, said sites being selected from the group consisting of D403, S404, C405, R406, F407, and E408;
preferably, wherein the Cas protein further comprises mutations at four or more key amino acid sites related to cleavage activity in SEQ ID NO: 1, said sites being selected from the group consisting of L394, D395, R396, D397, and R398;
preferably, wherein the Cas protein further comprises mutations at five or more key amino acid sites related to cleavage activity in SEQ ID NO: 1, said sites being selected from the group consisting of D397, R398, K399, E400, D401, and E402;
preferably, wherein the Cas protein further comprises mutations at key amino acid sites related to cleavage activity in SEQ ID NO: 1, said sites being selected from the group consisting of S242, 5243, and S246;
preferably, wherein the Cas protein further comprises mutations at four or more key amino acid sites related to cleavage activity in SEQ ID NO: 1, said sites being selected from the group consisting of E118, G119, D120, V121, Q122, and Y123;
preferably, wherein the Cas protein further comprises mutations at key amino acid sites related to cleavage activity in SEQ ID NO: 1, said sites being selected from the group consisting of D397, R398, E400R, D401, E402, and L789;
preferably, wherein the Cas protein further comprises mutations at key amino acid sites related to cleavage activity in SEQ ID NO: 1, said sites being selected from the group consisting of T285, 5286, C287, D288, D403, C405, R406, F407, E408, K502, and K557;
preferably, wherein the Cas protein further comprises mutations at key amino acid sites related to cleavage activity in SEQ ID NO: 1, said sites being selected from the group consisting of E289, I292, K399, E400, D401, E402, and D403;
preferably, wherein the Cas protein further comprises mutations at key amino acid sites related to cleavage activity in SEQ ID NO: 1, said sites being selected from the group consisting of E289, A290, I292, P758, and V860;
preferably, wherein the Cas protein further comprises mutations at key amino acid sites related to cleavage activity in SEQ ID NO: 1, said sites being selected from the group consisting of K299, R300, W301, C672, and P758;
preferably, wherein the Cas protein further comprises mutations at key amino acid sites related to cleavage activity in SEQ ID NO: 1, said sites being selected from the group consisting of D397, R398, E400, D401, E402, L657, and L789;
preferably, wherein the Cas protein further comprises mutations at key amino acid sites related to cleavage activity in SEQ ID NO: 1, said sites being selected from the group consisting of E118, G119, D120, V121, Q122, C672, and P758;
preferably, wherein the Cas protein further comprises mutations at key amino acid sites related to cleavage activity in SEQ ID NO: 1, said sites being selected from the group consisting of K399, E400, D401, E402, D403, P758, and K827;
preferably, wherein the Cas protein further comprises mutations at key amino acid sites related to cleavage activity in SEQ ID NO: 1, said sites being selected from the group consisting of E118, G119, D120, V121, Q122, L394, D395, R396, D397, R398, C672, and P758;
preferably, wherein the Cas protein further comprises mutations at twelve or more key amino acid sites related to cleavage activity in SEQ ID NO: 1, said sites being selected from the group consisting of E118, G119, D120, V121, Q122, D403, S404, C405, R406, F407, E408, C672, and P758;
preferably, wherein the Cas protein further comprises mutations at key amino acid sites related to cleavage activity in SEQ ID NO: 1, said sites being selected from the group consisting of K241, S242, S243, S246, D397, R398, E400, D401, E402, and L789.

2. A protein variant, wherein the variant is an unnatural protein and comprises mutations at one or more key amino acid sites related to cleavage activity in SEQ ID NO: 1 corresponding to the wild-type protein, said sites being selected from the groups consisting of:
the aspartic acid (D) site at position 659; and/or
the aspartic acid (D) site at position 711; and/or
the glutamic acid (E) site at position 895; and/or
the aspartic acid (D) site at position 1069;
preferably, wherein the mutation comprises one or more amino acid substitutions selected from D659A, D711A, E895A, and D1069A.

3. A fusion protein comprising:
the Cas protein according to claim 1 or the protein variant according to claim 2; and
one or more functional domains.

4. An isolated polynucleotide, encoding the Cas protein according to claim 1, the protein variant according to claim 2, or the fusion protein according to claim 3.

5. An isolated nucleic acid molecule, comprising or consisting of a sequence selected from:
(i) the sequence of SEQ ID NO: 5;
(ii) a sequence having one or more base substitutions, deletions or additions (e.g., 1, 2, **3, 4,** 5, 6, 7, 8, 9 or 10 base substitutions, deletions or additions) relative to the sequence of SEQ ID NO: 5;
(iii) a sequence having at least 20%, at least 30%, at least 40%, at least 50%, at least 60%, at least 70%, at least 80%, at least 90%, or at least 95% sequence identity to the sequence of SEQ ID NO: 5;
(iv) a sequence that hybridizes to the sequence of any one of (i)-(iii) under stringent conditions; or
(v) a sequence complementary to the sequence of any one of (i)-(iii);
and wherein the sequence of any one of (ii)-(v) substantially retains the biological function of the sequence from which it is derived;
for example, wherein the isolated nucleic acid molecule is RNA;
for example, wherein the isolated nucleic acid molecule comprises a direct repeat sequence in the CRISPR/Cas system.

6. A guide RNA (gRNA), comprising a direct repeat (DR) sequence capable of binding to the Cas protein according to claim 1 or the protein variant according to claim 2, and a spacer sequence capable of targeting a target sequence.

7. A complex comprising:
(i) a protein component selected from the group consisting of the Cas protein according to claim 1, the protein variant according to claim 2, the fusion protein according to claim 3, and a combination thereof; and
(ii) a nucleic acid component selected from the group consisting of the guide RNA according to claim 6, a nucleic acid encoding the guide RNA according to claim 6, a precursor RNA of the guide RNA according to claim 6, a nucleic acid encoding the precursor RNA of the guide RNA according to claim 6, and a combination thereof;
wherein the protein component and the nucleic acid component bind to each other to form a complex.

8. A vector, comprising the polynucleotide according to claim 4.

9. A CRISPR-Cas composition, comprising:
(i) a first component selected from the group consisting of the Cas protein according to claim 1, the protein variant according to claim 2, the fusion protein according to claim 3, a nucleotide sequence encoding the Cas protein according to claim 1 or the protein variant according to claim 2 or the fusion protein according to claim 3, and any combination thereof; and
(ii) a second component, which is a nucleotide sequence comprising one or more guide RNAs according to claim 6, or a nucleotide sequence encoding the one or more guide RNAs according to claim 6;
wherein the guide RNA is capable of forming a complex with the protein, protein variant or fusion protein described in (i).

10. A CRISPR-Cas system, comprising one or more vectors comprising:
(i) a first nucleic acid, which is a nucleotide sequence encoding the Cas protein according to claim 1, the protein variant according to claim 2, or the fusion protein according to claim 3; optionally, wherein the first nucleic acid is operably linked to a first regulatory element; and
(ii) a second nucleic acid, which encodes a nucleotide sequence comprising the guide RNA according to claim 6; optionally, wherein the second nucleic acid is operably linked to a second regulatory element;
wherein:
the first nucleic acid and the second nucleic acid are present on the same vector or on different vectors;
the guide RNA is capable of forming a complex with the Cas protein or fusion protein described in (i).

11. A kit, comprising one or more components selected from the group consisting of: the Cas protein according to claim 1, the protein variant according to claim 2, the fusion protein according to claim 3, the polynucleotide according to claim 4, the complex according to claim 7, the vector according to claim 8, the CRISPR-Cas composition according to claim 9, and the system according to claim 10.

12. A delivery composition, comprising a delivery vector, and one or more selected from the group consisting of: the Cas protein according to claim 1, the protein variant according to claim 2, the fusion protein according to claim 3, the polynucleotide according to claim 4, the complex according to claim 7, the vector according to claim 8, the CRISPR-Cas composition according to claim 9, and the system according to claim 10.

13. A host cell, comprising: the Cas protein according to claim 1, the protein variant according to claim 2, the fusion protein according to claim 3, the polynucleotide according to claim 4, the complex according to claim 7, the vector according to claim 8, the composition according to claim 9, the system according to claim 10, or the delivery composition according to claim 12.

14. An enzyme preparation, comprising: the Cas protein according to claim 1, the protein variant according to claim 2, the fusion protein according to claim 3, the complex according to claim 7, the CRISPR-Cas composition according to claim 9, the system according to claim 10, or the delivery composition according to claim 12.

15. A medicine kit, comprising:
a first container, and
within the first container, the complex according to claim 7, the composition according to claim 9, or the system according to claim 10; or a drug containing the complex according to claim 7, the composition according to claim 9, or the system according to claim 10.

16. A medicine kit, comprising:
(a1) a first container, and
within the first container, the Cas protein according to claim 1, the protein variant according to claim 2, or the fusion protein according to claim 3, or an encoding gene thereof or an expression vector thereof; or a drug containing the Cas protein according to claim 1, the protein variant according to claim 2, or the fusion protein according to claim 3, or an encoding gene thereof or an expression vector thereof; and
optionally (b1) a second container, and
within the second container, the guide RNA according to claim 6 or its expression vector; or a drug containing the guide RNA according to claim 6 or its expression vector.

17. A method for targeting and editing a target gene or cleaving a target gene, comprising:
contacting the Cas protein according to claim 1, the protein variant according to claim 2, the fusion protein according to claim 3, the complex according to claim 7, the composition according to claim 9, the system according to claim 10, the delivery composition according to claim 12, the enzyme preparation according to claim 14, or the medicine kit according to claim 15 or 16 with the target gene, or delivering it into a cell containing the target gene,
wherein the target sequence is present in the target gene.

18. A method for inducing a change in the state of a cell, comprising:
contacting the Cas protein according to claim 1, the protein variant according to claim 2, the fusion protein according to claim 3, the complex according to claim 7, the composition according to claim 9, the system according to claim 10, the delivery composition according to claim 12, the enzyme preparation according to claim 14, or the medicine kit according to claim 15 or 16 with the target gene in the cell.

19. A method for altering the expression of a gene product, comprising:
contacting the Cas protein according to claim 1, the protein variant according to claim 2, the fusion protein according to claim 3, the complex according to claim 7, the composition according to claim 9, the system according to claim 10, the delivery composition according to claim 12, the enzyme preparation according to claim 14, or the medicine kit according to claim 15 or 16 with a nucleic acid molecule encoding the gene product, or delivering it into a cell containing the nucleic acid molecule,
wherein the target sequence is in the nucleic acid molecule.

20. Use of the Cas protein according to claim 1, the protein variant according to claim 2, the fusion protein according to claim 3, the polynucleotide according to claim 4, the complex according to claim 7, the vector according to claim 8, the CRISPR-Cas composition according to claim 9, the system according to claim 10, the kit according to claim 11, the delivery composition according to claim 12, the enzyme preparation according to claim 14, or the medicine kit according to claim 15 or 16, for the manufacture of a medicament or formulation for nucleic acid editing, e.g., gene editing or genome editing.

21. Use of the Cas protein according to claim 1, the protein variant according to claim 2, the fusion protein according to claim 3, the polynucleotide according to claim 4, the complex according to claim 7, the vector according to claim 8, the CRISPR-Cas composition according to claim 9, the system according to claim 10, the kit according to claim 11, the delivery composition according to claim 12, the enzyme preparation according to claim 14, or the medicine kit according to claim 15 or 16, for the manufacture of a medicament or formulation for one or more of:
(i) *ex vivo* gene editing or genome editing;
(ii) *ex vivo* detection of a single-stranded DNA;
(iii) modifying a biological or non-human organism by editing a target sequence in a target locus;
(iv) treating a disorder caused by a defect in a target sequence in a target locus; and
(v) treating a disorder or disease in a subject in need.

22. A method for detecting the presence of a target nucleic acid molecule in a sample, comprising:
contacting the sample with the Cas protein according to claim 1, the protein variant according to claim 2, the fusion protein according to claim 3, the complex according to claim 7, the CRISPR-Cas composition according to claim 9, the system according to claim 10, the kit according to claim 11, the delivery composition according to claim 12, or the enzyme preparation according to claim 14, and a non-target sequence; and
detecting a detectable signal generated by the cleavage of the non-target sequence to detect the target nucleic acid molecule,
wherein the non-target sequence does not hybridize to the guide RNA.
